(19) Europäisches Patentamt
European Patent Office
Office européen des brevets

(11) EP 0 688 314 B1

(12) EUROPÄISCHE PATENTSCHRIFT

(45) Veröffentlichungstag und Bekanntmachung des Hinweises auf die Patenterteilung:
**04.10.2001 Patentblatt 2001/40**

(21) Anmeldenummer: **94909071.6**

(22) Anmeldetag: **25.02.1994**

(51) Int Cl.[7]: **C07C 317/44**, C07C 323/52, A61K 31/22

(86) Internationale Anmeldenummer:
**PCT/EP94/00561**

(87) Internationale Veröffentlichungsnummer:
**WO 94/21604 (29.09.1994 Gazette 1994/22)**

(54) **PRODRUG-DERIVATE VON ENZYMINHIBITOREN MIT HYDROXYLGRUPPEN, VERFAHREN ZU DEREN HERSTELLUNG UND IHRE VERWENDUNG**

PRODRUG DERIVATIVES OF ENZYME INHIBITORS WITH HYDROXYL GROUPS, METHODS OF PREPARING THEM AND THEIR USE

DERIVES PROMEDICAMENTEUX D'INHIBITEURS D'ENZYMES AVEC DES GROUPES HYDROXYLE, PROCEDE DE PREPARATION ET UTILISATION

(84) Benannte Vertragsstaaten:
**AT BE CH DE DK ES FR GB GR IE IT LI LU MC NL PT SE**

(30) Priorität: **13.03.1993 DE 4308096**

(43) Veröffentlichungstag der Anmeldung:
**27.12.1995 Patentblatt 1995/52**

(73) Patentinhaber: **HOECHST AKTIENGESELLSCHAFT**
**65929 Frankfurt am Main (DE)**

(72) Erfinder:
- **BUDT, Karl-Heinz**
  **D-65779 Kelkheim/Taunus (DE)**
- **STOWASSER, Bernd**
  **D-65428 Russelsheim (DE)**
- **PEYMAN, Anuschirwan**
  **D-65779 Kelkheim/Taunus (DE)**
- **KNOLLE, Jochen**
  **D-65830 Kriftel (DE)**
- **WINKLER, Irvin**
  **D-65835 Liederbach (DE)**
- **BERSCHEID, Hans-Gerd**
  **D-61476 Kronberg (DE)**

(56) Entgegenhaltungen:
**EP-A- 0 428 849**

- **JOURNAL OF MEDICINAL CHEMISTRY Bd. 33, Nr. 5 , Mai 1990 , WASHINGTON US Seiten 1505 - 10 S.K. AGGARWAL ET. AL. 'Synthesis and Biological Evaluation of Prodrugs of Zidovudine.'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 688 314 B1

## Beschreibung

Die vorliegende Erfindung betrifft Prodrugs, mit deren Hilfe die Wasserlöslichkeit und pharmakokinetische Parameter von Enzymihibitoren verbessert werden können.

Es ist bekannt, daß eine breite Vielfalt von Verbindungen mit einer Hydroxylgruppe (-OH) nützliche Wirkstoffe für die Behandlung oder Bekämpfung verschiedener Krankheitsbilder oder -zustände sind. Es ist ebenfalls bekannt, daß solche, dem Stand der Technik entsprechende Verbindungen, teilweise durch einen gewissen inhärenten Nachteil charakterisiert sind, insbesondere durch Probleme mit der Bioverfügbarkeit nach der Verabreichung sowie durch Probleme mit der Stabilität oder der pharmazeutischen Formulierung. Eine solche verringerte Bioverfügbarkeit kann teilweise auf schlechte Löslichkeit in Wasser und auch auf Stoffwechselverluste während und nach der konventionellen Verabreichung zurückzuführen sein. Auch macht es die schlechte Wasserlöslichkeit vieler dem Stand der Technik entsprechenden Produkte schwierig oder unmöglich, Formulierungen herzustellen, die sich für intravenöse oder intramuskuläre Injektionen oder ophtalmische Anwendungen eignen.

Ein vielversprechender Ansatz zur Lösung dieser Probleme lag in der Veresterung der Hydroxylfunktion von Wirkstoffen, um pharmakologisch verbesserte Prodrug-Formen zu erhalten.

Einige Typen von Estern wurden bereits als Prodrugs hydroxylhaltiger Wirkstoffe (Hans Bundgaard, Design of Prodrugs, Elsevier Science Publishers B. V., Amsterdam, 1985) beschrieben.

Diese Ester-Prodrugs sind von enzymatischer Hydrolyse (Esterasen) abhängig, um brauchbare Umwandlungsgeschwindigkeiten von Prodrugs in Wirkstoffe zu erhalten. Dieser Ansatz hat demzufolge mehrere Nachteile:

1) Es gibt starke Schwankungen der Esterasekonzentrationen bei verschiedenen Individuen. Dies kann zu ungleichmäßigen und unvorhersagbaren Wirkstoffspiegeln führen.

2) Die Geschwindigkeit der enzymatischen Hydrotyse der Ester durch Esterasen hängt sowohl von der Säure als auch vom hydroxylhaltigen Teil des Esters ab (Bundgaard s.o.). Einige Ester, z. B. Ester von sterisch ausspruchsvollen hydroxylhaltigen Wirkstoffen, sind sehr schlechte Substrate für Esterasen.

Es ist das Ziel dieser Erfindung, Ester-Prodrugs von hydroxylhaltigen Arzneimitteln zur Verfügung zu stellen, bei denen die vorstehend aufgeführten Nachteile nicht auftreten.

Es wurde nun gefunden, daß bestimmte Prodrugs von Enzyminhibitoren die zuvor genannten Nachteile nicht aufweisen. Erfindungsgegenstand sind demzufolge Verbindungen der Formel I worin mehrfach vorkommende gleich definierte Reste unabhängig voneinander sein können

$$W\text{-}[R^5]_a \quad \text{I,}$$

worin W einen mono-, bis- oder tris-deshydroxylierten Rest einer Verbindung der nachfolgenden Formeln bedeutet

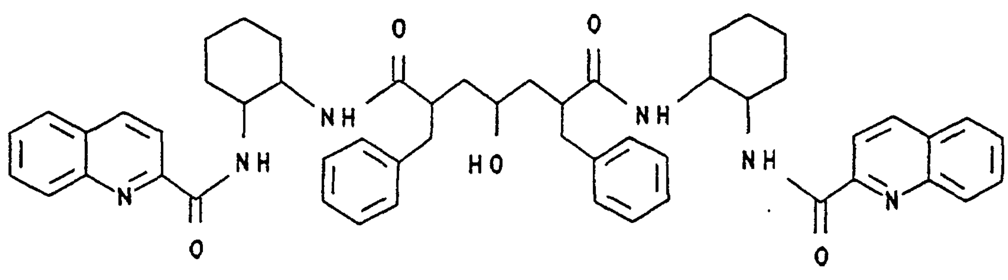

F. 1

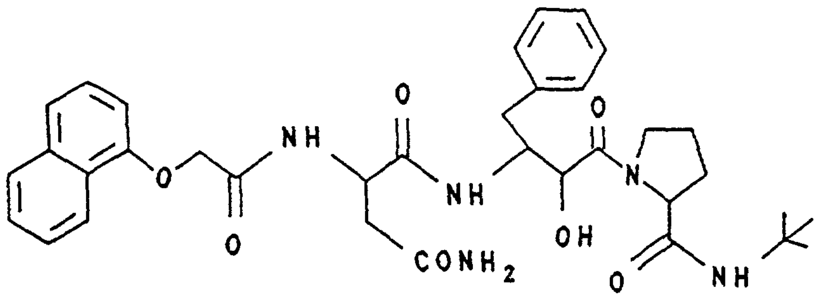

J. Med. Chem., 35, 1319, (1992) F. 2

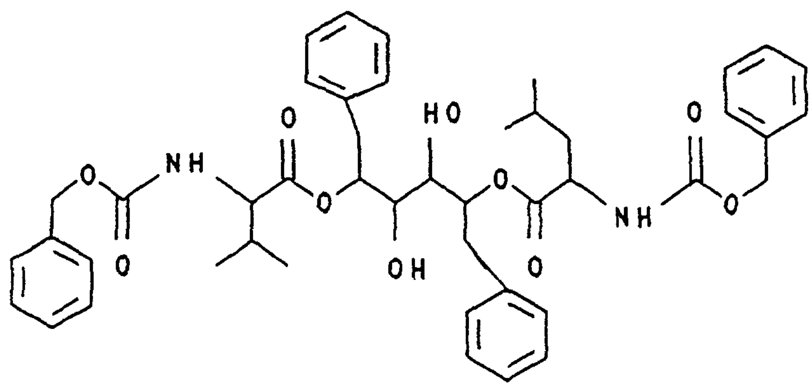

WO 92 16501-A F. 3

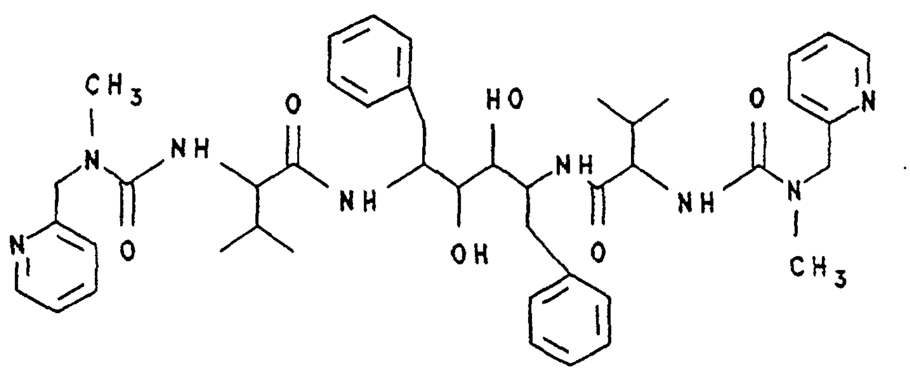

Antimicrobial Agents and Chemotherapy, 35, 2209, (1991) F. 4

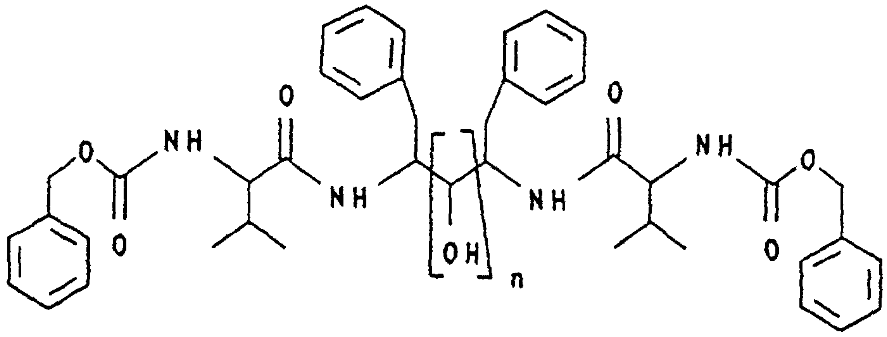

n = 1,2 F. 5

J. Med. Chem., 35, 2687, (1990)

Antimicrobial Agents and Chemotherapy, 35, 2209, (1991)

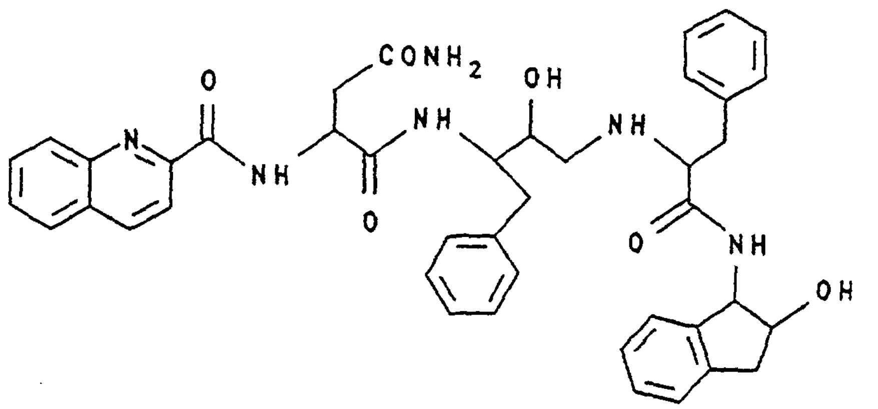

J. Med. Chem., 35, 2528, (1992) F. 6

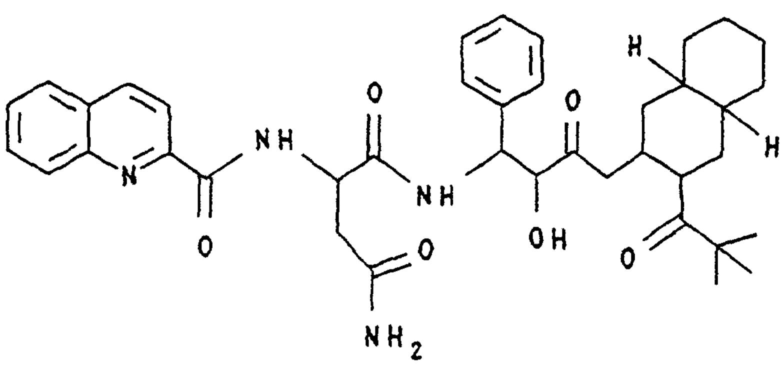

F. 7

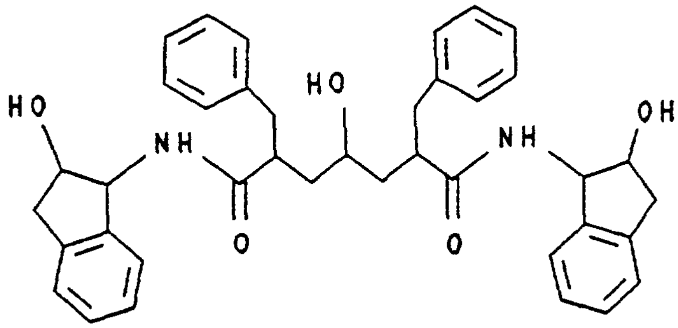

J. Med. Chem., 34, 9382, (1991) F. 8

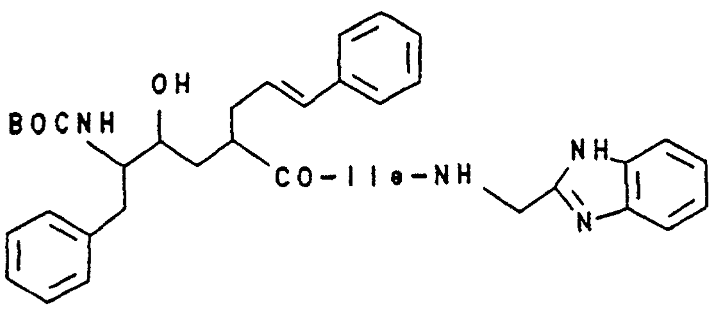

J. Med. Chem., 34, 1226, (1992) F. 9

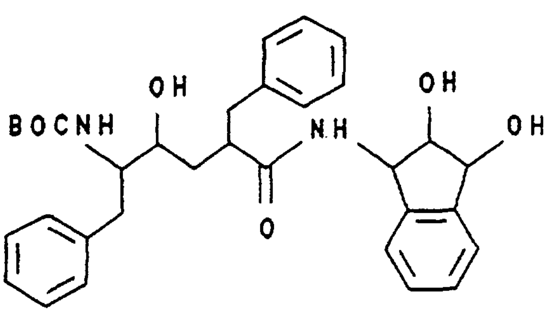

J. Med. Chem. 34, 1228, (1991) F. 10

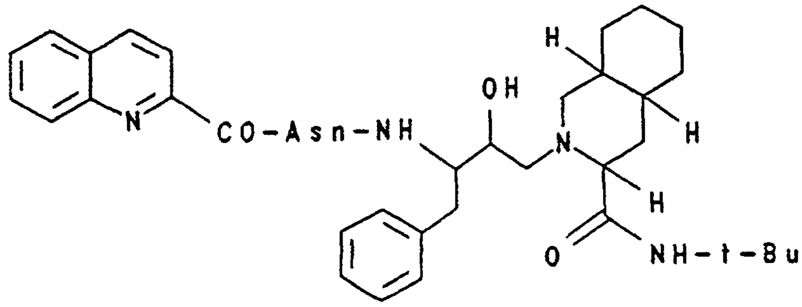

Antiviral Research, 17, 265, (1992) F. 11

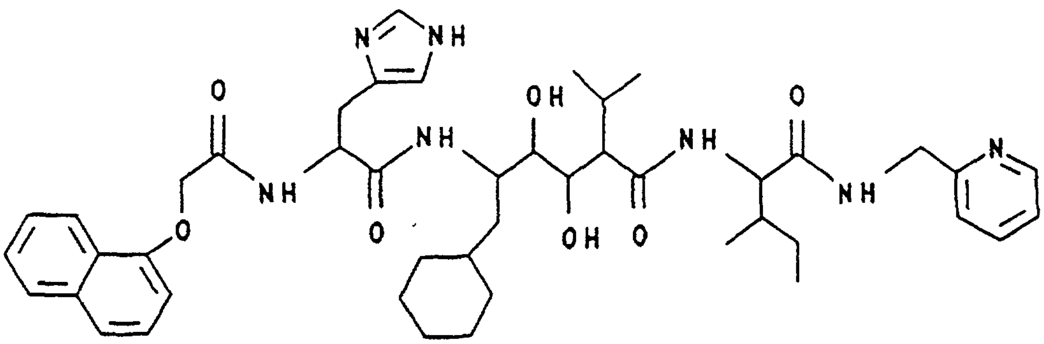

J. Med. Chem., 34, 2348, (1991) F. 12

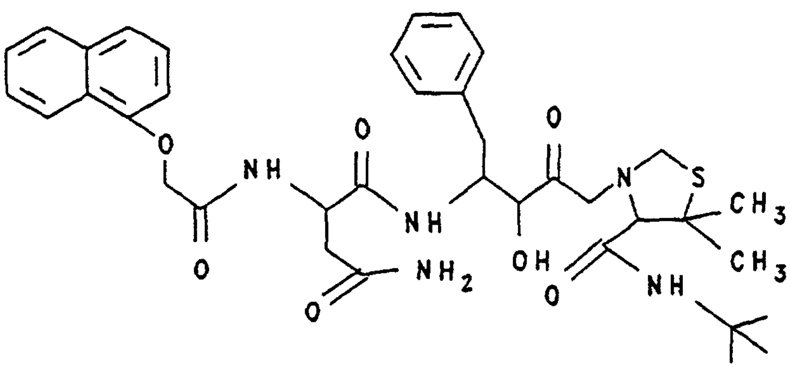

Peptide Chemistry, 399, (1991) F. 13

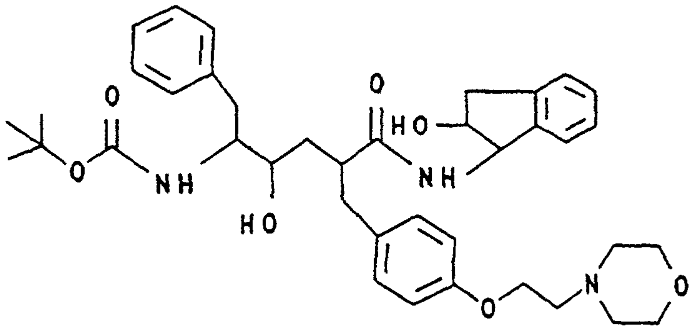

Biochem. Biophys. Res. Commun., 181, 1459, (1991) F. 14

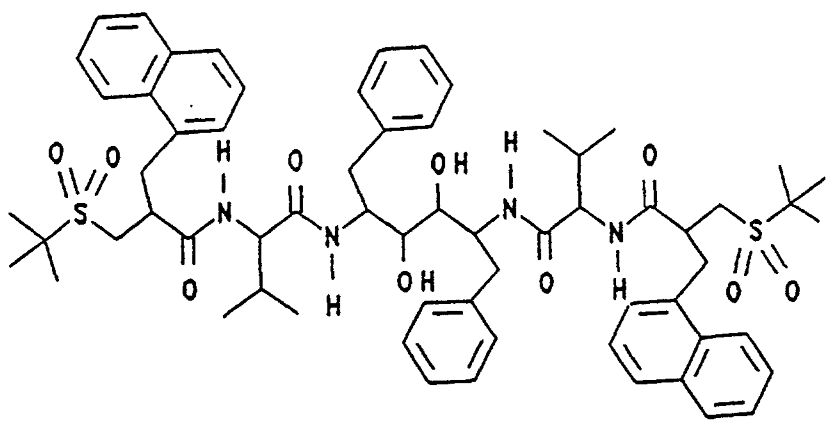

EP 0428 849 A2 F. 15

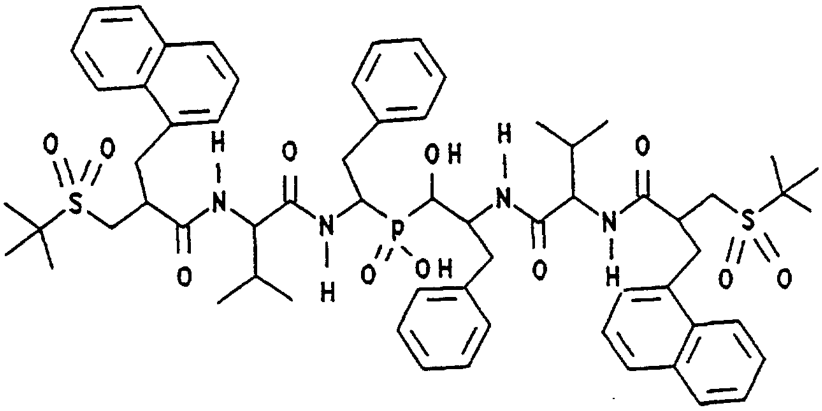

P 42 20566.2 F. 16

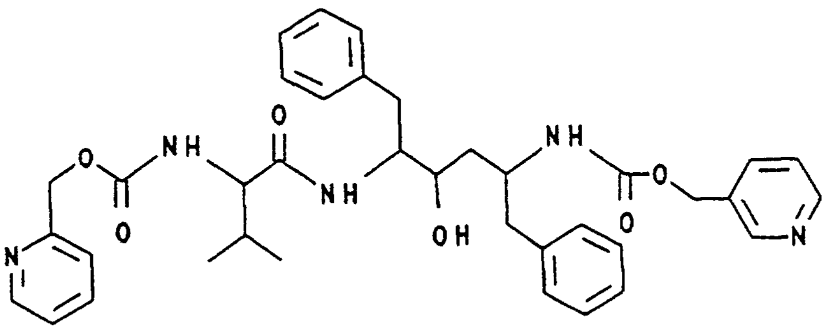

F. 17

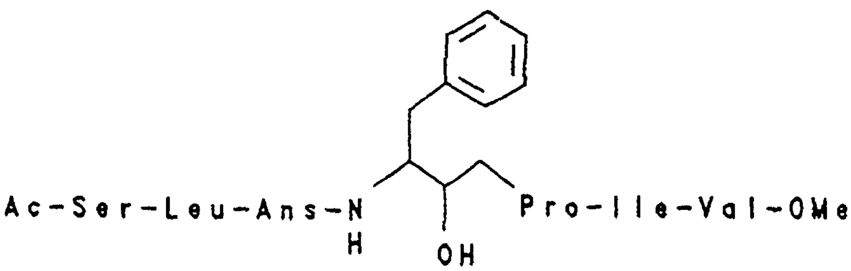

Proc. Natl. Acad. Sci. USA, 87, 8805, (1990) F. 18

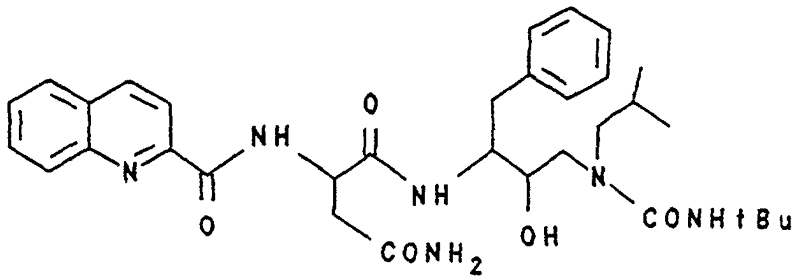

F. 19

a 1, 2 oder 3 bedeutet und $R^5$ für einen Rest der Formel III, steht

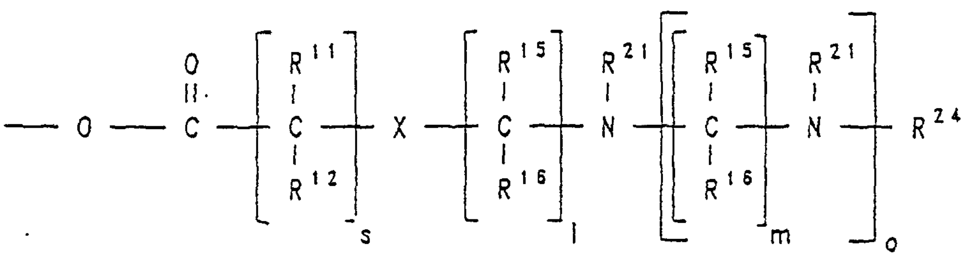

(III),

X steht für O, S, $NR^{20}$ oder $N^+(R^{20})_2$, bevorzugt für S oder $NR^{20}$,

wobei

| | |
|---|---|
| $R^{20}$ | H, $(C_1-C_6)$-Alkyl, $(C_3-C_7)$-Cycloalkyl, $-CH_2-(CH_2)_n-NR^{21}R^{24}$, $-CH_2-C(O)-R^{28}$, $CH_2CONH$ $(C_1-C_6)$-Alkyl, das mit bis zu 5 OH-Gruppen substituiert sein kann, $COO(C_1-C_6)$-Alkyl, oder $-CH_2-P(O)((C_1-C_4)\text{-Alkyl})_2$ bedeutet, bevorzugt H, $(C_1-C_4)$-Alkyl, $-CH_2-C(O)-R^{28}$ oder $-CH_2-P(O)Me_2$, besonders bevorzugt H, $(C_1-C_4)$-Alkyl bedeutet, |
| $R^{11}$, $R^{12}$, $R^{15}$ und $R^{16}$ | bedeuten unabhängig voneinander H oder $(C_1-C_4)$-Alkyl, bevorzugt H oder Methyl, |
| $R^{21}$ | bedeutet H, $(C_1-C_6)$-Alkyl, $(C_3-C_7)$-Cycloalkyl oder $(C_1-C_4)$-Alkoxycarbonyl, bevorzugt H, $(C_1-C_4)$-Alkyl, |
| $R^{24}$ | bedeutet H, $(C_1-C_{18})$-Alkyl, $(C_3-C_{14})$-Cycloalkyl, $(C_2-C_{18})$-Alkenyl, $(C_2-C_{18})$-Alkinyl oder $(C_6-C_{14})$-Aryl, die jeweils einfach, zweifach oder dreifach durch $(C_6-C_{14})$-Aryl, $(C_6-C_{14})$-Aryloxy, $(C_3-C_{14})$-Cycloalkyl, Hydroxy, $(C_1-C_4)$-Alkoxy, $-C(O)-R^{28}$, $P(O)((C_1-C_4)\text{-Alkyl})_2$ oder Halogen substituiert sein können und direkt oder gegebenenfalls über CO mit $NR^{21}$ verknüpft sind, oder eine über die Carbonylgruppe verknüpfte α-Aminosäure, oder $R^{24}$ bedeutet $((C_1-C_4)\text{-Alkyl})-N((C_1-C_4)\text{-Alkyl})-(C_1-C_4)\text{-Alkyl}-NH-((C_1-C_4)\text{-Alkyl})$ oder $R^{24}$ bildet mit $R^{21}$ einen Heterocyclus, bevorzugt bedeutet |
| $R^{24}$ | H, $(C_1-C_4)$-Alkyl, das gegebenenfalls durch Phenyl, $NH_2$ oder $(C_3-C_7)$-Cycloalkyl substituiert sein kann und direkt oder gegebenenfalls über CO mit $NR^{21}$ verknüpft ist, eine natürliche, über die Carbonylgruppe verknüpfte α-Aminosäure, $-CH_2C(O)R^{28}$, $-CH_2-P(O)Me_2$, $-(CH_2)_3N(Me)(CH_2)_2NHMe$, besonders bevorzugt bedeutet $R^{24}$ H, oder $(C_1-C_4)$-Alkyl, |
| $R^{28}$ | steht für OH, $O-(C_1-C_6)$-Alkyl, $O-(C_3-C_6)$-Cycloalkyl, $NH_2$, $-NH-(C_1-C_6)$-Alkyl, das mit bis zu 5 OH-Gruppen substituiert sein kann, $-NH-(C_3-C_7)$-Cycloalkyl oder $-N\text{-bis}(C_1-C_4)$-Al- |

kyl, bevorzugt steht und $R^{28}$ für OH, $NH_2$, $O(C_1-C_4)$-Alkyl, -NH-$(C_1-C_4)$-Alkyl, oder -N-bis $(C_1-C_4)$-Alkyl, besonders bevorzugt steht $R^{28}$ unabhängig voneinander für OH, $NH_2$ oder $O(C_1-C_4)$-Alkyl,

worin

- l 2 oder 3, bevorzugt 2,
- s 1, 2,3, 4 oder 5, bevorzugt 1, 4 oder 5, besonders bevorzugt 1,
- m 0, 1, 2, 3 oder 4, bevorzugt 2 oder 3,
- n 1, 2 oder 3, bevorzugt 1 oder 2,
- o 0, 1, 2 oder 3, bevorzugt 0, 1 oder 2, besonders bevorzugt 0,

Besonders bevorzugte Reste $R^5$, mit denen die OH-Gruppen der obengenannten Verbindungen derivatisiert sind, sind die folgenden Reste:

$R^5$ ist wie oben definiert, und $R^{5*}$ kann unabhängig von $R^5$ die oben für $R^5$ genannten Bedeutungen haben oder Wasserstoff oder Hydroxyl sein.

Besonders bevorzugte Reste $R^5$, mit denen die OH-Gruppen der obengenannten Verbindungen derivatisiert sind, sind die folgenden Reste:

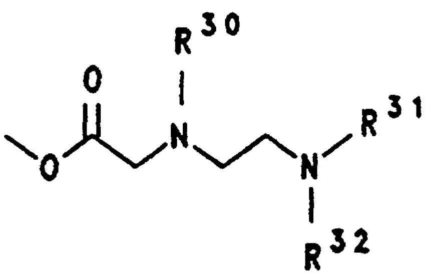

I.1.

| | $R^{30}$ | $R^{31}$ | $R^{32}$ |
|---|---|---|---|
| I.1.1 | H | H | H |
| I.1.2 | Methyl | H | H |
| I.1.3 | Ethyl | H | H |
| I.1.4 | H | Methyl | H |
| I.1.5 | H | Ethyl | H |
| I.1.6 | H | Propyl | H |
| I.1.7 | H | Isopropyl | H |
| I.1.8 | H | Butyl | H |
| I.1.9 | H | 2-Butyl | H |
| I.1.10 | H | t-Butyl | H |
| I.1.11 | H | Cyclohexylmethyl | H |
| I.1.12 | H | 2-Cyclohexylethyl | H |
| I.1.13 | H | Benzyl | H |
| I.1.14 | Methyl | Methyl | H |
| I.1.15 | Ethyl | Ethyl | H |
| I.1.16 | Propyl | Propyl | H |

(fortgesetzt)

| | $R^{30}$ | $R^{31}$ | $R^{32}$ |
|---|---|---|---|
| I.1.17 | Butyl | Butyl | H |
| I.1.18 | Methyl | Ethyl | H |
| I.1.19 | Methyl | Propyl | H |
| I.1.20 | Methyl | Isopropyl | H |
| I.1.21 | Methyl | Butyl | H |
| I.1.22 | Methyl | 2-Butyl | H |
| I.1.23 | Methyl | t-Butyl | H |
| I.1.24 | Methyl | Cyclohexylmethyl | H |
| I.1.25 | Methyl | 2-Cyclohexylethyl | H |
| I.1.26 | Methyl | Benzyl | H |
| I.1.27 | H | Methyl | Methyl |
| I.1.28 | H | Ethyl | Ethyl |
| I.1.29 | Methyl | Methyl | Methyl |
| I.1.30 | Ethyl | Ethyl | Ethyl |
| I.1.31 | Methyl | $-(CH_2)_3N(Me)CH_2CH_2NHMe$ | Methyl |
| I.1.32 | $-CH_2POMe_2$ | Methyl | H |
| I.1.33 | Methyl | $-CH_2POMe_2$ | H |
| I.1.34 | $-CH_2CO_2tBu$ | Methyl | H |
| I.1.35 | $-CH_2CO_2H$ | Methyl | H |
| I.1.36 | $-CH_2CO_2tBu$ | H | H |
| I.1.37 | $-CH_2CO_2H$ | H | H |
| I.1.38 | Methyl | $-CH_2CO_2tBu$ | H |
| I.1.39 | Methyl | $-CH_2CO_2H$ | H |
| I.1.40 | H | $-CH_2CO_2tBu$ | H |
| I.1.41 | H | $-CH_2CO_2H$ | H |
| I.1.42 | Methyl | Glycyl | Methyl |
| I.1.43 | Methyl | Glycyl | H |
| I.1.44 | H | Glycyl | H |
| I.1.45 | H | Glycyl | Methyl |
| I.1.46 | Methyl | Arginyl | Methyl |
| I.1.47 | Methyl | Arginyl | H |
| I.1.48 | H | Arginyl | H |
| I.1.49 | H | Arginyl | Methyl |
| I.1.50 | Methyl | Glutamyl | Methyl |
| I.1.51 | Methyl | Glutamyl | H |
| I.1.52 | H | Glutamyl | H |
| I.1.53 | H | Glutamyl | Methyl |
| I.1.54 | Methyl | Lysyl | Methyl |

(fortgesetzt)

| | $R^{30}$ | $R^{31}$ | $R^{32}$ |
|---|---|---|---|
| I.1.55 | Methyl | Lysyl | H |
| I.1.56 | H | Lysyl | H |
| I.1.57 | H | Lysyl | Methyl |
| I.1.58 | Methyl | Asparagyl | Methyl |
| I.1.59 | Methyl | Asparagyl | H |
| I.1.60 | H | Asparagyl | H |
| I.1.61 | H | Asparagyl | Methyl |
| I.1.62 | Methyl | Sarcosyl | Methyl |
| I.1.63 | Methyl | Sarcosyl | H |
| I.1.64 | H | Sarcosyl | H |
| I.1.65 | H | Sarcosyl | Methyl |
| I.1.66 | Methyl | Acetyl | H |
| I.1.67 | H | Acetyl | H |
| I.1.68 | Methyl | Propionyl | H |
| I.1.69 | H | Propionyl | H |
| I.1.70 | Methyl | Butanoyl | H |
| I.1.71 | H | Butanoyl | H |
| I.1.72 | Methyl | Pivaloyl | H |
| I.1.73 | H | Pivaloyl | H |
| I.1.74 | Methyl | Pentanoyl | H |
| I.1.75 | H | Pentanoyl | H |
| I.1.76 | Methyl | Hexanoyl | H |
| I.1.77 | H | Hexanoyl | H |
| I.1.78 | Methyl | Benzoyl | H |
| I.1.79 | H | Benzoyl | H |
| I.1.80 | Methyl | Phenylacetyl | H |
| I.1.81 | H | Phenylacetyl | H |
| I.1.82 | Methyl | 3-Phenylpropionyl | H |
| I.1.83 | H | 3-Phenylpropiony) | H |
| I.1.84 | Methyl | 3,3-Dimethylbutanoyl | H |
| I.1.85 | H | 3,3-Dimethylbutanoyl | H |

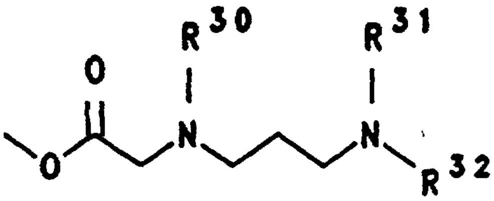

I.2.

| | $R^{30}$ | $R^{31}$ | $R^{32}$ |
|---|---|---|---|
| I.2.1 | H | 2-(Methylamino)ethyl | Methyl |
| I.2.2 | H | 2-(Dimethylamino)ethyl | Methyl |
| I.2.3 | H | $-(CH_2)_2N(Me)(CH_2)_3NH_2$ | Methyl |
| I.2.4 | H | H | H |
| I.2.5 | Methyl | H | H |
| I.2.6 | Ethyl | H | H |
| I.2.7 | H | Methyl | H |
| I.2.8 | H | Ethyl | H |
| I.2.9 | H | Propyl | H |
| I.2.10 | H | Isopropyl | H |
| I.2.11 | H | Butyl | H |
| I.2.12 | H | 2-Butyl | H |
| I.2.13 | H | t-Butyl | H |
| I.2.14 | H | Cyclohexylmethyl | H |
| I.2.15 | H | 2-Cyclohexylethyl | H |
| I.2.16 | H | Benzyl | H |
| I.2.17 | Methyl | Methyl | H |
| I.2.18 | Ethyl | Ethyl | H |
| I.2.19 | Propyl | Propyl | H |
| I.2.20 | Butyl | Butyl | H |
| I.2.21 | Methyl | Ethyl | H |
| I.2.22 | Methyl | Propyl | H |
| I.2.23 | Methyl | Isopropyl | H |
| I.2.24 | Methyl | Butyl | H |
| I.2.25 | Methyl | 2-Butyl | H |
| I.2.26 | Methyl | t-Butyl | H |
| I.2.27 | Methyl | Cyclohexylmethyl | H |
| I.2.28 | Methyl | 2-Cyclohexylethyl | H |
| I.2.29 | Methyl | Benzyl | H |
| I.2.30 | H | Methyl | Methyl |
| I.2.31 | H | Ethyl | Ethyl |
| I.2.32 | Methyl | Methyl | Methyl |
| I.2.33 | Ethyl | Ethyl | Ethyl |
| I.2.34 | $-CH_2CO_2tBu$ | Methyl | H |
| I.2.35 | $-CH_2CO_2H$ | Methyl | H |
| I.2.36 | $-CH_2CO_2tBu$ | H | H |
| I.2.37 | $-CH_2CO_2H$ | H | H |
| I.2.38 | Me | $-CH_2CO_2tBu$ | H |

(fortgesetzt)

| | $R^{30}$ | $R^{31}$ | $R^{32}$ |
|---|---|---|---|
| I.2.39 | Me | $-CH_2CO_2H$ | H |
| I.2.40 | H | $-CH_2CO_2tBu$ | H |
| I.2.41 | H | $-CH_2CO_2H$ | H |
| I.2.42 | Methyl | Glycyl | Methyl |
| I.2.43 | Methyl | Glycyl | H |
| I.2.44 | H | Glycyl | H |
| I.2.45 | H | Glycyl | Methyl |
| I.2.46 | Methyl | Arginyl | Methyl |
| I.2.47 | Methyl | Arginyl | H |
| I.2.48 | H | Arginyl | H |
| I.2.49 | H | Arginyl | Methyl |
| I.2.50 | Methyl | Glutamyl | Methyl |
| I.2.51 | Methyl | Glutamyl | H |
| I.2.52 | H | Glutamyl | H |
| I.2.53 | H | Glutamyl | Methyl |
| I.2.54 | Methyl | Lysyl | Methyl |
| I.2.55 | Methyl | Lysyl | H |
| I.2.56 | H | Lysyl | H |
| I.2.57 | H | Lysyl | Methyl |
| I.2.58 | Methyl | Asparagyl | Methyl |
| I.2.59 | Methyl | Asparagyl | H |
| I.2.60 | H | Asparagyl | H |
| I.2.61 | H | Asparagyl | Methyl |
| I.2.62 | Methyl | Sarcosyl | Methyl |
| I.2.63 | Methyl | Sarcosyl | H |
| I.2.64 | H | Sarcosyl | H |
| I.2.65 | H | Sarcosyl | Methyl |
| I.2.66 | Methyl | Acetyl | H |
| I.2.67 | H | Acetyl | H |
| I.2.68 | Methyl | Propionyl | H |
| I.2.69 | H | Propionyl | H |
| I.2.70 | Methyl | Butanoyl | H |
| I.2.71 | H | Butanoyl | H |
| I.2.72 | Methyl | Pivaloyl | H |
| I.2.73 | H | Pivaloyl | H |
| I.2.74 | Methyl | Pentanoyl | H |
| I.2.75 | H | Pentanoyl | H |
| I.2.76 | Methyl | Hexanoyl | H |

(fortgesetzt)

| | $R^{30}$ | $R^{31}$ | $R^{32}$ |
|---|---|---|---|
| I.2.77 | H | Hexanoyl | H |
| I.2.78 | Methyl | Benzoyl | H |
| I.2.79 | H | Benzoyl | H |
| I.2.80 | Methyl | Phenylacetyl | H |
| I.2.81 | H | Phenylacetyl | H |
| I.2.82 | Methyl | 3-Phenylpropionyl | H |
| I.2.83 | H | 3-Phenylpropionyl | H |
| I.2.84 | Methyl | 3,3-Dimethylbutanoyl | H |
| I.2.85 | H | 3,3-Dimethylbutanoyl | H |

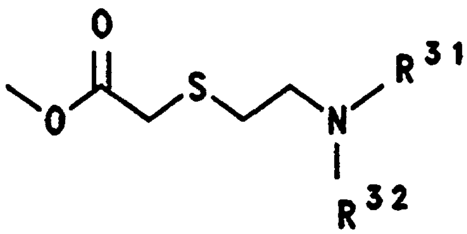

I.3.

| | $R^{31}$ | $R^{32}$ |
|---|---|---|
| I.3.1 | H | H |
| I.3.2 | Methyl | H |
| I.3.3 | Ethyl | H |
| I.3.4 | Propyl | H |
| I.3.5 | Isopropyl | H |
| I.3.6 | Butyl | H |
| I.3.7 | 2-Butyl | H |
| I.3.8 | t-Butyl | H |
| I.3.9 | Cyclohexylmethyl | H |
| I.3.10 | 2-Cyclohexylethyl | H |
| I.3.11 | Benzyl | H |
| I.3.12 | Methyl | Methyl |
| I.3.13 | Ethyl | Ethyl |
| I.3.14 | $-CH_2CO_2tBu$ | H |
| I.3.15 | $-CH_2CO_2H$ | H |
| I.3.16 | Glycyl | H |
| I.3.17 | Glycyl | Methyl |
| I.3.18 | Arginyl | H |
| I.3.19 | Arginyl | Methyl |
| I.3.20 | Glutamyl | H |

(fortgesetzt)

| | $R^{31}$ | $R^{32}$ |
|---|---|---|
| I.3.21 | Glutamyl | Methyl |
| I.3.22 | Lysyl | H |
| I.3.23 | Lysyl | Methyl |
| I.3.24 | Asparagyl | H |
| I.3.25 | Asparagyl | Methyl |
| I.3.26 | Sarcosyl | H |
| I.3.27 | Sarcosyl | Methyl |
| I.3.28 | Acetyl | H |
| I.3.29 | Propionyl | H |
| I.3.30 | Butanoyl | H |
| I.3.31 | Pivaloyl | H |
| I.3.32 | Pentanoyl | H |
| I.3.33 | Hexanoyl | H |
| I.3.34 | Benzoyl | H |
| I.3.35 | Phenylacetyl | H |
| I.3.36 | 3-Phenylpropionyl | H |
| I.3.37 | 3,3-Dimethylbutanoyl | H |

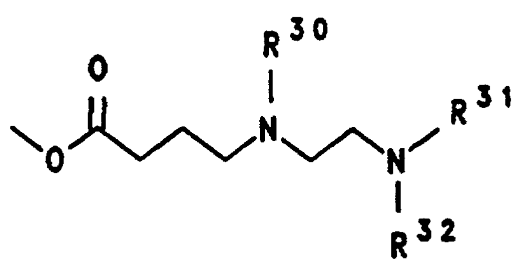

I.4.

| | $R^{30}$ | $R^{31}$ | $R^{32}$ |
|---|---|---|---|
| I.4.1 | H | H | H |
| I.4.2 | H | Methyl | H |
| I.4.3 | H | Ethyl | H |
| I.4.4 | Methyl | Methyl | H |
| I.4.5 | Ethyl | Ethyl | H |
| I.4.6 | H | Methyl | Methyl |
| I.4.7 | H | Ethyl | Ethyl |
| I.4.8 | Methyl | Methyl | Methyl |
| I.4.9 | Ethyl | Ethyl | Ethyl |

Besonders bevorzugt sind die erfindungsgemäßen Verbindungen, in denen $W(OH)_a$ F.15 bedeutet, das einfach oder zweifach substituiert durch einen Rest der Formel I.1., worin

$R^{30}$ Wasserstoff, $C_1$-$C_4$-Alkyl, $CH_2P(O)Me_2$, $CH_2$-$CO_2H$ oder $CH_2CO_2$t.but,
$R^{31}$ Wasserstoff, $C_1$-$C_4$-Alkyl, gegebenenfalls substituiert mit $NH_2$ oder NH($C_1$-$C_3$)-Alkyl; Arg, Gly, Sar, Lys, Pro, His, Trp, -$(CH_2)_3$-N(Me)$CH_2$-$CH_2$NHMe, $CH_2P(O)Me_2$, $CH_2CO_2$t.Bu oder $CH_2CO_2H$ und
$R^{32}$ Wasserstoff oder $C_1$-$C_4$-Alkyl bedeuten,

oder einfach oder zweifach durch einen Rest der Formel I.2. substituiert ist,
worin

$R^{30}$ Wasserstoff oder $C_1$-$C_3$-Alkyl,
$R^{31}$ Wasserstoff, $C_1$-$C_3$-Alkyl, 2(Methylamino)-ethyl, Arg oder -$(CH_2)_2$N(Me)$(CH_2)_3$-$NH_2$ und
$R^{32}$ Wasserstoff oder $C_1$-$C_3$-Alkyl bedeuten,

oder einfach oder zweifach durch einen Rest der Formel I.3. substituiert ist,
worin

$R^{31}$ Wasserstoff, Gly, Leu, ($C_1$-$C_5$)-Alkanoyl, gegebenenfalls substituiert mit Phenyl oder bis zu 3 Methylgruppen und
$R^{32}$ Wasserstoff oder $C_1$-$C_3$-Alkyl bedeuten,

oder einfach oder zweifach durch einen Rest der Formel I.4. substituiert ist,
worin $R^{30}$ $C_1$-$C_3$-Alkyl, $R^{31}$ $C_1$-$C_3$-Alkyl und $R^{32}$ $C_1$-$C_3$-Alkyl oder Wasserstoff bedeuten.

Beispielhaft seien weiterhin die folgenden Verbindungen der Formel I W-[R5]a genannt, dabei soll W' der zugrunde liegende Enzyminhibitor, sein.

| | |
|---|---|
| 1) a = 1; W' = | F.15, jeweils mit I.1.1 bis I.1.85, I.2.1 bis I.2.85, I.3.1 bis I.3.37 und I.4.1 bis I.4.9 |
| 2) a = 2; W' = | F.15, jeweils mit I.1.1 bis I.1.85, I.2.1 bis I.2.85, I.3.1 bis I.3.37 und I.4.1 bis I.4.9 |
| 3) a = 1; W' = | F.1, F.2, F.4, F.5 (n = 1), F.5 (n = 2), F.7, F.11, F.12, F.16, F.17 und F.19 jeweils |
| mit: | I.1.2, I.1.8, I.1.14, I.1.15, I.1.16, I.1.17, I.1.29, I.1.31, I.1.55, I.1.66, I.1.71, I.1.83, I.1.85, I.2.17, I.2.18, I.2.44, I.2.45, I.2.70, I.2.71, I.2.82, I.2.83, I.3.2, I.3.3, I.3.4, I.3.16, I.3.30, I.3.36, I.3.37, I.4.4, I.4.5, I.4.8, I.4.9 |
| 4) a = 2; W' = | F.3, F.4, F.5 (n = 2), F.12 jeweils |
| mit: | I.1.2, I.1.14, I.1.15, I.1.16, I.1.17, I.1.31, I.1.55, I.1.66, I.1.71, I.1.82, I.1.84, I.2.17, I.2.18, I.2.44, I.2.45, I.2.70, I.2.71, I.2.82, I.2.83, I.3.2, I.3.3, I.3.4, I.3.16, I.3.30, I.3.36, I.3.37, I.4.4, I.4.5, I.4.8, I.4.9 |
| 5) a = 3; W' = | F.8 jeweils |
| mit: | I.1.2, I.1.14, I.1.15, I.1.16, I.1.17, I.1.31, I.1.55, I.1.66, I.1.71, I.1.82, I.1.84, I.2.17, I.2.18, I.2.44, I.2.45, I.2.70, I.2.71, I.2.82, I.2.83, I.3.2, I.3.3, I.3.4, I.3.16, I.3.30, I.3.36, I. 3.37, I.4.4, I.4.5, I.4.8, I.4.9 |

Ganz besonders bevorzugt sind Verbindungen, in denen $W(OH)_a$ die Verbindung der Formel F.15 ist, die einfach oder zweifach substituiert ist durch einen Rest der Formel I.1. oder 1.2 worin

$R^{30}$ H oder ($C_1$-$C_3$)-Alkyl,
$R^{31}$ H oder ($C_1$-$C_3$)-Alkyl, gegebenenfalls substituiert mit $NH_2$ oder NH($C_1$-$C_3$)-Alkyl und
$R^{32}$ H oder $CH_3$

bedeutet.

Zum Erfindungsgegenstand gehören weiterhin die pharmakologisch verträglichen Salze der Verbindungen der Formel I, beispielsweise Hydrochloride, Hydrobromide, Citronate, Tartrate, Ethansulfonate, Fumarate, Glucuronate, Sulfate, Isopropansulfonate, Malonate, Gluconate, Lactate, Methansulfonate, Tosylate, Tartronate, Propansulfonate.

Weiterhin gehört zum Gegenstand der vorliegenden Erfindung ein Verfahren zur Herstellung der Verbindungen der Formel Ia, das dadurch gekennzeichnet ist, daß ein Wirkstoff der Formel W-$(OH)_b$ wobei W wie oben definiert ist und b 1, 2 oder 3 sein kann, aber größer oder gleich a ist
mit

A) einer Verbindung der Formel (X)

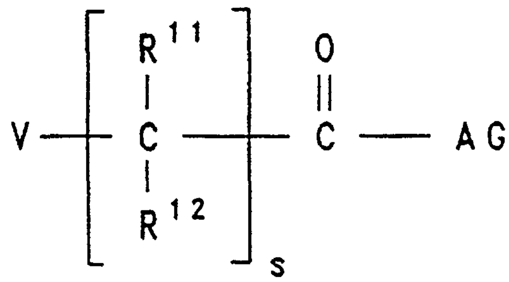

(X)

zu einer Verbindung der Formel (XX)

$$[OH]_{b-a}\text{-W-}[OCO\text{-}(CR^{11},R^{12})_s\text{-}V]_a \qquad (XX)$$

umgesetzt wird
und anschließend
B) die resultierende Verbindung der Formel (XX) ihrerseits mit Nucleophilen der Formeln (XI), umgesetzt wird

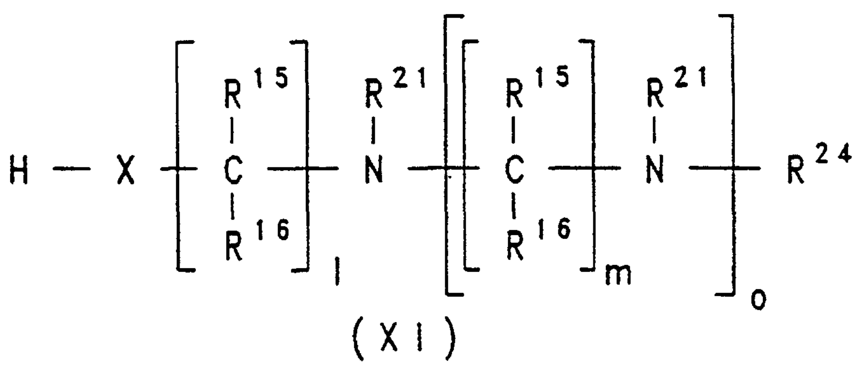

(XI)

wobei W, a, b, X, $R^{11}$, $R^{12}$, $R^{15}$, $R^{16}$, $R^{21}$, $R^{24}$, l, m, n, o, q, r und s die obengenannten Bedeutungen haben, V ist eine geeignete Abgangsgruppe, bevorzugt Br, Cl, OTs, besonders bevorzugt Br,
AG steht für eine zur Veresterung geeignete Abgangsgruppe, bevorzugt Br oder Cl oder eine Gruppe wie sie sich aus den Aktivestermethoden für Carbonsäuren ergibt (Bodanszky, Peptide Research, Bd. 3, 1992, S. 134 bis 139).

Schritt A des obengenannten Verfahrens erfolgt in einem organischem Lösungsmittel wie Acetonitril, THF oder Essigsäureethylester (EE). Die Monoacylierungen erfolgen mit oder bevorzugt ohne Basenkatalyse oder unter Molekularsiebzusatz, die Bisacylierungen werden in Gegenwart einer Base, bevorzugt N-Ethyl-Diisopropylamin und/oder 4-Dimethylaminopyridin (DMAP), oder durch Zusatz von Molekularsieb, vorzugsweise bei -78°C bis 66°C durchgeführt, besonders bevorzugt bei 0 bis 40°C. Die Aufarbeitung erfolgt nach an sich bekannten Verfahren durch Extraktion, Chromatographie, Umkristallisation, Ausfällung, etc.
Schritt B des obengenannten Verfahrens erfolgt ebenfalls in einem organischen Lösungsmittel wie, z. B. THF, EE, Dimethylformamid (DMF) in Acetonitril, bevorzugt bei -78 bis 82°C, besonders bevorzugt bei 0 bis 40°C, ggf. unter Zusatz von Iodid.
Schutzgruppen, die nach der Substitution noch im Molekül enthalten sind, bevorzugt Benzyloxycarbonyl (Z), Methoxytrityl (Mtr), tert.butyloxycarbonyl (BOC) und O-tert.-Butylester (OtBu) werden nach Standardverfahren (Greene, Protective Groups in Organic Synthesis, Wiley 1979) abgespalten. Die Aufarbeitung erfolgt ebenfalls nach an sich bekannten Verfahren durch Extraktion, Chromatographie, Umkristallisation, Ausfällung, etc.
Die Herstellung der Verbindungen der Formeln 1 bis 15 erfolgt gemäß den dazu zitierten Quellenangaben.
Die Verbindungen der Formeln XI bis XIII sind käuflich (z. B. Aldrich Chemie GmbH & Co. KG, Steinheim)

oder können nach allgemein bekannten Methoden synthetisiert werden, z. B. gemäß Beilstein, Handbuch der organischen Chemie, Bd. IV sowie Ergänzugswerke, Springer Verlag Berlin, Heidelberg, New York oder Houben Weyl, Methoden der organischen Chemie, Bd. 11/1, 11/2 und E 16d2, Thieme Verlag Stuttgart,
Helv. Chim. Act. 74, 654 ff. (1991),
J. Med. Chem 34, 2, 269 ff. (1991)
oder Greence a.a.O. bzw. gemäß der in den vorgenannten Dokumenten zitierte Literatur.

Aufgrund ihrer Eigenschaften sind die erfindungsgemäßen neuen Verbindungen besonders für die Verabreichung in Form von sterilen wässrigen Lösungen mittels intravenöser Injektion, intravenöser Infusion oder intramuskulärer oder subkutaner Injektion oder für die topische Anwendung am Auge geeignet. Wegen ihrer guten Löslichkeit bei sauren pH-Werten sind diese Prodrugs auch nützlich für die perorale Verabreichung, z. B. zur Verbesserung der Bioverfügbarkeit von gering wasserlöslichen Ausgangs-Wirkstoffen, die eine Hydroxyl-Gruppe enthalten. Des weiteren sind die neuen Prodrugs aufgrund der Löslichkeits- und Stabilitätseigenschaften sehr gut für die rektale oder dermale Verabreichung geeignet.

Die Lipophilie der Prodrug-Derivate der Formel I kann durch die entsprechende Auswahl der Substituenten in den Verbindungen leicht modifiziert oder kontrolliert werden, sowohl im Hinblick auf die Aminbasizität und dementsprechend dem Grad der Ionisierung bei physiologischen pH-Werten, als auch hinsichtlich der Hydrophobie der Substituenten. Daher können Prodrug-Derivate der Formel I gewählt wird, die durch eine Kombination von verbesserter Wasserlöslichkeit und Lipophilie in der Lage sind, einen verbesserten Biomembran-Transport aufzuweisen, so daß eine bessere Bioverfügbarkeit der Ausgangs-Wirkstoffe vom Verabreichungsort aus gegeben ist.

Die Verbindungen gemäß Formel I können zur Behandlung jeglicher Erkrankungen eingesetzt werden, für die die hydroxylhaltigen Ausgangs-Wirkstoffe, -Medikamente oder -Pharmazeutika nützlich sind. Daher können die Prodrug-Verbindungen der Formel I oral, topisch, parenteral, rektal oder mittels Inhalationsspray in Darreichungsformen oder Formulierungen verabreicht werden, die konventionelle, nicht-toxische, pharmazeutisch akzeptable Trägersubstanzen, Hilfsstoffe und Vehikel enthalten. Die Formulierung und Zubereitung einer jeden aus diesem breiten Spektrum von Darreichungsformen, in die die erfindungsgemäßen Prodrugs eingearbeitet werden können, ist dem Fachmann bekannt. Zusätzliche Informationen können "Remington's Pharmaceutical Sciences", sechzehnte Auflage, 1980, entnommen werden.

Die pharmazeutischen Zusammensetzungen, die den Wirkstoff enthalten, können in einer geeigneten Form vorliegen für die orale Verabreichung, beispielsweise als Tabletten, Pastillen, Lutschbonbons, wässrige Suspensionen oder Lösungen, dispergierbare Pulver oder Granulate, Emulsionen, harte oder weiche Kapseln, Sirup oder Elixier. Für die orale Verabreichung bestimmte Zusammensetzungen können hergestellt werden nach jeder einschlägigen, dem Stand der Technik entsprechenden Methode für die Herstellung pharmazeutischer Zusammensetzungen, und diese Zusammensetzungen können eine oder mehrere Substanzen aus der Gruppe der Süßstoffe, Geschmacksstoffe, Farbstoffe und Konservierungsstoffe enthalten, um eine pharmazeutisch elegante und gut einzunehmende Zubereitung zu erhalten.

Formulierungen für die orale Verabreichung umfassen Tabletten die den Wirkstoff in einer Mischung mit nichttoxischen, pharmazeutisch akzeptablen Trägerstoffen enthalten. Diese Trägerstoffe können beispielsweise inerte Streckmittel sein (wie etwa Calciumcarbonat, Natriumchlorid, Lactose, Kalciumphosphat oder Natriumphosphat), Granulations- oder Aufschlußmittel (zum Beispiel Kartoffelstärke, Alginsäure), Bindemittel (wie etwa Stärke, Gelatine oder Gummi Arabicum) und Schmiermittel (wie etwa Magnesiumstearat, Stearinsäure oder Talkum). Die Tabletten können unbeschichtet sein oder sie können mittels den bekannten Techniken beschichtet sein, um die Auflösung und Resorption im Magen-Darm-Trakt zu verzögern und somit eine anhaltende Wirkung über einen längeren Zeitraum zu bieten. So kann beispielsweise ein zeitverzögemder Stoff wie etwa Glycerylmonostearat oder Glyceryldistearat eingesetzt werden.

Formulierungen für die orale Verabreichung können auch angeboten werden in Form von harten Gelatinekapseln, in denen der Wirkstoff mit einem inerten, festen Streckmittel, beispielsweise Calciumcarbonat, Calciumphosphat oder Kaolin, gemischt ist, oder in Form von weichen Gelatinekapseln, in denen der Wirkstoff mit Wasser oder einem öligen Medium, beispielsweise Erdnußöl, flüssiges Paraffin oder Olivenöl, gemischt ist.

Für die rektale Anwendung der Verbindungen gemäß Formel I dieser Erfindung umfassen typische Dosierungsformen, Suppositorien, rektale Gelatinekapseln (Lösungen und Suspensionen) und Klistiere oder Mikro-Klistiere (Lösungen und Suspensionen). So wird bei einer typischen Formulierung für ein Suppositorium jegliche erfindungsgemäße Verbindung kombiniert mit jeglicher pharmazeutisch akzeptablen Suppositoriums-Basis wie etwa Kakaobutter, veresterte Fettsäuren (C10-C18), glyzerinierte Gelatine, und verschiedene wasserlösliche oder dispergierbare Basis-Substanzen wie etwa Polyethylenglykole und Polyethylensorbitanfettsäureester. Verschiedene Additive wie Salicylate oder Tenside können einbezogen werden. Klistiere oder Mikro-Klistiere des Lösungstyps können einfach mittels Auflösen der erfindungsgemäßen wasserlöslichen Prodrugs in Wasser oder in wasserhaltiger z. B. 0,5 % Methylcellulose oder einer anderen viskositätssteigernden Substanz aufgelöst werden.

Für die topische Anwendung werden die die Prodrug enthaltenden Cremes, Salben, Gels, Lösungen oder

ähnliches nach den als Stand der Technik bekannten Methoden verwendet.

Sterile wässrige Lösungen der Verbindungen von Formel I für die parenterale Verabreichung oder den ophthalmischen Einsatz enthalten auch andere Bestandteile wie Konservierungsmittel, Antioxidantien, Chelatbildner, Puffersubstanzen oder andere Stabilisierungsmittel.

Selbstverständlich variiert das therapeutische Dosierungsspektrum der erfindungsgemäßen Verbindungen je nach Größe und Bedürfnissen der Patienten und den jeweils zu behandelnden Schmerzen oder Krankheitssymptomen. Im allgemeinen kann man jedoch sagen, daß die folgenden Dosierungsrichtlinien ausreichen. Für die orale Verabreichung entspricht die erforderliche therapeutische Dosierung einer erfindungsgemäßen Verbindung auf molekularer Basis derjenigen, die für den hydroxylhaltigen Ausgangswirkstoff benötigt wird. Bei topischer Anwendung dürfte das Auftragen einer erfindungsgemäßen Verbindung in einer Konzentration von 0,01 % bis 5 % (in einem geeigneten topischen Trägermaterial) auf die betroffene Stelle genügen.

Die Menge des Wirkstoffs, der mit Trägersubstanzen kombiniert werden kann, um eine einzige Darreichungsform zu bilden, variiert je nach dem zu behandelnden Wirt und der jeweiligen Verabreichungsart. So kann beispielsweise eine für Menschen bestimmte Formulierung für die orale Verabreichung zwischen 5 mg und 5 g der Wirkstoff-Verbindung mit einer angemessenen und sinnvollen Menge von Trägerstoffen enthalten, die zwischen 5 und 95 % der Gesamtzusammensetzung ausmachen können. Andere Darreichungsformen, wie etwa ophthalmische Darreichungsformen, enthalten einen geringeren Anteil des Wirkstoffs, beispielsweise zwischen 0,1 mg und 5 mg. Dosierungs-Einheiten enthalten im allgemeinen zwischen etwa 0,1 mg und 500 mg des Wirkstoffs.

Es versteht sich, daß die spezifische Dosierung für jeden einzelnen Patienten von einer Vielzahl von Faktoren abhängig ist, einschließlich Wirksamkeit der spezifischen Verbindung, die eingesetzt wird, Alter, Körpergewicht, allgemeiner Gesundheitszustand, Geschlecht, Ernährung, Verabreichungszeit, Verabreichungsweg, Ausscheidungsgeschwindigkeit, Wechselwirkungen mit anderen Arzneimitteln und Schwere der jeweils behandelten Erkrankung.

Beispiele

Beispiel 1

R5 = $OCOCH_2Br$
Bromacetoxy
R5* = OH
W = F.15

1,13 g N,N'-bis-[(2S-(1,1-Dimethylethylsulfonylmethyl)-3-(1-naphthyl)-propionyl) -L-valyl]-2S,5S-diamino-1,6-diphenyl-hexan-3R,4R-diol(R5 = R5* = OH) wurden in 25 ml trockenem THF mit 610 mg Dimethylaminopyridin und einer Lösung aus 400 mg Bromacetylbromid in 1 ml trockenem THF unter Argon bei 0°C in 10 min versetzt. Nach 20 minütigem Rühren bei 0°C ließ man auf RT kommen und entfernte das Lösungsmittel i. Vak. bei RT. Der Rückstand wurde in EE gelöst und mit $KHSO_4$ (3x) und dann mit NaCl-Lösung ausgeschüttelt. Die organische Phase wurde i. Vak. eingeengt und der Rückstand an Kieselgel gereinigt (Toluol:EE 1:1 auf 2:1).
Ausbeute: 340 mg
NMR(270MHz, CDCl3): 0,57(d, 7Hz, 3H); 0,69-0,80 (m, 9H); 1,23 (s, 9H); 1,33 (s, 9H); 2,07 (m, 2H); 2,56 (dd, 14Hz, 8Hz, 1H); 2,70-2,97 (m, 3H); 3,09 (dd, 14Hz, 2Hz, 1H); 3,17 (dd, 14Hz, 3Hz, 1H); 3,28-3,60 (m, 9H); 3,75-3,90 (m, 3H); 4,08 (s, 2H); 4,25 (m, 1H); 4,58 (m, 1H); 4,88 (d, 9Hz, 1H); 5,68 (d, 8Hz, 1H); 5,86 (d, 8Hz, 1H); 6,09 (d, 8Hz, 1H); 6,19 (d, 10Hz, 1H); 6,98 (m, 2H); 7,08-7,44 (m, 12H); 7,52 (m, 2H); 7,63 (m, 2H); 7,74-7,94 (m, 4H); 8,09 (d, 8Hz, 1H); 8,15 (d, 8Hz, 1H)
MS(FAB): [1253.7, 1251.7](M+H)$^{\oplus}$,1235.6, 1233.6

**Beispiel 2**

R5 = $OCOCH_2Br$
Bromacetoxy
R5* = R5
W = F.15

Methode A:

10 g N,N'-bis-[(2S-(1,1-Dimethylethylsulfonylmethyl)-3-(1-naphthyl)-propionyl)-L-valyl]-2S,5S-diamino-1,6-diphenyl-hexan-3R,4R-diol (R5 = R5* = OH) wurden in 100 ml Toluol suspendiert und mit 3,2 g DMAP in 10 ml Toluol versetzt. Unter Rühren wurde bei -20°C 10,7 g Bromacetylbromid in 10 ml Toluol zugetropft. Man gab 70 ml Acetonitril hinzu und rührte 7 h bei RT. Die leicht trübe Lösung wurde mit EE verdünnt und mit eiskalter 0,5 N HCI (2x), $NaHCO_3$- (2x) und NaCl-Lösung gewaschen, über wasserfreiem $MgSO_4$ getrocknet und i. Vak. eingeengt. Man reinigte an Kieselgel (Toluol:EE 1:1) und erhielt 12,6 g Produkt.

Methode B:

5 g N,N'-bis-[(2S-(1,1-Dimethylethylsulfonylmethyl)-3-(1-naphthyl)-propionyl)-L-valyl]-2S,5S-diamino-1,6-diphenyl-hexan-3R,4R-diol (R5 = R5* = OH) wurden mit 2,5 g trockenem und gepulverten Molekularsieb 4Å in 80 ml trockenem EE bei RT gerührt. Man gab langsam 2,35 ml Bromacetylbromid hinzu und rührt 6 h bei RT. Das Molekularsieb wurde abgesaugt und die organische Lösung mit Wasser (2x) und $NaHCO_3$-Lösung gewaschen. Man trocknete über wasserfreiem $MgSO_4$ und entfernte das Lösungsmittel i. Vak.
Der Rückstand wurde mit Toluol aufgenommen und erneut i. Vak. eingeengt. Nach Trocknung erhielt man 6,4 g Produkt. NMR(270MHz, DMSO<D6>): 0,83 (d, 7Hz, 6H); 0,88 (d, 7Hz, 6H); 1,06 (s, 18H); 1,92 (m, 2H); 2,60-2,77 (m, 6H); 3,05 (dd, 10Hz, 14Hz, 2H); 3,21 (dd, 14Hz, 5Hz, 2H); 3,41 (m, 2H); 3,63 (dd, 14Hz, 9Hz, 2H); 4,02-4,15 (m, 2H); 4,20 (m, 2H); 5,07 (m, 2H); 5,14 (s, 2H); 6,98 (m, 2H); 7,12-7,45 (m, 12H); 7,49-7,64 (m, 4H); 7,82 (m, 2H); 7,88-7,98 (m, 4H); 8,20 (d, 8Hz, 4H);.
MS(FAB): [1375.6, 1373.6, 1371.6](M+H)$^{\oplus}$

Beispiel 3

| | |
|---|---|
| R5 = | $OCOCH_2Cl$<br>Chloracetoxy |
| R5* = | OH |
| W = | F.15 |

1,13 g N,N'-bis-[(2S-(1,1-Dimethylethylsulfonylmethyl)-3-(1-naphthyl)-propionyl)-L-valyl]-2S,5S-diamino-1,6-diphenyl-hexan-3R,4R-diol (R5 = R5* = OH) wurden in 25 ml trockenem THF gelöst und mit 610 mg DMAP versetzt. Bei 0°C fügte man tropfenweise 120 µl Chloracetylchlorid in 1 ml THF hinzu und rührte für 10 min bei 0°C. Man ließ auf RT erwärmen, verdünnte mit 100 ml EE und extrahierte mit $KHSO_4$-Lösung. Die EE-Phase wurde über $MgSO_4$ getrocknet, i. Vak. eingeengt, der Rückstand an Kieselgel chromatographiert (EE:Toluol 1:1 auf 1,5:1). Man erhielt 644 mg Monoacylierungsprodukt. NMR(270MHz, CDCl3):0,56 (d, 7Hz, 3H); 0,69-0,80 (m, 9H); 1,23 (s, 9H); 1,34 (5, 9H); 2,06 (m, 2H); 2,54 (dd, 14Hz, 8Hz, 1H); 2,68-2,97 (m, 3H); 3,09 (dd, 14Hz, 2Hz, 1H); 3,18 (dd, 14Hz, 3Hz, 1H); 3,28-3,60 (m, 9H); 3,82 (m, 2H); 4,08 (m, 3H); 4,24 (m, 1H); 4,58 (m, 1H); 4,89 (d, 9Hz, 1H); 5,68 (d, 8Hz, 1H); 5,86 (d, 8Hz, 1H); 6,05 (d, 8Hz, 1H); 6,21 (d, 10Hz, 1H); 6,97 (m, 2H); 7,08-7,44 (m, 12H); 7,53 (m, 2H); 7,63 (m, 2H); 7,74-7,94 (m, 4H); 8,08 (d, 8Hz, 1H); 8,15 (d, 8Hz, 1H).
MS(FAB): [1209.6, 1207.6](M+H)$^{\oplus}$ ; 1191.6, 1189.6

Beispiel 4

| | |
|---|---|
| R5 = | $OCOCH_2Cl$<br>Chloracetoxy |
| R5* = | R5 |
| W = | F.15 |

1,13 g N,N'-bis-[(2S-(1,1-Dimethylethylsulfonylmethyl)-3-(1-naphthyl)-propionyl)-L-valyl]-2S,5S-diamino-1,6-diphenyl-hexan-3R,4R-diol (R5 = R5* = OH) wurden in 25 ml trockenem THF gelöst und mit 1,22 g DMAP versetzt. Bei 0°C fügte man tropfenweise 320 µl Chloracetylchlorid in 5 ml THF hinzu. Man rührte 2 h bei 0°C, fügte weitere 150 µl Chloracetylchlorid hinzu. Nach 30 min entfernte man das THF i. Vak., nahm den Rückstand in EE auf, extrahierte mit $KHSO_4$-Lösung und trocknete über $MgSO_4$. Das Lösungsmittel wurde i. Vak. abdestilliert und der Rückstand an

Kieselgel (EE:Toluol 1:2 auf 1:1) gereinigt. Man erhielt 815 mg Bisacylierungsprodukt.
NMR(270MHz, CDCl3): 0,75 (d, 7Hz, 6H); 0,82 (d, 7Hz, 6H); 1,27 (s, 18H); 1,98 (m, 2H); 2,56 (dd, 8Hz, 14Hz, 2H); 2,84 (dd, 14Hz, 5Hz, 2H); 3,30-3,45 (m, 6H); 3,51 (dd, 13Hz, 7Hz, 2H); 3,93 (s, 4H); 4,07 (m, 2H); 5,00 (m, 2H); 5,18 (s, 2H); 5,95 (d, 9Hz, 2H); 6,07 (d, 8Hz, 2H); 7,07-7,28 (m, 12H); 7,35 (t, 8Hz, 2H); 7,52 (t, 8Hz, 2H); 7,61 (m, 2H); 7,77 (d, 8Hz, 2H); 7,88 (d, 8Hz, 2H); 8,10 (d, 8Hz, 2H).
MS(FAB): [1287.5, 1285.5, 1283.5](M+H)$^{\oplus}$

Beispiel 5

R5 = $OCOC(Me)_2Br$
*a*-Brom-isobutyryloxy
R5* = OH
W = F.15

2,26 g N,N'-bis-[(2S-(1,1-Dimethylethylsulfonylmethyl)-3-(1-naphthyl)-propionyl)-L-valyl]-2S,5S-diamino-1,6-diphenyl-hexan-3R,4R-diol (R5 = R5* = OH) wurden mit 1,48 g DMAP und 2,76 g a-Brom-iso-buttersäurebromid in 10 ml THF für 120 h bei RT gerührt. Man verdünnte mit EE, wusch mit eiskalter 0,5 N HCl (2x), $NaHCO_3$- (2x) und NaCl-Lösung. Man trocknete über wasserfreiem $MgSO_4$, engte i. Vak. ein und reinigte den Rückstand per Chromatographie an Kieselgel (Toluol:EE 4:1 auf 1:1). Man erhielt 1,7 g Produkt.
MS(FAB): [1282.5, 1281.5, 1280.5, 1279.5](M+H)$^{\oplus}$, 1264.5, 1263.5, 1262.5, 1261.5

Beispiel 6

R5 = $OCOCH_2N(Et)(CH_2)_2N(Et)_2$
[N-ethyl-N-(2-diethylaminoethyl)-amino]-acetoxy
R5* = R5
W = F.15

128 mg der Verbindung Beispiel 4 wurden in 2 ml trocknem THF mit 87 mg N,N'-Triethylethylendiamin 16 h bei 50°C gerührt. Die Reaktionslösung wurde mit 50 ml EE aufgenommen und gegen $KHCO_3$/NaCl-Lösung extrahiert, die organische Phase mit $Na_2SO_4$ getrocknet, filtriert und das Lösungsmittel i. Vak. abdestilliert. Das Rohprodukt wurde an Kieselgel gereinigt ($CH_3OH$:Triethylamin 98:2).
Die Ausbeute betrug: 40 mg
MS(FAB) : 1500.0 (M+H)$^{\oplus}$, 1315.8, 750.5

Beispiel 7

R5 = $OCOCH_2N(Et)(CH_2)_2N(Et)_2$
[N-ethyl-N-(2-diethylaminoethyl)-amino]-acetoxy
R5* = OH
W = F.15

121 mg der Verbindung Beispiel 3 wurden analog Beispiel 6 umgesetzt.
Ausbeute: 52 mg
MS(FAB) : 1315.8 (M+H)$^{\oplus}$

Beispiel 8

R5 = $OCOCH_2N(Me)(CH_2)_2N(Me)COOCMe_3$
[N-methyl-N-{2-(N-tert.-butoxycarbonyl-N-methyl)-aminoethyl}-amino]-acetoxy

R5* = OH
W = F.15

121 mg der Verbindung Beispiel 3 wurden in 1 ml trockenem THF mit 113 mg N-(t-Butoxycarbonyl)-N,N'-dimethyl-ethylendiamin (Beispiel 8-a) bei RT versetzt. Man rührte 16 h bei 50°C. Das THF wurde abdestilliert, der Rückstand in EE aufgenommen und 2x mit $KHCO_3$-Lösung ausgeschüttet, mit $MgSO_4$ getrocknet und i. Vak. eingeengt. Gereinigt wurde an Kieselgel (DCM:$CH_3OH$ 98:2 auf 90:10).Die Ausbeute betrug 95 mg
MS(FAB): 1359.7 $(M+H)^{\oplus}$

a) Herstellung von N-(tert.-Butoxycarbonyl)-N,N'-dimethyl-ethylendiamin

8,8 g N,N'-Dimethylethylendiamin wurden in 50 ml Ether gelöst und auf 0°C gekühlt. Man tropfte langsam 18,5 g BOC-ON [2-(tert.-Butoxycarbonyl-oxyimino)-2-phenylacetonitril] hinzu und rührte 16 h bei RT. Man gab 6 g Natriummethylat hinzu und rührte weitere 18 h bei RT. Die ausgefallene Substanz wurde abgesaugt und mit Ether gewaschen. Die vereinigte Etherlösung wurde i. Vak. eingeengt, der Rückstand destilliert (Kp 58-60°C/0,5 mbar).
Man erhielt 11,3 g als farbloses Öl.
MS(Cl): 189 $(M+H)^{\oplus}$, 133

Beispiel 9

R5 = $OCOCH_2N(Me)(CH_2)_2NHMe$
[N-methyl-N-(2-methylaminoethyl)-amino]-acetoxy (als HCl-Salz)
R5* = OH
W = F.15

75 mg der Verbindung Beispiel 8 wurden mit 3 ml 5 N HCl in Dimethoxyethan versetzt und bei RT 1 h gerührt. Dann wurde das Lösungsmittel i. Vak. abdestilliert und der ölige Rückstand mehrmals mit Ether verrieben.
Ausbeute: 55 mg
MS(FAB) : 1259.7 $(M+H)^{\oplus}$

Beispiel 10

R5 = $OCOCH_2NH(CH_2)_3NHCOOC(Me)_3$
[N-{3-(N-tert.-butoxycarbonyl)-aminopropyl}-amino]-acetoxy
R5* = OH
W = F.15

121 mg der Verbindung Beispiel 3 wurden mit 104 mg N-(tert.-butoxycarbonyl)-1,3-diaminopropan (Beispiel 10-a) analog Beispiel 8 umgesetzt. Die Reaktionszeit betrug 3 h bei 50°C.
Ausbeute: 69 mg
MS(FAB) : 1345.7 $(M+H)^{\oplus}$

a) Herstellung von N-(tert.-butoxycarbonyl)-1,3-diaminopropan

37 g 1,3 Diaminopropan wurden in 180 ml trockenem Dioxan bei RT mit einer Lösung von 14,7 g Di-tert.-butyl-dicarbonat in 200 ml trockenem Dioxan innerhalb 2 h versetzt. Man rührte 16 h bei RT. Man saugte den ausgefallenen Niederschlag ab und engte das Filtrat i. Vak. ein. Anschließend dampfte man den Rückstand 3x mit je 100 ml Toluol ab. Der ölige Rückstand wurde in 350 ml Wasser suspendiert und vom Ausgefallenen abfiltriert. Das Filtrat wurde 4x mit je 250 ml DCM extrahiert und dann mit $Na_2SO_4$ getrocknet und i. Vak. eingeengt. Die Reinigung erfolgte an Kieselgel ($CH_3OH$).
Ausbeute: 5 g
MS(Cl): 175 $(M+H)^{\oplus}$, 119

Beispiel 11

R5 = $OCOCH_2N(Me)(CH_2)_3N(Me)COOC(Me)_3$
[N-methyl-N-{3-(N-tert.-butoxycarbonyl-N-methyl)-aminopropyl}-amino]-acetoxy
R5* = OH
W = F.15

121 mg der Verbindung Beispiel 3 wurden mit 121 mg N-tert.-butoxycarbonyl-N,N'-dimethyl-1,3-propandiamin (Beispiel 11-a) analog Beispiel 10 umgesetzt. Ausbeute: 52 mg MS(FAB) : 1373.7 (M+H)⊕

a) Herstellung von N-tert.-Butoxycarbonyl-N,N'-dimethyl-1,3-propandiamin

10,2 g N,N'-Dimethyl-1,3-propandiamin wurden in 40 ml trocknem Dioxan mit einer Lösung von 2,79 g Di-tert.-butyl-dicarbonat in 40 ml trocknem Dioxan bei RT innerhalb von 2 h versetzt. Man rührte 16 h bei RT. Dann wurde das Lösungsmittel i. Vak. abdestilliert und 2x mit Toluol abgedampft. Der Rückstand wurde in 60 ml Wasser gelöst und 3x mit je 50 ml EE extrahiert. Die organische Phase wurde dann mit wäßriger $KHCO_3$/NaCl-Lösung gewaschen, mit $Na_2SO_4$ getrocknet und eingeengt.
Ausbeute: 2,14 g
MS(Cl): 203 (M+H)⊕, 147

Beispiel 12

R5 = $OCOCH_2N(Me)(CH_2)_3NHMe$
[N-methyl-N-(3-methylaminopropyl)-amino]-acetoxy (als HCl-Salz)
R5* = OH
W = F.15

40 mg der Verbindung Beispiel 11 wurden analog Beispiel 9 umgesetzt.
Ausbeute: 32 mg
MS(FAB) : 1273.9 (M+H)⊕

Beispiel 13

R5 = $OCOCH_2NH(CH_2)_3NH_2$
[N-(3-aminopropyl)-amino]-acetoxy (als HCl-Salz)
R5* = OH
W = F.15

50 mg der Verbindung Beispiel 10 wurden analog Beispiel 9 umgesetzt. Ausbeute: 35 mg
MS(FAB) : 1245.8 (M+H)⊕

Beispiel 14

R5 = $OCOCH_2N(Me)(CH_2)_3N(Me)COOC(Me)_3$
[N-methyl-N-{3-(N-tert.-butoxycarbonyl-N-methyl)-aminopropyl}-amino]-acetoxy
R5* = R5
W = F.15

193 mg der Verbindung Beispiel 4 wurden mit 364 mg
N-tert.-Butoxycarbonyl-N,N'-dimethyl-1,3-propandiamin (Beispiel 11-a) in 3 ml trockenem THF unter N2 gelöst und 16

h bei 50°C gerührt. Das Lösungsmittel wurde abdestilliert, der Rückstand in EE aufgenommen und mit $KHCO_3$- und $KHSO_4$-Lösung ausgeschüttelt. Die organische Phase wurde mit $Na_2SO_4$ getrocknet und eingeengt. Die Reinigung erfolgte an Kieselgel (EE:$CH_3OH$ 98:2 auf 90:10).
Ausbeute: 183 mg
MS(FAB): 1615.8 $(M+H)^{\oplus}$

Beispiel 15

R5 = $OCOCH_2N(Me)(CH_2)_3NHMe$
[N-methyl-N-(3-methylaminopropyl)-amino]-acetoxy (als HCl-Salz)
R5* = R5
W = F.15

150 mg der Verbindung Beispiel 14 wurden analog Beispiel 9 umgesetzt.
Ausbeute: 121 mg
MS(FAB) : 1415.8 $(M+H)^{\oplus}$

Beispiel 16

R5 = $OCOCH_2N(Me)(CH_2)_2N(Me)COOC(Me)_3$
[N-methyl-N-{2-(N-tert.-butoxycarbonyl-N-methyl)-aminoethyl}-amino]-acetoxy
R5* = R5
W = F.15

3,6 g der Verbindung Beispiel 4 wurden mit 1,24 ml N-Butoxycarbonyl-N,N'-dimethylethylendiamin und 1,06 ml N-Ethyl-diisopropylamin in 40 ml EE für 20 h bei RT gerührt. Man extrahierte mit 5%iger wäßriger Citronensäure-Lösung (2x), Wasser, $NaHCO_3$-Lösung (2x) und NaCl-Lösung. Nach Trocknung über wasserfreiem $MgSO_4$ wurde das Lösungsmittel i. Vak. abdestilliert. Man reinigte mit EE an Kieselgel, das vorher mit 10% Wasser desaktiviert wurde. Man erhielt 3,15 g Produkt.
MS(FAB): 1587.9 $(M+H)^{\oplus}$

Beispiel 17

R5 = $OCOCH_2N(Me)(CH_2)_2NHMe$
[N-methyl-N-(3-methylaminoethyl)-amino]-acetoxy (als HCl-Salz)
R5* = R5
W = F.15

110 mg der Verbindung Beispiel 16 wurden analog Beispiel 9 mit 5 N HCI in Dioxan umgesetzt.
Ausbeute: 85 mg
MS(FAB) : 1387.7 $(M+H)^{\oplus}$

Beispiel 18

R5 = $OCOCH_2N(Me)(CH_2)_2N(Me)(CH_2)_3N(Me)(CH_2)_2NHMe$
[(3,6,10-trimethyl-3,6,10,13-tetraaza-tetradecanoyloxy]
R5* = OH
W = F.15

125 mg der Verbindung Beispiel 1 wurden in 1,5 ml trockenem DMF mit einer Lösung von 29 mg 5,9-Dimethyl-(2,5,9,12-tetraaza)-tridecan (Beispiel 18-a) in 1,5 ml trockenem DMF bei RT versetzt. Anschließend gab man 26 mg N-Ethyl-diisopropylamin zu. Man rührte 5 h bei 50°C. Die Reaktionslösung wurde i. Vak. abdestilliert und der Rückstand in DCM aufgenommen. Es wurde mit wäßriger $KHSO_4$-Lösung 2x ausgeschüttelt und dann.mit gesättigter NaCl-Lösung gewaschen. Die organische Phase wurde mit $MgSO_4$ getrocknet und eingedampft. Der Rückstand wurde mehrmals mit Ether digeriert. Ausbeute: 101 mg
MS(FAB) : 1387.8 $(M+H)^{\oplus}$

a) Herstellung von 5,9-dimethyl-(2,5,9,12-tetraaza)-tridecan (als HCl-Salz)

208 mg 2,12-di-tert.-butoxycarbonyl-5,9-dimethyl-(2,5,9,12-tetraaza)-tridecan (Beispiel 18-b) wurden analog Beispiel 9 umgesetzt.
Ausbeute: 160 mg
NMR(200MHz, DMSO<D6>): 2,15(m, 2H); 2,54(s, 6H); 2,76(s, 6H); 3,1-3,5(m, ca 14H); 9,4(m, br, ca 4H).
MS(CI): 217 $(M+H)^{\oplus}$, 172

b) , Herstellung von 2,12-di-tert.-butoxycarbonyl-5,9-dimethyl-(2,5,9,12-tetraaza)-tridecan

414 mg N-(t-Butoxycarbonyl)-N,N'-dimethyl-ethylendiamin (Beispiel 8-a) wurden in 10 ml trockenem EtOH mit 202 mg 1,3-dibrompropan unter Zusatz von 253 mg N-Ethylmorpholin 6 h unter Rückfluß gehalten. Anschließend wurde das Reaktionsgemisch mit 185 mg Propionylchlorid 30 min. bei RT gerührt und dann mit 10 ml Wasser versetzt. Nach weiteren 30 min. verdünnte man mit EE und extrahierte mit wäßriger $KHSO_4$-Lösung. Die wäßrige Phase stellte man mit festem $NaHCO_3$ auf pH: 7-8 und extrahierte dann 2x mit EE, trochnete die vereinigten organischen Phasen mit $Na_2SO_4$, filtrierte und engte i. Vak. ein. Die Reinigung des Rohproduktes erfolgte an Kieselgel (DCM:MeOH 95:5).
Ausbeute: 225 mg
NMR(200MHz, CDCl3): 1,48(s, 18H); 1,63(quint, 2H); 2,26(s, 6H); 2,33-2,58(m, 8H); 2,88(s, 6H); 3,31(m, 4H).
MS(CI): 417 $(M+H)^{\oplus}$, 317, 272

Beispiel 19

R5 = $OCOCH_2N(Me)(CH_2)_2N(Me)_2$
N-(2-dimethylamino-ethyl)-N-methyl-amino-acetoxy (als HCl-Salz)
R5* = R5
W = F.15

94 mg der Verbindung Beispiel 4 wurden in 2,5 ml trockenem DMF gelöst und bei RT mit 84 mg N,N,N'-Trimethylethylendiamin und einer Spatelspitze KJ versetzt. Man rührte 3d bei RT und 8h bei 60°C. Das Lösungsmittel wurde i. Vak. abdestilliert, der Rückstand in EE aufgenommen, mit gesättigter $KHCO_3$-(2x) und NaCl-Lösung gewaschen, über $MgSO_4$ getrocknet und i. Vak. eingeengt. Nach Chromatographie an Kieselgel (DCM:MeOH 8:2 auf 1:1, dann DCM:MeOH:$NEt_3$ 15:15:1) erhielt man 21 mg Produkt, welches durch Behandeln mit 0,1N ethanolischer HCl ins Hydrochlorid überführt wurde.
MS(FAB): 1415,8 $(M+H)^{\oplus}$

Beispiel 20

R5 = $OCOCH_2NH(CH_2)_3N(Me)(CH_2)_2N(Me)(CH_2)_3NH_2$
7,10-dimethyl-3,7,10,14-tetraaza-tetradecanoyl (als HCl-Salz)
R5* = OH
W = F.15

204 mg der Verbindung Beispiel 3 wurden mit 250 mg N-(3-Aminopropyl)-N'-(3-tert.-butoxycarbonylamino-propyl)-N,N'-dimethyl-ethylendiamin (Beispiel 20-a) in 5 ml Acetonitril und einer Spatelspitze KJ 16 h bei RT gerührt. Man engte i. Vak. ein und verteilte den Rückstand zwischen EE und $KHCO_3$-Lösung. Die organische Phase wurde getrocknet und i. Vak. eingeengt. Chromatographie an Kieselgel (EE:MeOH:Triethylamin 90:10:0,2 auf 80:20:1) lieferte 86 mg noch tert.-butoxycarbonyl-geschütztes Produkt, [MS(FAB): 1474.2 $(M+H)^{\oplus}$], wovon 33 mg durch Behandlung

mit 6N HCl in Dioxan analog Beispiel 9 entschützt und ins Salz überführt wurden. Man erhielt 34 mg Produkt.
MS(FAB): 1373,8 (M+H)⊕

a) Herstellung von N-(3-Aminopropyl)-N'-(3-tert.-butoxycarbonylaminopropyl)-N,N'-dimethyl-ethylendiamin

4,4g N,N'-Dimethyl-ethylendiamin wurden unter Eiskühlung langsam mit 5,3 g Acrylnitril versetzt. Man ließ langsam auf RT erwärmen und rührte 14 h bei RT. Man erhitzte 3 h auf 80°C und entfernte anschließend die flüchtigen Bestandteile i. Vak. bei 80°C Badtemperatur. Man erhielt 9,6 g N,N'-Bis-(2-Cyanoethyl)-N,N'-dimethyl-ethylendiamin.
MS(Cl): 195 (M+H)⊕, 154

9,5 g davon wurden unter Kühlung in 35 ml Eisessig und 35 ml conc. HCl gelöst. Nach Zugabe von 0,4 g $PtO_2$ wurde 24 h bei RT und 5atm $H_2$ hydriert. Nach Abfiltration des Katalysators wurde das Filtrat i. Vak. eingeengt, mehrmals in Ethanol aufgenommen und wieder i. Vak. eingeengt. Der Rückstand wurde dann mit Ethanol verrührt, das Ethanol dekantiert, der Rest i. Vak. getrocknet. Man erhielt N,N'-Bis-(3-Aminopropyl)-N,N'-dimethyl-ethylendiamin als Salz. MS(Cl): 203 (M+H)⊕

9,81 g davon wurden in 200 ml Dioxan/Wasser (1:1) gelöst und bei 0°C mit 141 ml 1N NaOH und anschließend über 2 h mit 3,70 g Di-tert.-butyldicarbonat in 25 ml Dioxan versetzt. Man rührte 1 h bei 0°C, 2 h bei RT. Das Dioxan wurde i. Vak. abdestilliert, der Rückstand zwischen EE und $K_2CO_3$-Lösung verteilt. Die organische Phase wurde getrocknet, filtriert und i. Vak. eingeengt. Man erhielt 4,4 g N-(3-Aminopropyl)-N-(3-tert.-butoxycarbonylamino-propyl)-N, N'-dimethyl-ethylendiamin, das wie folgt gereinigt wurde:

0,7 g davon, gelöst in 30 ml THF, wurden bei 0°C langsam mit 750 mg N-(Benzoxycarbonyloxy)-succinimid in 30 ml THF versetzt. Man rührte 1 h bei 0°C, 1 h bei RT, entfernte das Lösungsmittel i. Vak. und verteilte den Rückstand zwischen EE und $KHCO_3$-Lösung. Die organische Phase wurde getrocknet ($Na_2SO_4$) und i. Vak. eingeengt. Chromatographie an Kieselgel (EE:MeOH 8:2, 1% Triethylamin) lieferte 0.38 g N-(3-tert.-Butoxycarbonylamino-propyl)-N'-(3-carbobenzoxycarbonyl-amino-propyl)-N,N'-di methyl-ethylendiamin.
NMR(200MHz, $CDCl_3$): 1,42 (s, 9H); 1,50-1,70 (m, 4H); 2,1-2,5 (m, 14H); 3,05-3,35 (m, 4H); 5,1 (s,2H); ca. 6,15 (br, 2H); 7,3-7,4 (m, 5H).
MS(Cl): 437 (M+H)⊕, 337

0,36 g davon wurden in 30 ml MeOH gelöst und mit 100 ml Pd(10%ig in Kohle) versetzt. Man rührte 2 h bei RT und 1atm $H_2$. Nach Abfiltration des Katalysators erhielt man 0,25 g gewünschtes Amin.

Beispiel 21

R5 = $OCOCH_2NH(CH_2)_3N(Me)(CH_2)_2N(Me)(CH_2)_3NH_2$
7,10-dimethyl-3,7,10,14-tetraaza-tetradecanoyl (als HCl-Salz)
R5* = R5
W = F.15

Synthese analog Beispiel 20 ausgehend von der Verbindung Beispiel 4.
MS(FAB): 1116,9 (M+H)⊕

Beispiel 22

R5 = $OCOCH_2N(Et)(CH_2)_2NH(Et)$
N-ethyl-N-(2-ethylaminoethyl)-amino-acetoxy (als HCl-Salz)
R5* = OH
W = F.15

Synthese analog Beispiel 24 und 23 aus der Verbindung Beispiel 1.
MS(FAB): 1287,6 (M+H)⊕

Beispiel 23

R5 = $OCOCH_2N(Et)(CH_2)_2N(Et)COOC(Me)_3$
N-[2(N-tert.-Butoxycarbonyl-N-ethyl-amino)-ethyl]-N-ethyl-amino-acetoxy
R5* = R5
W = F.15

Aus 216 mg der Verbindung Beispiel 4 in 5 ml Acetonitril und 272 mg N-tert.-Butoxycarbonyl-N,N'-diethylethylendiamin erhielt man mit einer Spatelspitze KJ nach 2-tägigem Rühren bei RT, üblicher Aufarbeitung und Chromatographie an Kieselgel (EE:Toluol 7:3) 157 mg Produkt.
MS(FAB): [1644.9, 1643,9](M+H)$^{\oplus}$

Beispiel 24

R5 = $OCOCH_2N(Et)(CH_2)_2NH(Et)$
N-ethyl-N-(2-ethylaminoethyl)-amino-acetoxy (als HCl-Salz)
R5* = R5
W = F.15

Synthese analog Beispiel 9 aus der Verbindung Beispiel 23. Die Verbindung fällt als Hydrochlorid an.
MS(FAB): 1443,7 (M+H)$^{\oplus}$

Beispiel 25

R5 = $OCOCH_2NH(CH_2)_3N(Me)(CH_2)_2NH(Me)$
7-methyl-3,7,10-triaza-undecanoyloxy (als HCl-Salz)
R5* = OH
W = F.15

218 mg der Verbindung Beispiel 3 wurden in 5 ml Acetonitril mit 133 mg N-(3-Aminopropyl)-N'-(tert.-butoxycarbonyl)-N,N'-dimethyl-ethylendiamin und einer Spatelspitze KJ 24 h bei RT gerührt. Das Lösungsmittel wurde i. Vak. abdestilliert und der Rückstand in EE aufgenommen. Es wurde 2x mit gesättigter $KHCO_3$-Lösung ausgeschüttelt, die organische Phase mit $Na_2SO_4$ getrocknet und i. Vak. eingedampft. Die Reinigung erfolgte an Kieselgel (EE:MeOH: Triethylamin 90:10:0,2). Das Zielprodukt wurde 2x mit Toluol i. Vak. abgedampft und anschließend mit n-Pentan verrieben und getrocknet. Man erhielt 146 mg Produkt, welches durch Behandlung mit 6N HCl in Dioxan analog Beispiel 9 in das gewünschte Endprodukt überführt wurde. Die Ausbeute betrug 102 mg.
MS(FAB): 1316,6 (M+H)$^{\oplus}$

a) Herstellung von N-(3-Aminopropyl)-N'-(tert.-butoxycarbonyl)-N,N'-dimethyl-ethylendiamin

1,9 g N-(tert.-Butoxycarbonyl)-N'-(2-cyanoethyl)-N,N'-dimethyl-ethylendiamin (Beispiel 25-b) wurden in 100 ml gesättigtem methanolischem Ammoniak gelöst und mit 5 g Raney-Nickel 24 h bei RT und 10atm $H_2$ hydriert. Man filtrierte vom Katalysator ab, der Katalysator wurde mit MeOH gewaschen, das Filtrat i. Vak. eingeengt. Der Rückstand wurde in Toluol aufgenommen, Unlösliches abfiltriert, das Filtrat i. Vak. eingeengt und getrocknet. Man erhielt 1,93 g Produkt. MS(Cl): 246 (M+H)$^{\oplus}$, 190, 146

b) Herstellung von N-(tert.-Butoxycarbonyl)-N'-(2-cyanoethyl)-N,N'-dimethyl-ethylendiamin

1,88 g N-(tert.-Butoxycarbonyl)-N,N'-dimethyl-ethylendiamin wurden unter Eiskühlung langsam mit 530 mg Acrylnitril versetzt. Man rührte 1 h bei 0°C, 14 h bei RT und 2 h bei 60°C. Man entfernte die flüchtigen Bestandteile i. Vak. und reinigte den Rückstand durch Chromatographie an Kieselgel (EE:MeOH 9:1).
Man erhielt 1,92 g Produkt.

MS(Cl): 242 $(M+H)^{\oplus}$, 186, 142

Beispiel 26

R5 = $OCOCH_2NH(CH_2)_3N(Me)(CH_2)_2NH(Me)$
7-methyl-3,7,10-triaza-undecanoyloxy
R5* = R5
W = F.15

Darstellung analog Beispiel 25 aus der Verbindung Beispiel 3.
MS(FAB): 1501,8 $(M+H)^{\oplus}$

Beispiel 29

R5 = $OCOC(Me)_2NH(CH_2)_2N(Me)_2$
a-[2-(Dimethylamino)-ethyl-amino]-isobutyryloxy
R5* = OH
W = F.15

Synthese aus Verbindung Beispiel 3 und N,N-Dimethylethylendiamin in Acetonitril (8h, 50°C), Reinigung durch Chromatographie an Kieselgel (EE:MeOH 9:1, 1% Triethylamin)
MS(FAB): 1287,7 $(M+H)^{\oplus}$; 1197,7

Beispiel 31

R5 = $OCOCH_2S(CH_2)_2NHCO(CH_2)_2C_6H_5$
2-(3-Phenylpropionylamino)-ethyl-thioacetoxy
R5* = OH
W = F.15

125 mg der Verbindung Beispiel 1, 42 mg 2-(3-Phenylpropionylamino)-ethyl-thiol (Beispiel 30-a) und 26 mg N-Ethyl-diisopropylamin wurden 3d bei RT in 2,5 ml trockenem THF gerührt. Man entfernte das Lösungsmittel i. Vak., nahm den Rückstand in EE auf und extrahierte mit NaCl-Lösung (3x), trocknete die organische Phase und engte i. Vak. ein. Chromatographie an Kieselgel (DCM:MeOH 97:3) lieferte das gewünschte Produkt, welches noch mit Ether gewaschen wurde.
Ausbeute: 131 mg
MS(FAB): 1380.6 $(M+H)^{\oplus}$, 1362.5

a) Herstellung von 2-(3-Phenylpropionylamino)-ethyl-thiol

Zu einer Lösung von 5,68 g 2-Mercaptoethylaminhydrochlorid und 10,12 g Triethylamin in 200 ml DCM wurden bei 0°C 6,75 g 3-Phenylpropionsäurechlorid in 10 ml DCM während 20 min zugetropft. Nach beendeter Zugabe wurde noch 40 min bei 0°C nachgerührt. Das Reaktionsgemisch wurde nacheinander mit Wasser, 0,4N HCl, dann wieder mit Wasser gewaschen, über $Na_2SO_4$ getrocknet und i. Vak. eingeengt. Der Rückstand wurde an Kieselgel gereinigt (Toluol:EE 7:3).
Ausbeute: 6,2 g Fp: 59-61°C
MS(DI): 210 $(M+H)^{\oplus}$

Beispiel 32

R5 = $OCOCH_2S(CH_2)_2NHCO(CH_2)_2C_6H_5$
2-(3-Phenylpropionylamino)-ethyl-thio-acetoxy
R5* = R5
W = F.15

Synthese analog Beispiel 31 aus der Verbindung Beispiel 4.
MS(FAB): 1629.7 (M+H)*

Beispiel 33

R5 = $OCOCH_2S(CH_2)_2NHCOCH_2NHCO_2C(Me)_3$
2-(tert. Butoxycarbonylaminomethylcarbonylamino)-ethyl-thio-acetoxy
R5* = OH
W = F.15

Synthese analog Beispiel 31 aus den Verbindungen Beispiel 1 und Beispiel 33-b.
MS(FAB): 1405.7 $(M+H)^{\oplus}$ , 1387.6, 1305.7

a) Herstellung von Bis-[2-(tert. Butoxycarbonylaminomethylcarbonylamino)-ethan]-disulfid

Zu 0,9 g Cystamindihydrochlorid, 2,1 g N-tert.-Butoxycarbonylglycin, 1,62 g HOBT und 3,85 g TBTU in 50 ml absolutem DMF wurden bei 10-15°C 4,14 g N-Ethyl-diisopropylamin innerhalb von 5min zugetropft. Nach beendeter Zugabe wurde noch 2 h bei RT gerührt. Das Lösungsmittel wurde i. Vak. abgedampft, der Rückstand in EE gelöst und 2x mit gesättigter $NaHCO_3$-Lösung, dann mit NaCl-Lösung gewaschen, über $Na_2SO_4$ getrocknet, filtriert und i. Vak. eingeengt. Der Rückstand wurde an Kieselgel gereinigt (EE:MeOH 99:1 auf 95:5).
Ausbeute: 1,8 g
MS(DI): 467 $(M+H)^{\oplus}$, 367, 267

b) Herstellung von 2-(tert. Butoxycarbonylaminomethylcarbonylamino)-ethyl-thiol

Zu 1,0 g Bis-[2-(tert. Butoxycarbonylaminomethylcarbonylamino)-ethan]-disulfid (Beispiel 33-a) in 35 ml MeOH wurden unter Eiskühlung portionsweise 0,33 g NaBH4 zugegeben. Nach beendeter Zugabe wurde noch 30 min bei 0°C, dann 30 min bei RT nachgerührt. Das Reaktionsgemisch wurde i. Vak. eingeengt und der Rückstand in EE aufgenommen. Durch Zugabe von 0,1N HCI wurde vorsichtig auf pH 3 eingestellt, 15 min gerührt, die Phasen getrennt, die organische Phase 2x mit NaCl-Lösung gewaschen, über $Na_2SO_4$ getrocknet, filtriert und i. Vak. eingeengt. Der Rückstand wurde an Kieselgel gereinigt (EE:MeOH 96:4).
Ausbeute: 0,8 g
MS(CI): 235 $(M+H)^{\oplus}$, 135

Beispiel 34

R5 = $OCOCH_2S(CH_2)_2NHCOCH_2NHCO_2C(Me)_3$
2-(tert. Butoxycarbonylaminomethylcarbonylamino)-ethyl-thio-acetoxy
R5* = R5
W = F.15

Synthese analog Beispiel 33 aus den Verbindungen Beispiel 4 und 33-a.
MS(FAB): 1679.7 $(M+H)^{\oplus}$, 1580.0, 1479.9

Beispiel 35

R5 = $OCOCH_2S(CH_2)_2NHCOCH_2NH_2$
2-(Aminomethylcarbonylamino)-ethyl-thio-acetoxy (als HCl-Salz)
R5* = OH

Synthese aus der Verbindung Beispiel 33 analog Beispiel 9.
MS(FAB): 1305.7 $(M+H)^{\oplus}$

Beispiel 36

R5 = $OCOCH_2S(CH_2)_2NHCOCH_2NH_2$
2-(Aminomethylcarbonylamino)-ethyl-thio-acetoxy (als HCl-Salz)
R5* = R5

Synthese aus der Verbindung Beispiel 34 analog Beispiel 9.
MS(FAB): 1479.9 $(M+H)^{\oplus}$

Beispiel 37

R5 = $OCOCH_2S(CH_2)_2NHCO(CH_2)_2CH_3$
2-(Butanoylamino)-ethyl-thioacetoxy
R5* = OH
W = F.15

Synthese analog Beispiel 31 aus den Verbindungen Beispiel 1 und Beispiel 37-a
MS(FAB): 1340.7 $(M+Na)^{\oplus}$, 1318 $(M+H)^{\oplus}$, 1300.6

a) Herstellung von 2-(Butanoylamino)-ethyl-thiol

Zu einer Lösung von 5,68 g 2-Mercaptoethylaminhydrochlorid und 10,12 g Triethylamin in 200 ml DSM wurden bei 0°C 4,26 g Buttersäurechlorid in 10 ml DSM während 20 min zugetropft. Nach beendeter Zugabe wurde noch 40 min bei 0°C nachgerührt. Das Reaktionsgemisch wurde nacheinander mit Wasser, 0,4N HCl, dann wieder mit Wasser gewaschen, über $Na_2SO_4$ getrocknet und i. Vak. eingeengt. Der Rückstand wurde an Kieselgel gereinigt (Toluol:EE 7:3).
Ausbeute: 4,1 g
MS(DI): 148 $(M+H)^{\oplus}$

Beispiel 38

R5 = $OCOCH_2S(CH_2)_2NHCO(CH_2)C(CH_3)_3$
2-(2,2-Dimethylbutanoylamino)-ethyl-thioacetoxy
R5* = OH
W = F.15

Synthese analog Beispiel 31 ausden Verbindungen Beispiel 1 und Beispiel 38-a
MS(FAB): 1368.7 $(M+Na)^{\oplus}$, 1346 $(M+H)^{\oplus}$, 1328.7

a) Herstellung von 2-(2,2-Dimethylbutanoylamino)-ethyl-thiol

Zu einer Lösung von 5,68 g 2-Mercaptoethylaminhydrochlorid und 10,12 g Triethylamin in 200 ml DSM wurden bei 0°C 5,39 g Dimethylbuttersäurechlorid in 10 ml DSM während 20 min zugetropft. Nach beendeter Zugabe wurde noch 40 min bei 0°C nachgerührt. Das Reaktionsgemisch wurde nacheinander mit Wasser, 0,4N HCl, dann wieder mit Wasser gewaschen, über $Na_2SO_4$ getrocknet und i. Vak, eingeengt. Der Rückstand wurde an Kieselgel gereinigt (Toluol:EE 7:3).
Ausbeute: 4,5 g
MS(DI): 176 $(M+H)^{\oplus}$

Beispiel 39

R5 = $OCOCH_2S(CH_2)_2NHCOCH(CH_2CH(CH_3)_2)NHCO_2C(Me)_3$
2-(N-tert.Butoxycarbonyl-L-Leucylamino)-ethyl-thio-acetoxy
R5* = OH
W = F.15

Synthese analog Beispiel 31 aus den Verbindungen Beispiel 1 und Beispiel 39-b
MS(FAB): 1484.2 $(M+Na)^{\oplus}$ , 2462.2 $(M+H)^{\oplus}$, 1444.2, 1362.0

a) Herstellung von Bis-[2-(N-tert.Butoxycarbonyl-L-Leucylamino)ethan]disulfid

Synthese analog Beispiel 33-a aus Cystamindihydrochlorid und N-tert.-Butoxycarbonyl-L-leucin
MS(DI): 579 $(M+H)^{\oplus}$, 479, 379

b) Herstellung von 2-(N-tert.Butoxycarbonyl-L-Leucylamino)-ethy-thiol

Synthese analog Beispiel 33-b aus 39-a.
MS(CI): 291 $(M+H)^{\oplus}$, 191

Beispiel 41

R5 = $OCOCH_2S(CH_2)_2NHCOCH(CH_2CH(CH_3)_2)NH_2$
2-(L-Leucylamino)-ethyl-thio-acetoxy (als HCl-Salz)
R5* = OH
W = F.15

Synthese aus der Verbindung Beispiel 39 analog Beispiel 9.
MS(FAB): 1361.7 $(M+H)^{\oplus}$

Beispiel 42

R5 = $OCOCH_2S(CH_2)_2NHCOCH(CH_2CH(CH_3)_2)NH_2$
2-(L-Leucylamino)-ethyl-thio-acetoxy (als HCl-Salz)
R5* = R5
W = F.15

Synthese aus der Verbindung. Beispiel 40 analog Beispiel 9.
MS(FAB): 1592.2 $(M+H)^{\oplus}$

Beispiel 43

| | |
|---|---|
| R5 = | $OCOCH_2CH_2CH_2Br$<br>4-Brombutyroyloxy- |
| R5* = | OH |
| W = | F.15 |

1,13 g (1 mmol N,N'-bis[(2S-(1,1-Dimethylethylsulfonylmethyl)-3-(1-naphthyl)-propionyl)-L-valyl]-2S,5S-diamino-1,6-diphenyl-hexan-3R,4R-diol (R5 = R5* = OH) wurden in 5 ml THF abs. gelöst und mit 0,46 ml (4 mmol) 4-Brombutter-säurechlorid versetzt. Anschließend rührte man 12 h bei RT. Man verdünnte die Reaktionslösung mit 100 ml Ethylacetat und extrahierte mehrmals mit Wasser und gesättigter Kochsalzlösung. Die organische Phase wurde mit Natriumsulfat getrocknet, filtriert und i. Vak. zur Trockne eingedampft. Es verblieben 640 mg eines amorphen Produktes.
MS: 1279,5 $(M+H)^{\oplus}$

Beispiel 44

| | |
|---|---|
| R5 = | $OCOCH_2CH_2CH_2Br$<br>4-Brombutyroyloxy- |
| R5* = | R5 |
| W = | F.15 |

800 mg N,N'-bis [(2S-(1,1-Dimethylethylsulfonylmethyl)-3-(1-naphthyl)-propionyl)-L-valyl]-2S,5S-diamino-1,6-diphenyl-hexan-3R,4R-diol (R5 = R5* = OH) und 864 mg DMAP wurden zusammen in 20 ml THF abs. gelöst und mit 0,2 ml 4-Brombuttersäurechlorid versetzt. Man rührte 18 h bei RT. Anschließend verdünnte man mit Ethylacetat und extrahierte mehrmals mit 10 %iger Citronensäurelösung und ges. Kochsalziösung. Die organische Phase wurde über Natriumsulfat getrocknet, filtriert und i. Vak, zur Trockne verblieben 570 mg eines amorphen Produktes.
MS; 1429,5 $(M+H)^{\oplus}$

Beispiel 45

| | |
|---|---|
| R5 = | $OCOCH_2N(CH_3)CH_2CH_2NH$-Arg-H<br>[N-Methyl-N-(2-L-arginylamino)ethyl) |
| R5* = | OH |
| W = | F.15 |

205 mg Verbindung aus 45g wurden in 1 ml Dichlormethan, 5 ml Trifluoressigsäure und 0,45 ml m-Kresol gelöst. Anschließend versetzte man 0,48 ml Trimethylsilylbromid und rührte 2 h bei RT. Die Reaktionslösung wird in 100 ml tert.-Butylmethylether gegeben und das Reaktionsprodukt durch Zentrifugieren isoliert. Nach Trocknung verbleiben 160 mg der Titelverbindung als Trihydrobromid.
MS: 1403 (M+H)

Beispiel 45a

Z-$N(CH_3)$-$CH_2CH_2$-OH

N-Benzyloxycarbonyl-2-methylamino-ethanol

4,5 g (0,06 mol) 2-Methylamino-ethanol werden in 50 ml abs. Tetrahydrofuran gelöst und unter leichter Kühlung mit einer Lösung von 14,9 g (0,06 mol) N-(Benzyloxycarbonyloxy)-succinimid in 30 ml THF versetzt. Anschließend rührt man 4 h bei RT. Die Reaktionslösung wird i. Vak. einrotiert und der verbleibende Rückstand in 100 ml Ethylacetat aufgenommen. Die organische Phase wird mehrmals mit 10 %iger Citronensäurelösung und 10 %iger Kaliumhydrogencarbonatlösung und ges. NaCl-Lösung gewaschen. Man trocknet mit Natriumsulfat, filtriert und rotiert das Filtrat zur Trockne ein. Es verbleibt ein farbloses Öl.
Ausbeute: 11,4 g
MS: 210 (M+H)

Beispiel 45b

Z-N($CH_3$)-$CH_2CH_2$-OTos

N-Benzyloxycarbonyl-2-methylamino-1-p-tosyloxy-ethan

11,3 g (0,054 mol) N-Z-methylamino-ethanol (Verbindung aus Beispiel 45a) werden in 100 ml Pyridin gelöst, auf 0°C gekühlt. Man tropft innerhalb von 30 min. eine Lösung von 12,2 g (0,064 mol) 4-Toluol-sulfonsäurechlorid in 60 ml Chloroform zu und rührt über Nacht bei 0°C. Anschließend rotiert man die Reaktionslösung ein, nimmt den Rückstand in 200 ml Ethylacetat auf und wäscht mehrmals mit 10 %iger Citronensäurelösung und ges. Kochsalzlösung, trocknet mit Natriumsulfat, filtriert und rotiert das Filtrat i. Vak. ein. Das verbleibende Rohprodukt wird durch Chromatographie an Kieselgel gereinigt (Methyl-tert.butylether/Cyclohexan: 3/1).
Ausbeute: 13 g
MS: 364 (M+H)

Beispiel 45c

Z-N($CH_3$)-$CH_2CH_2$-$N_3$

N-Benzyloxycarbonyl-2-methylamino-ethylazid

4,7 g (0,013 mol) Verbindung aus Beispiel 45b mit 2,5 g (0,039 mol) Natriumazid und 1 g Kronenether 18-Krone-6 werden zusammen in 100 ml abs. Dimethylformamid 1 h auf 70°C erwärmt. Anschließend rotiert man i. Vak. ein, nimmt den Rückstand in 150 ml Ethylacetat auf, wäscht mehrmals mit 10 %iger Citronensäurelösung, 10 %iger $KHCO_3$-Lösung und ges. Kochsalzlösung. Man trocknet mit Natriumsulfat, filtriert und rotiert das Filtrat i. Vak. ein. Es verbleibt eine gelbliche, leicht bewegliche Flüssigkeit.
Ausbeute: 2,7 g
MS: 235 (M+H)

Beispiel 45d

Z-N($CH_3$)-$CH_2CH_2$-$NH_2$*HCl

2-(N-Benzyloxycarbonyl-N-methyl-amino)-ethylamin Hydrochlorid

2,1 g (9 mmol) Azid aus Beispiel 45c werden in 50 ml Methanol gelöst öund mit 6 g (27 mmol) Zinn-(II)-chlorid-dihydrat versetzt. Man rührt 2 h bei RT ($N_2$-Entwicklung beendet) und engt i. Vak. zur Trockne ein. Der Rückstand wird in wenig Wasser aufgenommen und mit 2M NaOH auf pH 11 gestellt. Die alkalische Lösung wird 3x mit 100 ml Methyl-tert.butylether extrahiert und die vereinigten organischen Phasen werden 2x mit ges. Kochsalzlösung gewaschen. Die organische Phase wird mit Natriumsulfat getrocknet, filtriert und i. Vak. einrotiert. Das zurückbleibende Öl wird in 100 ml Diethylether gelst und zur Fällung des Hydrochlorids mit 1M etherischer HCl versetzt. Der Niederschlag wird abgesaugt, gründlich mit Ether gewaschen und im Exsikkator getrocknet. Ausbeute: 2 g
MS: 209 (M+H)

Beispiel 45e

Boc-Arg(Mtr)-NH-$CH_2CH_2$-N($CH_3$)-Z

N-[$N_\alpha$-tert.Butyloxycarbonyl-$N_\omega$-(4-methoxy-2,3,6-trimethylbenzolsulfonyl)-L-arginyl]-N'-[benzyl-oxycarbonyl-N'-methylethylendiamin

2,43 g (5 mmol) Boc-Arg(Mtr)-OH, 675 mg (5 mmol) N-Hydroxybenzotriazol und 1,22 g (5 mmol) 2-(N-Benzyloxycarbonyl-N-methyl-amino)-ethylamin Hydrochlorid [45d] werden zusammen in 25 ml Dimethylformamid gelöst

und bei 0°C mit 1,13 g (5,5 mmol) N,N'-Dicyclohexylcarbodiimid und 0,85 ml (5 mmol) Diisopropylethylamin versetzt. Die Lösung wird 20 min. bei 0°C und 3 h bei RT gerührt. Der Niederschlag wird abfiltriert und das Filtrat i. Vak. einrotiert. Der Rückstand wird in Ethylacetat aufgenommen und mehrmals mit 10 %iger Citronensäurelösung, 10 %iger $KHCO_3$-Lösung und ges. NaCl-Lösung gewaschen. Man trocknet mit Natriumsulfat, filtriert und dampft das Filtrat i. Vak. ein.
Ausbeute: 2,4 g amorphes Produkt
MS: 677,3 (M+H)

Beispiel 45f

Boc-Arg(Mtr)-NH-$CH_2CH_2N(CH_3)$-H

N-[$N_\alpha$-tert.Butyloxycarbonyl-$N_\omega$-(4-methoxy-2,3,6-trimethylbenzolsulfonyl)-L-arginyl]-N'-methyl-ethylendiamin

2,1 g Verbindung aus Beispiel 45e werden in 30 ml Tetrahydrofuran gelöst und mit 400 mg Katalysator (10 % Pd/C) 4 h bei RT hydriert. Der Katalysator wird abfiltriert und das Filtrat i. Vak. zur Trockne eingedampf.
Ausbeute: 1,4 g amorphes Produkt
MS: 544 (M+H)

Beispiel 45g

R5 = $OCOCH_2N(CH_3)CH_2CH_2NH$-Arg(Mtr)-Boc
{N-Methyl-N-[2-($N_\alpha$-tert.butyloxycarbonyl-[$N_\omega$-(4-methoxy-2,3,6-trimethylbenzosulfonyl)-L-arginyl]-N-aminoethyl]amino}-acetoxy-
R5* = OH
W = F.15

250,4 mg (0,2 mmol) Verbindung aus Beispiel 1 mit (0,4 mmol) 217,1 mg Verbidnung aus Beispiel 45f und einer katalytischen Menge von 10 mg Kaliumjodid werden zusammen in 2 ml Acetonitril gelöst und 12 h bei RT gerührt. Nach Abdampfen des Lösungsmittels wird das Rohprodukt durch Chromatographie an Kieselgel gereinigt. (Dichlormethan/Methanol: 15/1)
MS: 1715 (M+H)

Beispiel 46

R5 = $OCOCH_2N(CH_3)CH_2CH_2NH$-Arg-H
[N-Methyl-N-(2-L-arginyl)-aminoethyl)amino]-acetyloxy-
R5* = R5
W = F.15

Die Abspaltung der Schutzgruppen erfolgt analog der in Beispiel 45 beschriebenen Methode aus 46a.
MS: 1673 (M+H)

Beispiel 46a

R5 = $OCOCH_2N(CH_3)CH_2CH_2NH$-Arg-(Mtr)-Boc
R5* = R5
W = F.15

274 mg (0,2 mmol) Verbindung aus Beispiel 2 mit 434 mg (0.8 mmol) Verbindung aus Beispiel 45f und einer katalytischen Menge von 20 mg Kaliumjodid werden zusammen in 3 ml Acetonitril gelöst und 24 h bei RT gerührt. Nach Entfernung des Lösungsmittels wird das Rohprodukt durch Chromatographie an Kieselgel gereinigt. (Dichlormethan/Methanol: 12/1)

Ausbeute: 330 mg
MS: 2297 (M+H)

Beispiel 47

R5 = $OCOCH_2N(CH_3)CH_2CH_2NH$-Gly-H
[N-Methyl-N-(2-glycyl-aminoethyl)amino]-acetyloxy-
R5* = OH
W = F.15

Die Abspaltung der Schutzgruppen erfolgt analog der in Beispiel 45 beschriebenen Methode aus 47c.
MS: 1302,7 (M+H)

Beispiel 47a

Boc-Gly-NH-$CH_2CH_2N(CH_3)$-Z

N-Benzyloxycarbonyl-[N-methyl-N-(tert.butyloxycarbonyl-glycyl-aminoethyl)]amin

Die Synthese erfolgt analog der in Beispiel 45e beschriebenen Methode aus Boc-Gly-OH und Verbindung aus Beispiel 45d.
MS: 366 (M+H)

Beispiel 47b

Boc-Gly-NH-$CH_2CH_2N(CH_3)$-H

[N-methyl-N-(tert.butyloxycarbonyl-glycyl-aminoethyl)]amin

Die Synthese erfolgt analog der in Beispiel 45f beschriebenen Methode aus 47a und Wasserstoff.
MS: 252 (M+H)

Beispiel 47c

R5 = $OCOCH_2N(CH_3)$-$CH_2CH_2NH$-Gly-Boc
[N-Methyl-N-{2-(N-tert.butyloxycarbonyl-glycyl)-aminoethyl}-amino]-acetoxy-
R5* = OH
W = F.15

Die Synthese erfolgt analog der in Beispiel 45g beschriebenen Methode aus 47b und Verbindung aus Beispiel 1.
MS: 1402,8 (M+H)

Beispiel 48

R5 = $OCOCH_2N(CH_3)CH_2CH_2NH$-Gly-H
[N-Methyl-N-(2-glycyl-aminoethyl)amino]-acetyloxy-
R5* = R5
W = F.15

Die Abspaltung der Schutzgruppen erfolgt analog der in Beispiel 45 beschriebenen Methode aus 48a.
MS: 1445 (M+H)

Beispiel 48a

R5 = $OCOCH_2N(CH_3)CH_2CH_2NH$-Gly-Boc
[N-Methyl-N-(2-tert.-butyloxycarbonyl-glycyl-aminoethyl)amino]-acetyloxy-
R5* = R5
W = F.15

Die Synthese erfolgt analog der in Beispiel 46a beschriebenen Methode aus 47b und Verbindung aus Beispiel 2.
MS: 1674 (M+H)

Beispiel 49

R5 = $OCOCH_2N(CH_3)CH_2CH_2NH$-Sar-H
[N-Methyl-N-(2-sarkoxyl-aminoethyl)amino]-acetyloxy-
R5* = OH
W = F.15

Die Abspaltung der Schutzgruppen erfolgt analog der in Beispiel 45 beschriebenen Methode aus 49c.
$C_{72}H_{97}N_7O_{12}S_2$ M: 1316,7
MS: 1316,7 (M+H)

Beispiel 49a

Boc-Sar-NH-$CH_2CH_2N(CH_3)$-Z

N-Benzyloxycarbonyl-[N-methyl-N-(tert.butyloxycarbonyl-sarkosyl-aminoethyl)]amin

Die Synthese erfolgt analog der in Beispiel 45e beschriebenen Methode aus Boc-Sar-OH und Verbindung aus Beispiel 45d.
MS: 380 (M+H)

Beispiel 49b

Boc-Sar-NH-$CH_2CH_2N(CH_3)$-H

[N-methyl-N-tert.butyloxycarbonyl-sarkosyl-aminoethyl)]amin

Die Synthese erfolgt analog der in Beispiel 45f beschriebenen Methode aus 49a und Wasserstoff.
MS: 246 (M+H)

Beispiel 49c

R5 = $OCOCH_2N(CH_3)CH_2CH_2NH$-Sar-Boc
[N-Methyl-N-(2-tert.butyloxycarbonyl-sarkosyl-aminoethyl)amino]-acetyloxy-
R5* = OH
W = F.15

Die Synthese erfolgt analog der in Beispiel 45g beschriebenen Methode aus 49b und Verbindung aus Beispiel 1.
MS: 1416,7 (M+H)

**Beispiel 50**

R5 = $OCOCH_2N(CH_3)CH_2CH_2NH$-Sar-H
[N-Methyl-N-(2-tert.butyloxycarbonyl-sarkosyl-aminoethyl)amino]-acetyloxy-
R5* = R5
W = F.15

Die Abspaltung der Schutzgruppen erfolgt analog der in Beispiel 45 beschriebenen Methode aus 50a und Verbindung aus Beispiel 2.
MS: 1503 (M+H)

Beispiel 50a

R5 = $OCOCH_2N(CH_3)CH_2CH_2NH$-Sar-Boc
[N-Methyl-N-(2-tert.butyloxycarbonyl-sarkosyl-aminoethyl)amino]-acetoxy-
R5* = R5

Die Synthese erfolgt analog der in Beispiel 46a beschriebenen Methode aus 49b und Verbindung aus Beispiel 2.
MS: 1702,6 (M+H)

Beispiel 51

R5 = $OCOCH_2N(CH_3)CH_2CH_2NH$-D-Lys-H
[N-Methyl-N-(2-D-lysyl-aminoethyl)amino]-acetyloxy-
R5* = OH
W = F.15

Die Abspaltung der Schutzgruppen erfolgt analog der in Beispiel 45 beschriebenen Methode aus 51c.
MS: 1373,4 (M+H)

Beispiel 51a

Boc-D-Lys(Boc)-NH-$CH_2CH_2N(CH_3)$-Z

Die Synthese erfolgt analog der in Beispiel 45e beschriebenen Methode aus Boc-D-Lys(Boc)-OH und Verbindung aus Beispiel 45d.
MS: 538 (M+H)

Beispiel 51b

Boc-D-Lys(Boc)-NH-$CH_2CH_2N(CH_3)$-H

Die Synthese erfolgt analog der in Beispiel 45f beschriebenen Methode aus 51a und Wasserstoff.
MS: 403 (M+H)

Beispiel 51c

R5 = $OCOCH_2N(CH_3)CH_2CH_2NH$-D-Lys(Boc)-Boc
[N-Methyl-N-(2-D-lysyl-aminoethyl)amino)-acetyloxy-

R5* = OH
W = F.15

Die Synthese erfolgt analog der in Beispiel 45g beschriebenen Methode aus 51b und Verbindung aus Beispiel 1. MS: 1573 (M+H)

Beispiel 52

R5 = $OCOCH_2N(CH_3)CH_2CH_2NH$-D-Lys-H
[N-Methyl-N-(2-D-lysyl-aminoethyl)amino]-acetyloxy-
R5* = R5
W = F.15

Die Abspaltung der Schutzgruppen erfolgt analog der in Beispiel 45 beschriebenen Methode aus 52a.
MS: 1617 $(M+H)^{\oplus}$

Beispiel 52a

R5 = $OCOCH_2N(CH_3)CH_2CH_2NH$-D-Lys(Boc)-Boc
[N-Methyl-N-(2-D-lysyl-aminoethyl)amino]-acetyloxy-
R5* = R5
W = F.15

Die Synthese erfolgt analog der in Beispiel 46a beschriebenen Methode aus 51b und Verbindung aus Beispiel 2.
MS: 2017 $(M+H)^{\oplus}$

Beispiel 53

R5 = $OCOCH_2N(CH_3)CH_2CH_2NH$-D-Arg-H
[N-Methyl-N-(2-D-arginyl)-aminoethyl)amino]-acetyloxy-
R5* = R5
W = F.15

Die Abspaltung der Schutzgruppen erfolgt analog der in Beispiel 45 beschriebenen Methode aus 53c.
MS: 1673 $(M+H)^{\oplus}$

Beispiel 53a

Boc-D-Arg(Mtr)-NH-$CH_2CH_2N(CH_3)$-Z

N-Benzyloxycarbonyl-{N-methyl-N-[$N_\alpha$-tert.butyloxycarbonyl-$N_\omega$-(4-methoxy-2,3,6-trimethyl-benzolsulfonyl)-D-arginyl]-aminoethyl}amin

Die Synthese erfolgt analog der in Beispiel 45e beschriebenen Methode aus Boc-D-Arg(Mtr)-OH und Verbindung aus Beispiel 45d.
MS: 678 (M+H)

Beispiel 53b

Boc-D-Arg(Mtr)-NH-$CH_2CH_2N(CH_3)$-H

{N-methyl-N-[$N_\alpha$-tert.butyloxycarbonyl-$N_\omega$ -(4-methoxy-2,3,6-trimethylbenzolsulfonyl)-D-arginyl]-aminoethyl}amin

Die Synthese erfolgt analog der in Beispiel 45f beschriebenen Methode aus 53a und Wasserstoff.
MS: 544 $(M+H)^{\oplus}$

Beispiel 53c

R5 = $OCOCH_2N(CH_3)CH_2CH_2NH$-D-Arg(Mtr)-Boc
{[N-Methyl-N-{2-$N_\alpha$-tert.butyloxycarbonyl-$N_\omega$-(4-methoxy-2,3,6-trimethylbenzolsulfonyl)-D-argiynl}N-aminoethyl]amino}-acetyloxy-
R5* = R5
W = F.15

Die Synthese erfolgt analog der in Beispiel 46a beschriebenen Methode aus 53b und Verbindung aus Beispiel 2.
MS: 2297 $(M+H)^{\oplus}$

Beispiel 54

R5 = $OCOCH_2N(CH_3)CH_2CH_2NH$-Pro-H
[N-Methyl-N-(2-L-prolyl-aminoethyl)amino]-acetyloxy-
R5* = OH
W = F.15

Die Abspaltung der Schutzgruppen erfolgt analog der in Beispiel 39h beschriebenen Methode aus 48g.
MS: 1344 $(M+H)^{\oplus}$

Beispiel 54a

Boc-Pro-NH-$CH_2CH_2N$-$(CH_3)$-Z

N-Benzyloxycarbonyl-[N-methyl-N-(tert.butyloxycarbonyl-L-prolylaminoethyl)]amin

Die Synthese erfolgt analog der in Beispiel 45e beschriebenen Methode aus Boc-Pro-OH und Verbindung aus Beispiel 45d.
MS: 406 $(M+H)^{\oplus}$

Beispiel 54b

Boc-Pro-NH-$CH_2CH_2N$-$(CH_3)$-H

Die Synthese erfolgt analog der in Beispiel 45f beschriebenen Methode aus 54a und Wasserstoff.
MS: 272 $(M+H)^{\oplus}$

Beispiel 54c

R5 = $OCOCH_2N(CH_3)CH_2CH_2NH$-Pro-Boc
R5* = OH

W = F.15

Die Synthese erfolgt analog der in Beispiel 45g beschriebenen Methode aus 54b und Verbindung aus Beispiel 1.
MS: 1442,6 $(M+H)^{\oplus}$

Beispiel 55

R5 = $OCOCH_2N(CH_3)CH_2CH_2NH$-Pro-H
[N-Methyl-N(2-L-prolyl-aminoethyl)amino]-acetyloxy-
R5* = R5
W = F.15

Die Synthese erfolgt analog der in Beispiel 40a beschriebenen Methode aus 54b und Verbindung aus Beispiel 2.
MS: 1555 $(M+H)^{\oplus}$

Beispiel 55a

R5 = $OCOCH_2N(CH_3)CH_2CH_2NH$-Pro-Boc
R5* = R5
W = F.15

Die Synthese erfolgt analog der in Beispiel 46a beschriebenen Methode aus 54b und Verbindung aus Beispiel 2.
MS: 1754 $(M+H)^{\oplus}$

Beispiel 56

R5 = $OCOCH_2N(CH_3)CH_2CH_2N(CH_3)$-Arg-D-H
[N-Methyl-N-(2-D-arginyl-N-2-methyl-aminoethyl)amino]-acetyloxy-
R5* = OH
W = F.15

300 mg Verbindung aus Beispiel 56d wurden in 1 ml Dichlormethan, 5 ml Trifluoressigsäure und 0,45 ml m-Kreson gelöst. Anschließend versetzte man mit 0,48 ml Trimethylsilylbromid und rührte 2 h bei RT. Die Reaktionslösung wird in 100 ml tert.-Butylmethylether gegeben und das Reaktionsprodukt durch Zentrifugieren isoliert. Nach Trocknung verbleiben 260 mg der Titelverbindung als Trihydrobromid.
MS: 1415,6 $(M+H)^{\oplus}$

Beispiel 56a

Z-$N(CH_3)$-$CH_2CH_2N(CH_3)$-H

N-Methyl-(N-benzyloxycarbonyl-2-methylaminoethyl)amin

Zu 22,8 g (0,25 mol) N,N'-Dimethylethylendiamin in 250 ml Tetrahydrofuran wird bei 0°C innerhalb von 30 min. eine Lösung aus 18,7 g (0.075 mol) z-OSu in 150 ml THF getropft. Nach 60 min. bei RT wird der entstandene Niederschlag (HOSU-Salz des Eduktes) abgesaugt und mit THF gewaschen. Das Filtrat wird i. Vak. einrotiert. Das erhaltene Rohprodukt wird durch Chromatographie an Kieselgel gereinigt. (Dichlormethan/Methanol/Essigsäure/Wasser: 50/12/2/2)
Die freie Base der gewünschten Verbindung erhält man durch Neutralisation einer wässrigen Lösung des angefallenen

Acetats und Extraktion mit Diethylether.
Ausbeute: 8,5 g
MS: 223 $(M+H)^{\oplus}$

Beispiel 56b

Boc-D-Arg(Mtr)-$N(CH_3)$-$CH_2CH_2N(CH_3)$-Z

N-Benzyloxycarbonyl-{N-methyl-N-[$N_{\alpha}$-tert.butyloxycarbonyl-$N_{\omega}$-(4-methoxy-2,3,6-trimethyl-benzolsulfonyl-D-arginyl]-methylaminoethyl}amin

Die Synthese erfolgt analog der in Beispiel 45e beschriebenen Methode aus Boc-D-Arg(Mtr)-OH und Verbindung aus Beispiel 56a.
MS: 691,3 $(M+H)^{\oplus}$

Beispiel 56c

Boc-D-Arg(Mtr)-$N(CH_3)$-$CH_2CH_2N(CH_3)$-H

{N-methyl-N-[$N_{\alpha}$-tert.butyloxycarbonyl-$N_{\omega}$-(4-methoxy-2,3,6-trimethylbenzolsuflonyl)-D-arginyl]-methylaminoethyl} amin

Die Synthese erfolgt analog der in Beispiel 45f beschriebenen Methode aus 56b und Wasserstoff.
MS: 557,3 $(M+H)^{\oplus}$

Beispiel 56d

R5 = $OCOCH_2N(CH_3)CH_2CH_2N(CH_3)$-D-Arg(Mtr)-Boc
{N-Methyl-N-[$N_{\alpha}$-tert.butyloxycarbonyl-[$N_{\omega}$-(4-methoxy-2,3,6-trimethyl-benzolsulfonyl)D-arginyl]-N-methylaminoethyl]amino}-acetyloxy-
R5* = OH
W = F.15

250,4 mg (0,2 mmol) Verbindung aus Beispiel 1 (0,4 mmol) 222,6 mg Verbindung aus Beispiel 56c und eine katalytische Menge von 10 mg Kaliumjodid werden zusammen in 2 ml Acetonitril gelöst und 12 h bei RT gerührt. Nach Abdampfen des Lösungsmittels wird das Rohprodukt durch Chromatographie an Kieselgel gereinigt. (Dichlormethan/Methanol: 15/1)
MS: 1728 $(M+H)^{\oplus}$

Beispiel 57

R5 = $OCOCH_2N(CH_3)CH_2CH_2N(CH_3)$-Gly-H
[N-Methyl-N-(2-glycyl-N-2-methylaminoethyl)amino]-acetyloxy-
R5* = OH
W = F.15

Die Abspaltung der Schutzgruppen erfolgt analog Beispiel 45 beschriebenen Methode aus 57c.
MS: 1317,6 $(M+H)^{\oplus}$

Beispiel 57a

Boc-Gly-$N(CH_3)-CH_2CH_2N-(CH_3)$-Z

N-Benzyloxycarbonyl-[N-methyl-N-(tert.butyloxycarbonyl-glycyl-methylaminoethyl)]amin

Die Synthese erfolgt analog der in Beispiel 45e beschriebenen Methode aus Boc-Gly-OH und Verbindung aus Beispiel 56a.
MS: 380,2 (M+H)⊕

Beispiel 57b

Boc-Gly-$N(CH_3)-CH_2CH_2N(CH_3)$-H

[N-methyl-N-(tert.butyloxycarbonyl-glycyl-methylaminoehtyl)]amin

Die Synthese erfolgt analog der in Beispiel 45f beschriebenen Methode aus 57a und Wasserstoff.
MS: 246 (M+H)⊕

Beispiel 57c

R5 = $OCOCH_2N(CH_3)CH_2CH_2N(CH_3)$-Gly-Boc
[N-Methyl-N-(2-tert.butyloxycarbonyl-glycyl-N-2-methyl-aminoethyl)amino]-acetyloxy-
R5* = OH
W = F.15

Die Synthese erfolgt analog der in Beispiel 45g beschriebenen Methode aus 57b und Verbindung aus Beispiel 1.
MS: 1688,2 (M+H)⊕

Beispiel 58

R5 = $OCOCH_2N(CH_3)CH_2CH_2N(CH_3)$-Gly-H
[N-Methyl-N-(2-glycyl-N-2-methylaminoethyl)amino]-acetyloxy-
R5* = R5
W = F.15

Die Abspaltung der Schutzgruppen erfolgt analog der in Beispile 45h beschriebenen Methode aus 58a.
MS: 1473,7 (M+H)

Beispiel 58a

R5 = $OCOCH_2N(CH_3)CH_2CH_2N(CH_3)$-Gly-Boc
[N-Methyl-N-(Boc-glycyl-N-2-methylaminoethyl)amino]-acetyloxy-
R5* = R5
W = F.15

Die Synthese erfolgt analog der in Beispiel 46a beschriebenen Methode aus 57b und Verbindung aus Beispiel 2.
MS: 1702,6 (M+H)⊕

Beispiel 59

R5 = $OCOCH_2N(CH_3)CH_2CH_2N(CH_3)$-Sar-H
[N-Methyl-N-(2-sarkosyl-N-2-methyl-aminoethyl)amino]-acetyloxy-
R5* = OH
W = F.15

Die Abspaltung der Schutzgruppen erfolgt analog der in Beispiel 45 beschriebenen Methode aus 59c.
MS: 1330,7 $(M+H)^{\oplus}$

Beispiel 59a

Boc-Sar-$N(CH_3)$-$CH_2CH_2N$-$(CH_3)$-Z

N-Benzyloxycarbonyl-[N-methyl-N-(tert.butyloxycarbonyl-sarkosyl-methylaminoethyl)]amin

Die Synthese erfolgt analog der in Beispiel 45e beschriebenen Methode aus Boc-Sar-OH und Verbindung aus Beispiel 56a.
MS: 394,4 $(M+H)^{\oplus}$

Beispiel 59b

Boc-Sar-$N(CH_3)$-$CH_2CH_2N$-$(CH_3)$-H

[N-methyl-N-(tert.butyloxycarbonyl-sarkosyl-methylaminoethyl]amin

Die Synthese erfolgt analog der in Beispiel 45f beschriebenen Methode aus 59a und Wasserstoff.
MS: 260,2 $(M+H)^{\oplus}$

Beispiel 59c

R5 = $OCOCH_2N(CH_3)CH_2CH_2N(CH_3)$-Sar-Boc
R5* = OH
W = F.15

Die Synthese erfolgt analog der in Beispiel 39g beschriebenen Methode aus 54 und Verbindung aus Beispiel 1. MS: 1431,4 $(M+H)^{\oplus}$

Beispiel 60

R5 = $OCOCH_2N(CH_3)CH_2CH_2N(CH_3)$-Sar-H
[N-Methyl-N-(2-sarkosyl-N-2-methyl-aminoethyl)amino]-acetyloxy-
R5* = R5
W = F.15

Die Abspaltung der Schutzgruppen erfolgt analog der in Beispiel 45 beschriebenen Methode aus 60a.
MS: 1531 $(M+H)^{\oplus}$

Beispiel 60a

R5 = $OCOCH_2N(CH_3)CH_2CH_2N(CH_3)$-Sar-Boc
[N-Methyl-N-(2-tert.butyloxycarbonyl-sarkosyl-N-2-methylaminoethyl)amino]-acetyloxy-
R5* = R5
W = F.15

Die Synthese erfolgt analog der in Beispiel 46a beschriebenen Methode aus 59b und Verbindung aus Beispiel 2.
MS: 1731 $(M+H)^{\oplus}$

Beispiel 61

R5 = $OCOCH_2N(CH_3)CH_2CH_2N(CH_3)$-L-Arg-H
[N-Methyl-N-(2-L-arginyl)-N-(2-methyl-aminoethyl)amino]-acetyloxy-
R5* = OH
W = F.15

Die Abspaltung der Schutzgruppen erfolgt analog der in Beispiel 45 beschriebenen Methode aus 61c.
MS: 1415,6 $(M+H)^{\oplus}$

Beispiel 61a

Boc-Arg(Mtr)-$N(CH_3)$-$CH_2CH_2N$-$(CH_3)$-Z

N-Benzyloxycarbonyl-{N-methyl-N-[$N_{\alpha}$-tert.butyloxycarbonyl-$N_{\omega}$(4-methoxy-2,3,6-trimethyl-benzolsulfonyl)-L-arginyl]-methylaminoethyl}amin

Die Synthese erfolgt analog der in Beispiel 45e beschriebenen Methode aus Boc-Arg(Mtr)-OH und Verbindung aus Beispiel 56a.
MS: 691,3 $(M+H)^{\oplus}$

Beispiel 61b

Boc-Arg(Mtr)-$N(CH_3)$-$CH_2CH_2N$-$(CH_3)$-H

{N-methyl-N-[$N_{\alpha}$-tert.butyloxycarbonyl-$N_{\omega}$-(4-methoxy-2,3,6-trimethylbenzolsulfonyl)-L-arginyl]aminoethyl}amin

Die Synthese erfolgt analog der in Beispiel 45f beschriebenen Methode aus 61a und Wasserstoff.
MS: 557,3 $(M+H)^{\oplus}$

Beispiel 61c

R5 = $OCOCH_2N(CH_3)CH_2CH_2N(CH_3)$-L-Arg(Mtr)-Boc
{[N-Methyl-N-[2-$N_{a}$-tert.butyloxycarbonyl-$N_{w}$-(4-methoxy-2,3,6-trimethylbenzol-sulfonyl)-L-arginyl]N-2-methylaminoethyl]amino}-acetyloxy-
R5* = OH
W = F.15

Die Synthese erfolgt analog der in Beispiel 46a beschriebenen Methode aus 61b und Verbindung aus Beispiel 2.
MS: 1729 $(M+H)^{\oplus}$

Beispiel 62

R5 = $OCOCH_2N(CH_3)CH_2CH_2N(CH_3)$-Arg-H
[N-Methyl-N-(2-L-arginyl)-N-(2-methyl-aminoethyl)amino]-acetyloxy-
R5* = R5
W = F.15

Die Spaltung der Schutzgruppen erfolgt analog der in Beispiel 45h beschriebenen Methode aus 62a. MS: 1700,9 $(M+H)^{\oplus}$

Beispiel 62a

R5 = $OCOCH_2N(CH_3)CH_2CH_2N(CH_3)$-Arg(Mtr)-Boc
R5* = R5
W = F.15

Die Synthese erfolgt analog der in Beispiel 46a beschriebenen Methode aus 61b und Verbindung aus Beispiel 2.
MS: 2325 $(M+H)^{\oplus}$

Beispiel 63

R5 = $OCOCH_2N(CH_{32})CH_2CH_2N(CH_3)$-Arg-D-H
[N-Methyl-N(2-D-arginyl)-N-(2-methyl-aminoethyl)amino]-acetyloxy-
R5* = R5
W = F.15

Die Äbspaltung der Schutzgruppen erfolgt analog der in Beispiel 45 beschriebenen Methode aus 63a. MS: 1700,9 $(M+H)^{\oplus}$

Beispiel 63a

R5 = $OCOCH_2N(CH_3)CH_2CH_2N(CH_3)$-Arg(Mtr)-D-Boc
R5* = R5
W = F.15

Die Synthese erfolgt analog der in Beispiel 46a beschriebenen Methode aus 56c und Verbindung aus Beispiel 2. MS: 2325 $(M+H)^{\oplus}$

Beispiel 64

R5 = $OCOCH_2N(CH_3)CH_2CH_2N(CH_3)$-Lys-H
[N-Methyl-N-(2-L-lysyl-N-2-methyl-aminoethyl)amino]-acetyloxy-
R5* = OH
W = F.15

Die Abspaltung der Schutzgruppen erfolgt analog der in Beispiel 39h beschriebenen Methode aus 60d. MS: 1389 $(M+H)^{\oplus}$

Beispiel 64a

Boc-Lys(Boc)-N($CH_3$)-$CH_2CH_2$N-($CH_3$)-Z

N-Benzyloxycarbonyl-[N-methyl-N-($N_\alpha$, $N_\varepsilon$-tert.butyloxycarbonyl-L-lysyl-methylamino-ethyl)]-amin

Die Synthese erfolgt analog der in Beispiel 45e beschriebenen Methode aus Boc-Lys(Boc)-OH und Verbindung aus Beispiel 56a.
MS: 551,3 (M+H)$^{\oplus}$

Beispiel 64b

Boc-Lys(Boc)-N($CH_3$)-$CH_2CH_2$N-($CH_3$)-H

[N-methyl-N-($N_\alpha$, $N_\varepsilon$ -tert.butyloxycarbonyl-L-lysyl-methylaminoethyl)]-amin

Die Synthese erfolgt analog der in Beispiel 45f beschriebenen Methode aus 64a und Wasserstoff. MS: 417,3 (M+H)$^{\oplus}$

Beispiel 64c

R5 = $OCOCH_2N(CH_3)CH_2CH_2N(CH_3)$-Lys(Boc)-Boc
R5* = OH
W = F.15

Die Synthese erfolgt analog der in Beispiel 45g beschriebenen Methode aus 64b und Verbindung aus Beispiel 1. MS: 1588,9 (M+H)$^{\oplus}$

Beispiel 65

R5 = $OCOCH_2N(CH_3)CH_2CH_2N(CH_3)$-Lys-H
[N-Methyl-N-(2-L-lysyl-N-2-methyl-aminoethyl)amino]-acetyloxy-
R5* = R5
W = F.15

Die Abspaltung der Schutzgruppen erfolgt analog der in Beispiel 45 beschriebenen Methode aus 65a. MS: 1644,2 (M + H)$^{\oplus}$

Beispiel 65a

R5 = $OCOCH_2N(CH_3)CH_2CH_2N(CH_3)$-Lys(Boc)-Boc
R5* = R5
W = F.15

Die Synthese erfolgt analog der in Beispiel 46a beschriebenen Methode aus 64b und Verbindung aus Beispiel 2.
MS: 2045 (M+H)$^{\oplus}$

Beispiel 66

R5 = $OCOCH_2N(CH_3)CH_2CH_2N(CH_3)$-D-Lys-H
R5* = OH
W = F,15

Die Abspaltung der Schutzgruppen erfolgt analog der in Beispiel 45 beschriebenen Methode aus 66c. MS: 1389 (M+H)$^{\oplus}$

Beispiel 66a

Boc-D-Lys(Boc)-$N(CH_3)$-$CH_2CH_2N$-$(CH_3)$-Z

N-Benzyloxycarbonyl-[N-methyl-N-($N_{\alpha}$, $N_{\varepsilon}$-tert.butyloxycarbonyl-D-lysyl-methylamino-ethyl)]-amin

Die Synthese erfolgt analog der in Beispiel 45e beschriebenen Methode aus Boc-D-Lys(Boc)-OH und Verbindung aus Beispiel 56a. MS: 551.3 (M+H)

Beispiel 66b

Boc-D-Lys(Boc)-$N(CH_3)$-$CH_2CH_2N$-$(CH_3)$-H

[N-methyl-N-($N_{\alpha}$, $N_{\varepsilon}$ -tert.butyloxycarbonyl-D-lysyl-methylaminoethyl)]-amin

Die Synthese erfolgt analog der in Beispiel 45f beschriebenen Methode aus 64a und Wasserstoff. MS: 417,3 (M+H)$^{\oplus}$

Beispiel 66c

R5 = $OCOCH_2N(CH_3)CH_2CH_2N(CH_3)$-Lys(Boc)-D-Boc
R5* = OH
W = F.15

Die Synthese erfolgt analog der in Beispiel 45g beschriebenen Methode aus 66b und Verbindung aus Beispiel 1.
MS: 1588,9 (M+H)$^{\oplus}$

Beispiel 67

R5 = $OCOCH_2N(CH_3)CH_2CH_2N(CH_3)$-D-Lys-H
[N-Methyl-N-(2-D-lysyl-N-2-methyl-aminoethyl]amino]-acetyloxy-
R5* = R5
W = F.15

Die Abspaltung der Schutzgruppen erfolgt analog der in Beispiel 45 beschriebenen Methode aus 67a. MS: 1644,2 (M+H)$^{\oplus}$

Beispiel 67a

R5 = $OCOCH_2N(CH_3)CH_2CH_2N(CH_3)$-D-Lys(Boc)-Boc

R5* = R5
W = F.15

Die Synthese erfolgt analog der in Beispiel 46a beschriebenen Methode aus 66b und Verbindung aus Beispiel 2.
MS: 2045 (M+H)⊕

Beispiel 68

R5 = $OCOCH_2N(CH_3)CH_2CH_2N(CH_3)$-Pro-H
[N-Methyl-N-(2-L-prolyl-N-2-methyl-aminoethyl)amino]-acetyloxy-
R5* = OH
W = F.15

Die Abspaltung der Schutzgruppen erfolgt analog der in Beispiel 45h beschriebenen Methode aus 68c. MS: 1358 (M + H)⊕

Beispiel 68a

Boc-Pro-$N(CH_3)$-$CH_2CH_2N$-$(CH_3)$-Z

N-Benzyloxycarbonyl-[N-methyl-N-tert.butyloxycarbonyl-L-prolyl-methylaminoethyl)]amin

Die Synthese erfolgt analog der in Beispiel 45e beschriebenen Methode aus Boc-Pro-OH und Verbindung aus Beispiel 56a.
MS: 420,3 (M+H)⊕

Beispiel 68b

Boc-Pro-$N(CH_3)$-$CH_2CH_2N$-$(CH_3)$-H

[N-methyl-N-(tert.butyloxycarbonyl-L-prolyl-methylaminoethyl)]amin

Die Synthese erfolgt analog der in Beispiel 45f bechriebenen Methode aus 68a und Wasserstoff.
MS: 286 (M+H)⊕

Beispiel 68c

R5 = $OCOCH_2N(CH_3)CH_2CH_2N(CH_3$-Pro-Boc
[N-Methyl-N-(2-prolyl-N-2-methyl-aminoethyl)amino]-acetyloxy-
R5* = OH
W = F.15

Die Synthese erfolgt analog der in Beispiel 45g beschriebenen Methode aus 68b und Verbindung aus Beispiel 1.
MS: 1457,6 (M+H)⊕

Beispiel 69

R5 = $OCOCH_2N(CH_3)CH_2CH_2N(CH_3)$-D-Pro-H
[N-Methyl-N-(2-D-prolyl-N-2-methyl-aminoethyl)amino]-acetyloxy-
R5* = OH

W = F.15

Die Abspaltung der Schutzgruppen erfolgt analog der in Beispiel 45 beschriebenen Methode aus 69c.
MS: 1357,9 (M+H)$^{\oplus}$

Beispiel 69a

Boc-D-Pro-N($CH_3$)-$CH_2CH_2$N-($CH_3$)-Z

Die Synthese erfolgt analog der in Beispiel 45e beschriebenen Methode aus Boc-D-Pro-OH und Verbindung aus Beispiel 56a.
MS: 420,2 (M+H)$^{\oplus}$

Beispiel 69b

Boc-D-Pro-N($CH_3$)-$CH_2$-$CH_2$-N($CH_3$)-H

[N-methyl-N-(tert.butyloxycarbonyl-D-prolyl-methylaminoethyl)]amin

Die Synthese erfolgt analog der in Beispiel 45f beschriebenen Methode aus 69a und Wasserstoff.
MS: 286 (M+H)$^{\oplus}$

Beispiel 69c

R5 = $OCOCH_2N(CH_3)CH_2CH_2N(CH_3)$-D-Pro-Boc
[N-Methyl-N-(2-D-prolyl-N-2-methyl-aminoethyl)amino]-acetyloxy-
R5* = OH
W = F.15

Die Synthese erfolgt analog der in Beispiel 45g beschriebenen Methode aus 69b und Verbindung aus Beispiel 1.
MS: 1457,6 (M+H)$^{\oplus}$

Beispiel 70

R5 = $OCOCH_2N(CH_3)CH_2CH_2N(CH_3)$-His-H
[N-Methyl-N-(2-L-histidyl-N-2-methyl-aminoethyl)amino]-acetyloxy-
R5* = OH
W = F.15

Die Abspaltung der Schutzgruppen erfolgt analog der in Beispiel 45 beschriebenen Methode aus 70c.

Beispiel 70a

Boc-His(Boc)-N($CH_3$)-$CH_2CH_2$N-($CH_3$)-Z

N-Benzyloxycarbonyl-{N-Methyl-N-[2-bis-($N_{\alpha}$,$N_{im}$-tert.butyloxycarbonyl)-L-histidyl]-N-2-methyl-aminoethyl}amin

Die Synthese erfolgt analog der in Beispiel 45e beschriebenen Methode aus Boc-His(Boc)-OH und Verbindung aus Beispiel 56a.
MS: 560,3 (M+H)$^{\oplus}$

Beispiel 70b

Boc-His(Boc)-N($CH_3$)-$CH_2CH_2$N-($CH_3$)-H

{N-Methyl-N-[2-bis-($N_\alpha$,$N_{im}$-tert.butyloxycarbonyl)-L-histidyl]-N-2-methyl-aminoethyl}amin

Die Synthese erfolgt analog der in Beispiel 45f beschriebenen Methode aus 70a und Wasserstoff.
MS: 426,3 $(M+H)^{\oplus}$

Beispiel 70c

R5 = $OCOCH_2N(CH_3)CH_2CH_2N(CH_3)$-His(Boc)-Boc
R5* = OH
W = F.15

Die Synthese erfolgt analog der in Beispiel 39g beschriebenen Methode aus 66c und Verbindung aus Beispiel 1.
MS: 1396,7 $(M+H)^{\oplus}$

Beispiel 71

R5 = $OCOCH_2N(CH_3)CH_2CH_2N(CH_3)$-Trp-H
[N-Methyl-N-(2-L-tryptophyl-N-2-methyl-aminoethyl)amino]-acetyloxy-
R5* = OH
W = F.15

Die Abspaitung der Schutzgruppen erfolgt analog der in Beispiel 45 beschriebenen Methode aus 71c.
MS: 1447 $(M+H)^{\oplus}$

Beispiel 71a

Boc-Trp-N($CH_3$)-$CH_2CH_2$N-($CH_3$)-Z

N-Benzyloxycarbonyl-[N-methyl-N-(tert.butyloxycarbonyl-L-tryptophylmethylaminoethyl)]amin

Die Synthese erfolgt analog der in Beispiel 45e beschriebenen Methode aus Boc-Trp-OH und Verbindung aus Beispiel 56a.
MS: 509,3 $(M+H)^{\oplus}$

Beispiel 71b

Boc-Trp-N($CH_3$)-$CH_2CH_2$N-($CH_3$)-H

[N-methyl-N-(tert.butyloxycarbonyl-L-tryptophyl-methylaminoethyl)]amin

Die Synthese erfolgt analog der in Beispiel 45f beschriebenen Methode aus 71a und Wasserstoff.
MS: 375,2 $(M+H)^{\oplus}$

Beispiel 71c

R5 = $OCOCH_2N(CH_3)CH_2CH_2N(CH_3)$-Trp-Boc
[N-Methyl-N-(2-tert.butyloxycarbonyl-tryptophyl-N-2-methylaminoethyl)amino]-acetyloxy-

R5* = OH
W = F.15

Die Synthese erfolgt analog der in Beispiel 45g beschriebenen Methode aus 71b und Verbindung aus Beispiel 1.
MS: 1447 $(M+H)^{\oplus}$

Beispiel 72

R5 = $OCOCH_2N(CH_3)CH_2CH_2CH_2N(CH_3)$-Arg-H
[N-Methyl-N-(2-L-arginyl)-N-(2-methyl-aminopropyl)amino]-acetyloxy-
R5* = OH
W = F.15

Die Abspaltung der Schutzgruppen erfolgt analog der in Beispiel 45h beschriebenen Methode aus 72d.
MS: 1431 $(M+H)^{\oplus}$

Beispiel 72a

Z-$N(CH_3)$-$CH_2$-$CH_2$-$CH_2$-N-$(CH_3)$-Z

Die Synthese erfolgt analog der in Beispiel 56a beschriebenen Methode aus N-(Benzyloxycarbonyloxy)-succinimid und N,N'-Dimethyl-1,3-propandiamin.
MS: 237 $(M+H)^{\oplus}$

Beispiel 72b

Boc-Arg-(Mtr)-$N(CH_3)$-$CH_2$-$CH_2$-$CH_2$-N-$(CH_3)$-Z

Die Synthese erfolgt analog der in Beispiel 45e beschriebenen Methode aus Boc-Arg(Mtr)-OH und Verbindung aus Beispiel 72a. MS: 706 $(M+H)^{\oplus}$

Beispiel 72c

Boc-Arg(Mtr)-$N(CH_3)$-$CH_2$-$CH_2$-$CH_2$-N-$(CH_3)$-H

Die Synthese erfolgt analog der in Beispiel 45f beschriebenen Methode aus 72b und Wasserstoff.
MS: 571,3 $(M+H)^{\oplus}$

Beispiel 72d

R5 = $OCOCH_2$-$N(CH_3)CH_2CH_2CH_2N(CH_3)$-Arg(Mtr)-Boc
R5* = OH
W = F.15

Die Synthese erfolgt analog der in Beispiel 45g beschriebenen Methode aus 72c und Verbindung aus Beispiel 1.
MS: 1743 $(M+H)^{\oplus}$

Beispiel 73

R5 = $OCOCH_2N(CH_3)CH_2CH_2CH_2N(CH_3)$-Arg-H

[N-Methyl-N-(2-L-arginyl)-N-2-methyl-aminopropyl)amino]-acetyloxy-
R5* = R5
W = F.15

Die Abspaltung der Schutzgruppen erfolgt analog der in Beispiel 45h beschriebenen Methode aus 73a.
MS: 1729 $(M+H)^{\oplus}$

Beispiel 73a

R5 = $OCOCH_2N(CH_3)CH_2CH_2CH_2N(CH_3)$-Arg(Mtr)-Boc
R5* = R5
W = F.15

Die Synthese erfolgt analog der in Beispiel 46a beschriebenen Methode aus 72c und Verbindung aus Beispiel 2.
MS: 2352 $(M+H)^{\oplus}$

Beispiel 74

R5 = $OCOCH_2CH_2CH_2(CH_3)CH_2CH_2N(CH_3)$-H
[N-Methyl-N-(2-methyl-aminoethyl)amino]-butyryloxy-
R5* = OH
W = F.15

Die Abspaltung der Schutzgruppen erfolgt analog der in Beispiel 45 beschriebenen Methode aus 74a.
$C_{72}H_{98}N_6O_{11}S_2$ M: 1287,9
MS: 1289 $(M+H)^{\oplus}$

Beispiel 74a

R5 = $OCOCH_2CH_2CH_2N(CH_3)CH_2CH_2N(CH_3)$-Boc
[N-Methyl-N-(2-methyl-aminoethyl)amino]-butyryloxy-
R5* = OH
W = F.15

Die Abspaltung erfolgt analog der in Beispiel 45g beschriebenen Methode aus 8a und Verbindung aus 43.
MS: 1389 $(M+H)^{\oplus}$

Beispiel 75

R5 = $OCOCH_2CH_2CH_2N(CH_3)CH_2CH_2N(CH_3)$-H
[N-Methyl-N-(2-methyl-aminoethyl)amino]-butyryloxy-
R5* = R5
W = F.15

Die Abspaltung der Schutzgruppen erfolgt analog der in Beispiel 45h beschriebenen Methode aus 75a.
MS: 1445 $(M+H)^{\oplus}$

Beispiel 75a

R5 = $OCOCH_2CH_2CH_2N(CH_3)CH_2CH_2N(CH_3)$-Boc
[N-Methyl-N-(N-Boc-methylaminoethyl)amino]-butyryloxy-
R5* = R5
W = F.15

Die Synthese erfolgt analog der in Beispiel 46a beschriebenen Methode aus 8a und Verbindung aus Beispiel 44.
MS: 1645 $(M+H)^{\oplus}$

Beispiel 76

R5 = $OCOCH_2$-N-$[CH_2PO(CH_3)_2]$-$CH_2CH_2$-$N(CH_3)$-H
R5* = OH
W = F.15

230 mg Produkt aus Beispiel 76d wurden 10 ml Eisessig/Methanol: 2/1 gelöst und mit 80 mg Katalysator (10 5 Pd/C) 40 min. bei RT hydriert. Der Katalysator wird abfiltriert und das Filtrat i. Vak. zur Trockne eingedampft. Es verbleiben 1110 mg der Titelverbindung.

Beispiel 76a

$CH_3$-NH-$CH_2CH_2Br$*HBr

2-Methylaminoethylbromid hydrobromid

26,2 g (0,35 mol) N-Methylaminoethanol werden in 200 ml Toluol gelöst, auf 0°C gekühlt und innerhalb 1 h mit 27,9 ml (0,36 mol) Thionylbromid versetzt. Die Reaktionslösung wird auf 60°C erwärmt und 45 min. heftig gerührt. Anschließend wird i. Vak. einrotiert. Die verbleibende zähe Masse wird in ca. 1 l siedendem Aceton aufgenommen, mit Aktivkohle versetzt und filtriert. Danach gibt man ca. 600 ml Diisopropylether zu und läßt zur Kristallisation bei 0°C stehen. Der Niederschlag wird abgesaugt, gründlich mit Diisopropylether gewaschen und sofort über KOH im Exsikkator getrocknet.
Ausbeute: 48 g

Beispiel 76b

Z-$(CH_3)$N-$CH_2CH_2Br$

(N-Benzyloxycarbonyl-2-methylaminoethyl)-bromid

10,94 g (0,05 mol) 2-Methylaminoethylbromid hdydrobromid werden in 80 ml Acetonitril gelöst und mit 12,5 g (0,05 mol) Z-OSu und 6,9 ml (0,05 mol) Triethylamin versetzt. Man rührt 45 min. bei RT, rotiert i. Vak. ein und nimmt den Kolbenrückstand in 150 ml Ethylacetat auf. Anschließend wäscht man mehrmals mit 10 %iger Citronensäurelösung und ges. Kochsalzlösung, trocknet mit Natriumsulfat, filtriert und engt das Filtrag i. Vak. zur Trockne ein. Das Rohprodukt wurde durch Chromatographie an Kieselgel gereinigt. (n-Heptan/Ethylacetat: 5/1)
Ausbeute: 7,8 g
MS: 272 $(M+H)^{\oplus}$

Beispiel 76c

Z-$(CH_3)N-CH_2CH_2-NH-CH_2PO(CH_3)_2$

N-Dimethyloxophosphorylmethyl-(N-benzyloxycarbonyl-2-methylaminoethyl)amin

2,7 g (10 mmol) (N-Benzyloxycarbonyl-2-methylamino)ethylbromid und 5,35 g (50 mmol) Dimethyloxophosphorylmethylamin werden zusammen mit 250 mg Silveroxid in 20 ml Acetonitril 24 h bei RT gerührt. Nach Abdampfen des Lösungsmittels reinigt man das Rohprodukt durch Chromatographie an Kieselgel (Dichlormethan/Methanol: 8/1)
Ausbbeute: 2,4 g Öl
MS: 299 $(M+H)^{\oplus}$

Beispiel 76d

R5 = $OCOCH_2-N-[CH_2PO(CH_3)_2]-CH_2CH_2-N(CH_3)-Z$
{[N-Benzyloxycarbonyl-N-methyl-N-(2-dimethyloxophosphorylmethyl)-amino-ethyl]amino}-acetyloxy-
R5* = OH
W = F.15

250,4 mg (0,2 mmol) Verbindung aus Beispiel 1, (0,4 mmol) 119,3 mg Verbindung aus Beispiel 76c und eine katalytische Menge von 10 mg Kaliumjodid werden zusammen in 2 ml Acetonitril gelöst und 12 h bei RT gerührt. Nach Abdampfen des Lösungsmittels wird das Rohprodukt durch Chromoatographie an Kieselgel gereinigt. (Dichlormethan/ Methanol: 15/1)
MS: 1470,5 $(M+H)^{\oplus}$

Beispiel 77

R5 = $OCOCH_2-N-[CH_2PO(CH_3)_2]-CH_2CH_2-N(CH_3)-H$
{[N-methyl-N-(2-dimethyloxophosphorylmethyl)-aminoethyl]amino}-acetyloxy-
R5* = R5
W = F.15

350 g Produkt aus Beispiel 77d wurden 10 ml Eisessig/Methanol: 2/1 gelöst und mit 80 mg Katalysator (10 % Pd/C) 40 min. bei RT hydriert. Der Katalysator wird abfiltriert und das Filtrat i. Vak. zur Trockne eingedampft. Es verbleiben 260 mg der Titelverbindung. MS: 1541 $(M+H)^{\oplus}$

Beispiel 77a

R5 = $OCOCH_2-N-[CH_2PO(CH_3)_2]-CH_2CH_2-N(CH_3)-Z$
{[N-Benzyloxycarbonyl-N-methyl-N-(2-dimethyloxophosphorylmethyl)-amino-ethyl]amino}-acetyloxy-
R5* = R5
W = F.15

Die Synthese erfolgt analog der in Beispiel 46a oder 76d beschriebenen Methode aus 76c und Verbindung aus Beispiel 2.
MS: 1808 $(M+H)^{\oplus}$

Beispiel 78

R5 = $OCO-CH_2-N(CH_3)CH_2CH_2-NH-CH_2PO(CH_3)_2$

{N-Methyl-N-[-(2-dimethyloxophosphorylmethyl)-aminoethyl]amino}-acetyloxy-
R5* = OH
W = F.15

Die Abspaltung der Schutzgruppen erfolgt analog der in Beispiel 45 beschriebenen Methode aus 78c. MS: 1336 $(M+H)^{\oplus}$

Beispiel 78a

$Z-(CH_3)N-CH_2CH_2-N(Boc)-CH_2PO(CH_3)_2$

N-tert.-Butyloxycarbonyl-[N-dimethyloxophosphorylmethyl-(N-benzyloxycarbonyl-2-methyl-aminoethyl)]amin

1,49 g (5 mmol) N-Dimathyloxophosphorylmethyl-(N-benzyloxycarbonyl-2-methyl-aminoethyl)amin 76c und 1,13 g (5,2 mmol) Di-tert.butyl-dicarbonat werden zusammen in 5 ml Acetonitril gelöst und 4 h bei RT gerührt. Anschließend rotiert man i. Vak. ein, nimmt den Rückstand in Ethylacetat auf und wäscht mehrmals mit 10 %iger Citronensäurelösung sowie ges. Kochsalzlösung. Man trocknet mit $Na_2SO_4$, filtriert und rotiert das Filtrat i. Vak. zur Trockne ein.
Ausbeute: 1,7 g Öl
MS: 399 $(M+H)^{\oplus}$

Beispiel 78b

$H-(CH_3)N-CH_2CH_2-N(Boc)-CH_2PO(CH_3)_2$

N-tert.Butyloxycarbonyl-[N-dimethyloxophosphorylmethyl-(N-2-methylaminoethyl)]amin

1,5 g N-tert.Butyloxycarbonyl-[N-dimethyloxophosphorylmethyl(N-benzyloxycarbonyl-2-methylaminoethyl)] amin werden in 20 ml Methanol mit 150 mg Katalysator (10 % Pd/Kohle) 4 h bei RT hydriert. Der Katalysator wird abfiltriert und das Filtrat i. Vak. zur Trockne eingedampft.
Ausbeute: 0,99 g Öl
MS: 265 $(M+H)^{\oplus}$

Beispiel 78c

R5 = $OCO-CH_2-N(CH_3)-CH_2CH_2-N(Boc)-CH_2PO(CH_3)_2$
R5* = OH

Die Synthese erfolgt analog der in Beispiel 45g beschriebenen Methode aus 78b und Verbindung aus Beispiel 1. MS: 1436 $(M+H)^{\oplus}$

Beispiel 79

R5 = $OCO-CH_2-N(CH_3)-CH_2CH_2-NH-CH_2PO(CH_3)_2$
{N-Methyl-N-[-(2-dimethyloxophosphorylmethyl)-aminoethyl]amino}-acetyloxy
R5* = R5
W = F.15

Die Abspaltung der Schutzgruppen erfolgt analog der in Beispiel 45 beschriebenen Methode aus 79a.
MS: 1541 $(M+H)^{\oplus}$

Beispiel 79a

R5 = $OCO\text{-}CH_2\text{-}N(CH_3)\text{-}CH_2CH_2\text{-}NH\text{-}CH_2PO(CH_3)_2$
{N-Methyl-n-[2-N-tert.butyloxycarbonyl-(2-dimethyloxophosphorylmethyl)aminoethyl]amino}-acetyloxy-
R5* = R5
W = F.15

Die Synthese erfolgt analog der in Beispiel 46a beschriebenen Methode aus 78b und Verbindung aus Beispiel 2.
MS: 1740 $(M+H)^{\oplus}$

Beispiel 80

R5 = $OCOCH_2\text{-}N\text{-}[CH_2CO_2H]\text{-}CH_2CH_2\text{-}N(CH_3)\text{-}H$
R5* = OH
W = F.15

Die Abspaltung der Schutzgruppen erfolgt analog der in Beispiel 45h beschriebenen Methode aus 80c.

Beispiel 80a

$Z\text{-}(CH_3)N\text{-}CH_2CH_2\text{-}NH\text{-}CH_2COOCH(CH_3)_3$

N-tert.Butyloxycarbonylmethyl-(N-benzyloxycarbonyl-2-methylaminoethyl)amin

6,5 g (50 mmol) Glycin-tert.butylester, 2,7 g (10 mmol) (N-Benzyloxycarbonyl-2-methylaminoethyl)bromid [76b] und 100 mg Silberoxid werden in 20 ml Acetonitril 24 h bei RT gerührt. Die Reaktionslösung wird filtriert und das Filtrat i. Vak. eingedampft. Der Rückstand wird in Ethylacetat aufgenommen und zur Entfernung von H-Gly-OBut mehrmals mit Wasser und ges. NaCl-Lösung gewaschen. Die Ethylacetatlösung wird mit $Na_2SO_4$ getrocknet, filtriert und i. Vak. eingedampft. Das Rohprodukt wird durch Chromatographie an Kieselgel gereinigt. (Dichlormethan/Methanol: 30/1)
Ausbeute: 2,7 g Öl
MS: 323 $(M+H)^{\oplus}$

Beispiel 80b

R5 = $OCOCH_2\text{-}N\text{-}[CH_2CO_2{}^tBu]\text{-}CH_2CH_2\text{-}N(CH_3)\text{-}Z$
{[N-Benzyloxycarbonyl-N-methyl-N(tert.-butyloxycarbonylmethyl)-amino-ethyl]amino}-acetyloxy-
R5* = OH
W = F.15

Die Synthese erfolgt analog der in Beispiel 45g beschriebenen Methode aus 80a und Verbindung aus Beispiel 1. MS: 1495 $(M+H)^{\oplus}$

Beispiel 80c

R5 = $OCOCH_2\text{-}N\text{-}[CH_2CO_2{}^tBu]\text{-}CH_2CH_2\text{-}N(CH_3)\text{-}H$
{[N-methyl-N-(tert.butyloxycarbonylmethyl)-aminoethyl]amino}-acetyloxy-
R5* = OH
W = F.15

550 mg Verbindung 80b wurden in 10 ml Methanol gelöst und mit 100 mg Katalysator (10 % Pd/C) 2 h bei RT hydriert. Der Katalysator wurde durch Filtration entfernt und das Filtrat i. Vak. zur Trockne eingedampft. Der Rückstand wurde mit Diethylether verrieben, abgesaugt, gewaschen und im Exsikkator getrocknet.
Ausbeute: 411 mg des Diacetats
MS: 1361 (M+H)⊕

Beispiel 81

R5 = $OCOCH_2$-N-[$CH_2CO_2{}^tBu$]-$CH_2CH_2$-N($CH_3$)-H
R5* = R5
W = F.15

200 mg 81a wurden in 10 ml Methanol/Eisessig (1/1) gelöst und mit 20 mg Katalysator (10 % Pd/C) 1 h bei RT hydriert. Nach Entfernung des Katalysators und Lösungsmittels wurde der Rückstand mit Diethylether verrieben, abgesaugt, gewaschen und im Exsikkator getrocknet.
Ausbeute: 140 mg des Tetraacetates
MS: 1588,8 (M+H)⊕

Beispiel 81a

R5 = $OCOCH_2$-N-[$CH_2CO_2{}^tBu$]-$CH_2CH_2$-N($CH_3$)-Z
R5* = R5
W = F.15

Die Synthese erfolgt analog der in Beispiel 46a beschriebenen Methode aus 80a und Verbindung aus Beispiel 2. MS: 1857,2 (M+H)⊕

Beispiel 82

R5 = $OCOCH_2$-N($CH_3$)-$CH_2CH_2$-N(Boc)$CH_2COOCH(CH_3)_3$
{N-Methyl-N-[2-N-tert.butyloxycarbonyl-(2-tert.-butyloxycarbonylmethyl)-amino-ethyl]-amino}-acetyloxy-
R5* = OH
W = F.15

Die Synthese erfolgt analog der in Beispiel 45g beschriebenen Methode aus 82b und Verbindung aus Beispiel 1.
MS: 1459,7 (M+H)⊕

Beispiel 82a

Z-N($CH_3$)-$CH_2CH_2$-N(Boc)-$CH_2COOCH(CH_3)_3$

N-tert.Butyloxycarbonyl-[N-tert.butyloxycarbonylmethyl-(N-benzyloaxycarbonyl-2-methyl-amino-ethyl)]amin

2,25 g (7 mmol) N-tert.Butyloxycarbonylmethyl-(N-benzyloxycarbonyl-2-methyl-aminoethyl)amin [80a] und 2,32 g (7,2 mmol) Di-tert.butyldicarbonat werden zusammen in 10 ml Acetonitril 4 h bei RT gerührt. Anschließend wird einrotiert und der Rückstand durch Chromatographie an Kieselgel gereinigt.
(Dichlormethan/Methanol: 60/1)
Ausbeute: 2,2 Öl
MS: 423 (M+H)⊕

Beispiel 82b

$H-(CH_3)N-CH_2CH_2-N(Boc)-CH_2COOCH(CH_3)_3$

N-tert.Butyloxycarbonyl-[N-tert.butyloxycarbonylmethyl-(2-methylaminoethyl)amin

2 g N-tert.butyloxycarbonyl-N-(tert.butyloxycarbonylmethyl-(N-benzyloxycarbonyl-2-methyl-aminoethyl)amin werden in 20 ml Methanol gelöst und mit 100 mg Katalysator (10 % Pd/C) 4 h bei RT hydriert. Der Katalysator wird abfiltriert und das Filtrat zur Trockne eingedampft.
Ausbeute: 1,4 g Öl
MS: 289 $(M+H)^{\oplus}$

Beispiel 83

R5 = $OCOCH_2-N(CH_3)-CH_2CH_2-N(Boc)-CH_2COOCH(CH_3)_3$
{N-Methyl-N-[2-N-tert.butyloxycarbonyl-(2-tert.butylocycarbonylmethyl)-amino-ethyl]-amino}-acetyloxy-
R5* = R5
W = F.15

Die Synthese erfolgt analog der in Beispiel 46a beschriebenen Methode aus 82b und Verbindung aus Beispiel 2.
MS: 1789 $(M+H)^{\oplus}$

Beispiel 84

Bestimmung der pharmakokinetischen Eigenschaften der Verbindungen der Formel I

Die Präparate wurden NMRI-Mäusen subkutan, intravenös oder oral mittels Schlundsonde appliziert. Zu bestimmten Zeitpunkten wurde je 2 oder 3 Mäusen unter Narkose eine Herzpunktion durchgeführt. Die Narkose erfolgt durch intraperitoneale Applikation von 0.3 ml einer Urethanlösung (0.2 mg/ml). Das entnommene Blut wurde bis zur Gerinnung bei 4°C aufbewahrt und anschließend zentrifugiert. Das so gewonnene Serum wurde zur Reinigung nochmals zentrifugiert (Eppendorff-Zentrifuge Modell 5414,5 min.). Bis zur Analyse wurde das Serum bei -20°C gelagert. Die Analyse der Proben erfolgte durch Ether-Extraktion und anschließende Auftrennung mittels RP-HPLC. Die Ergebnisse wurden graphisch dargestellt. In Fig. 1 (s.c-Applikation) und in Fig. 2 (i.v.-Applikation) ist die Serum-Konzentration des Wirkstoffes F.15 aufgetragen gegen die Zeit nach der Applikation des Wirkstoffs F.15 sowie nach der Applikation verschiedener Verbindungen aus den oben beschriebenen Beispielen. Neben der verbesserten Wasserlöslichkeit der Prodrugverbindungen läßt sich auch eine verbesserte Pharmakokinetik nachweisen.

## Patentansprüche

1. Verbindungen der Formel I worin mehrfach vorkommende gleich definierte Reste unabhängig voneinander sein können

$$W\text{-}[R^5]_a \qquad I,$$

worin W einen mono-, bis- oder tris-deshydroxylierten Rest einer Verbindung der nachfolgenden Formeln bedeutet

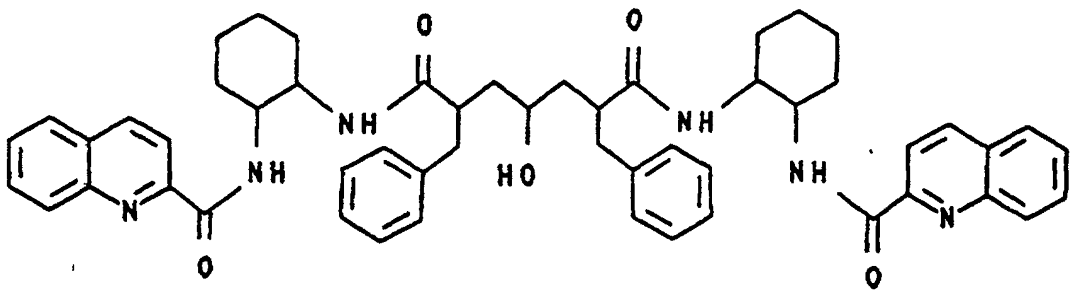

F. 1

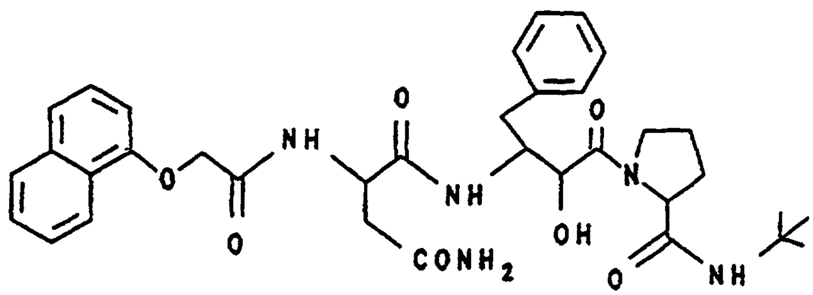

F. 2

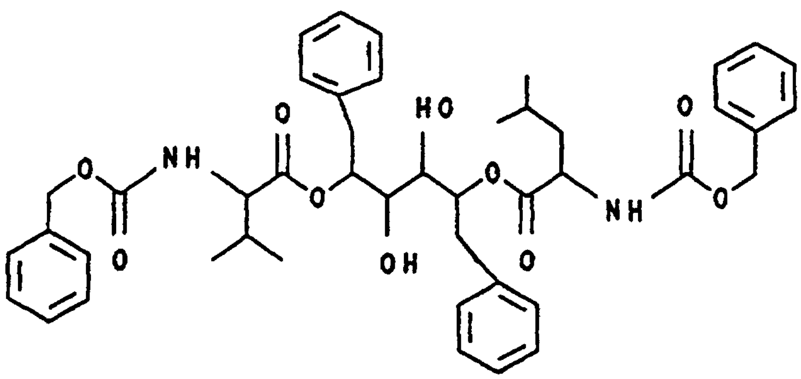

F. 3

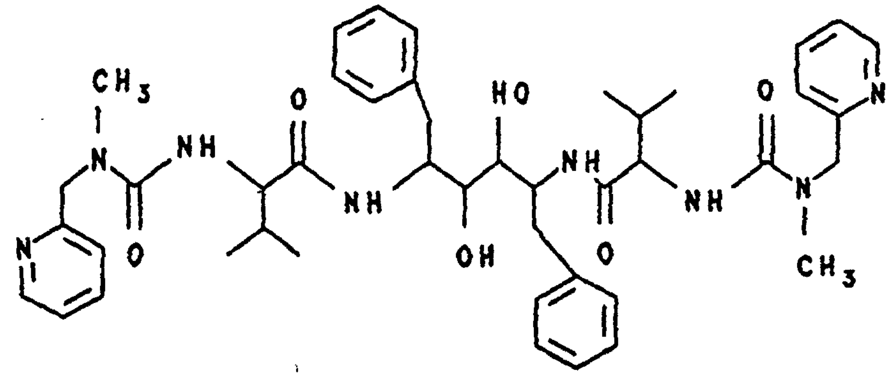

F. 4

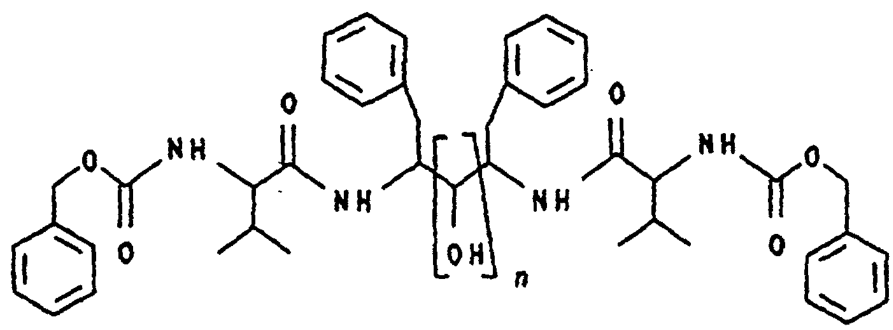

F. 5

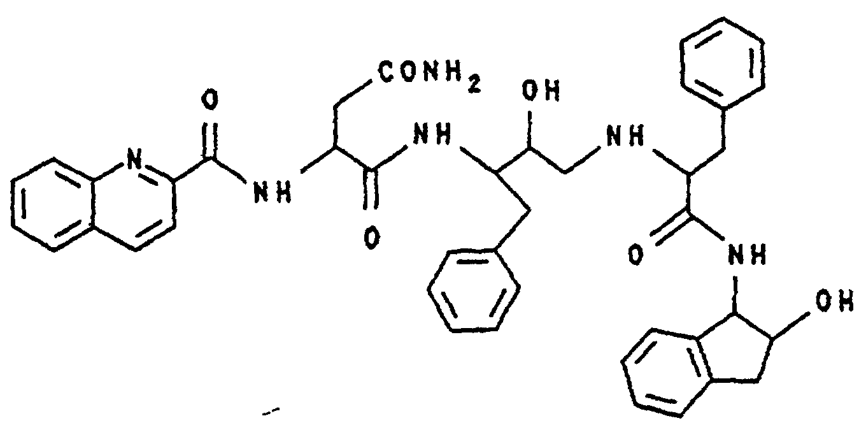

F. 6

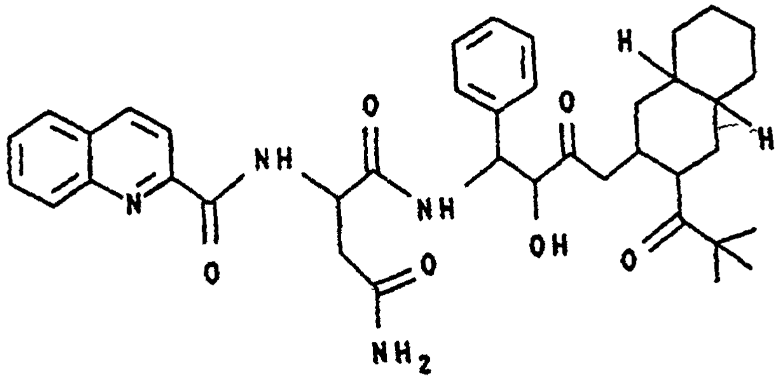

F. 7

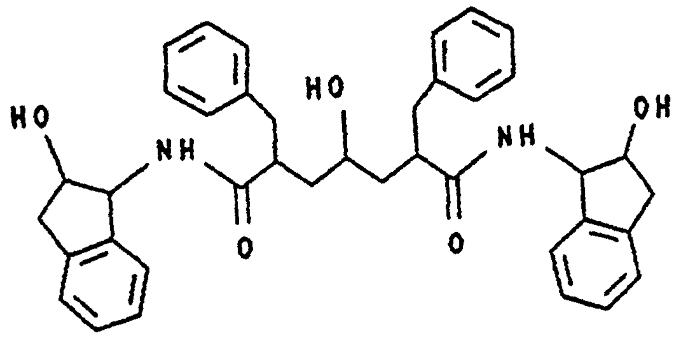

F. 8

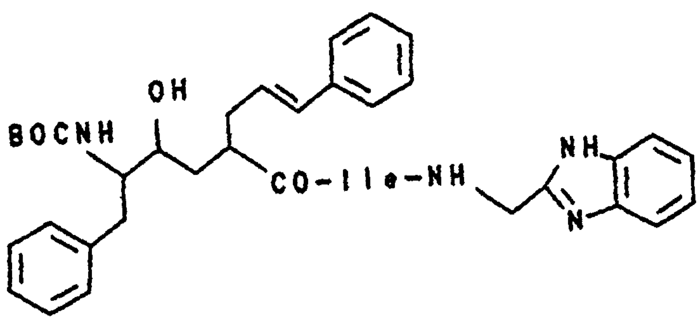

F. 9

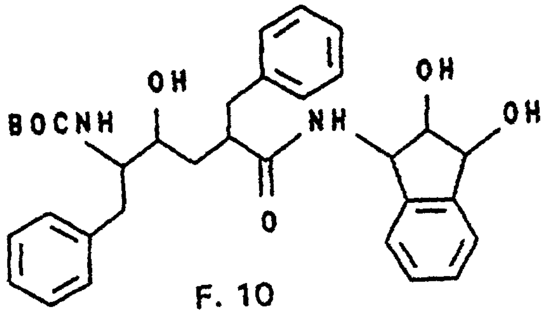

F. 10

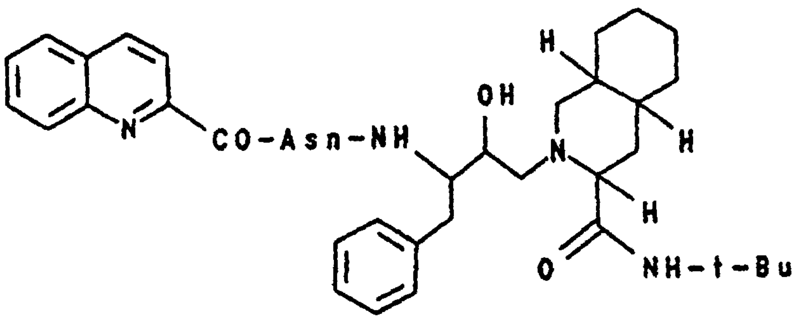

F. 11

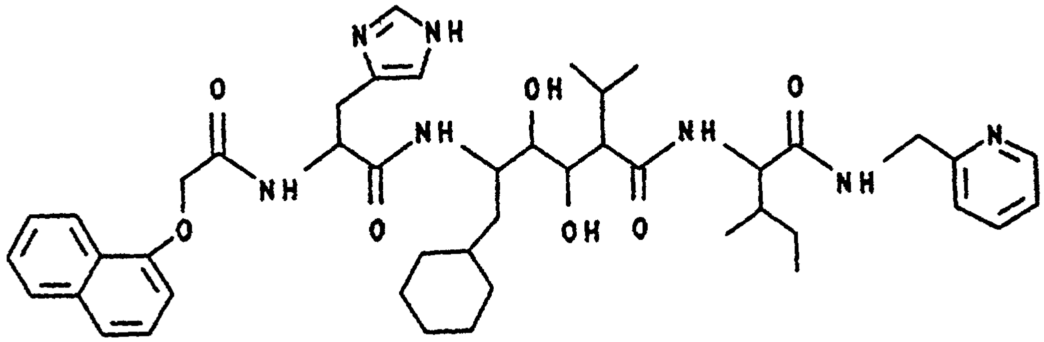

F. 12

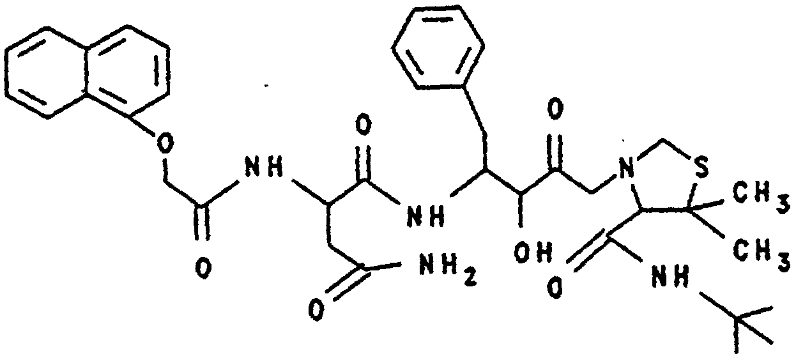

F. 13

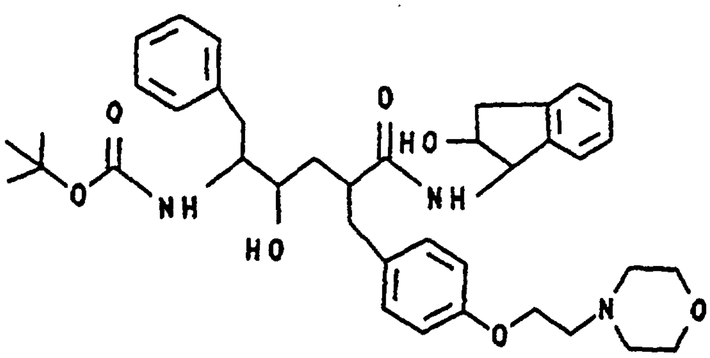

F. 14

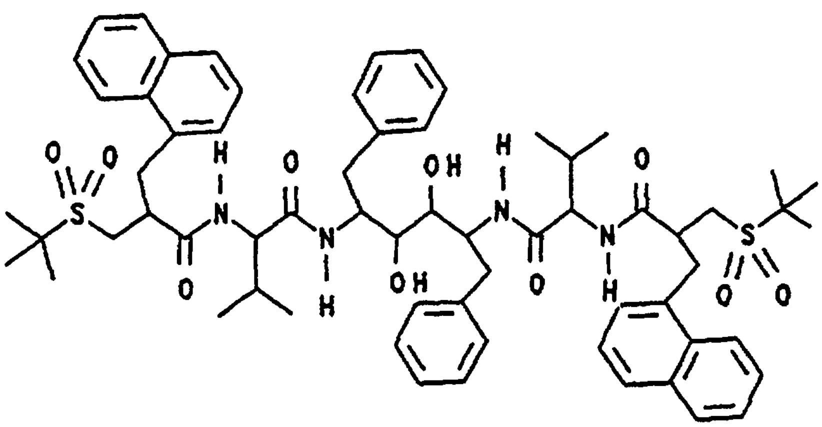

F. 15

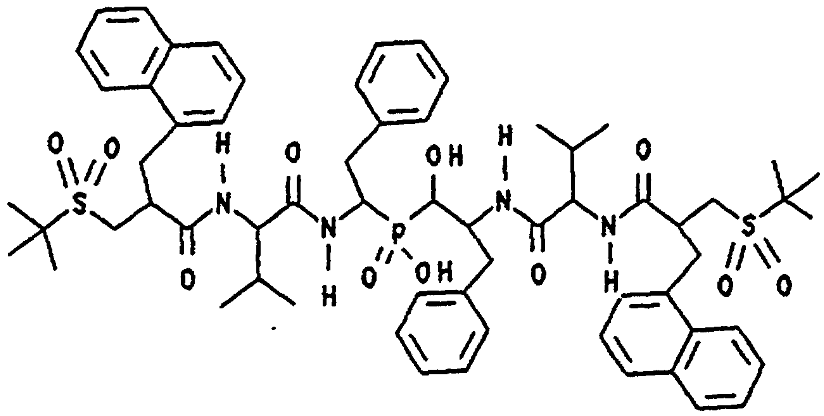

F. 16

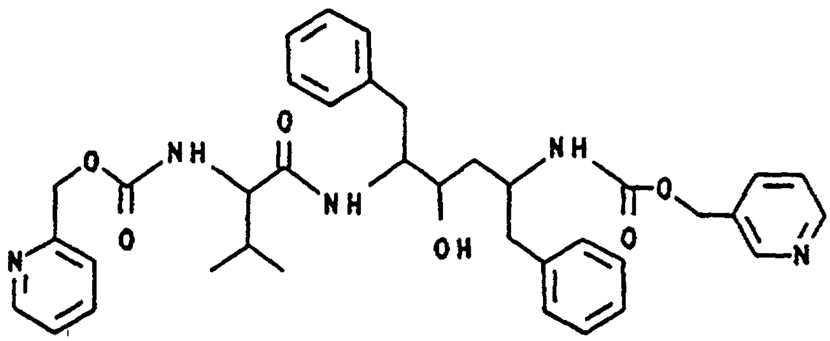

F. 17

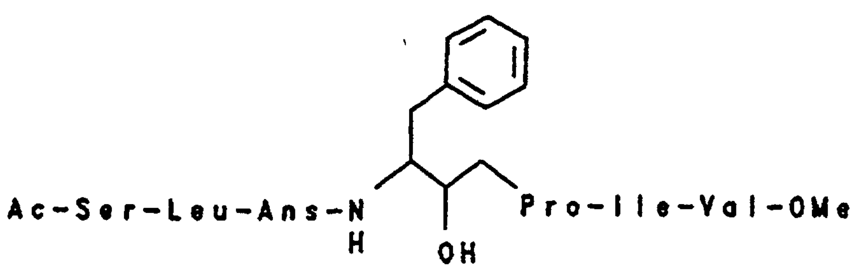

F. 18

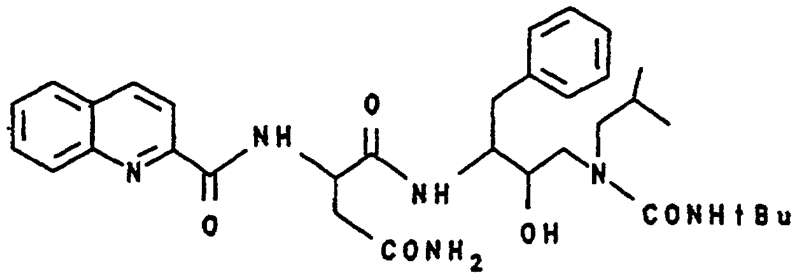

F. 19

a 1, 2 oder 3 bedeutet und $R^5$ für einen Rest der Formel III, steht

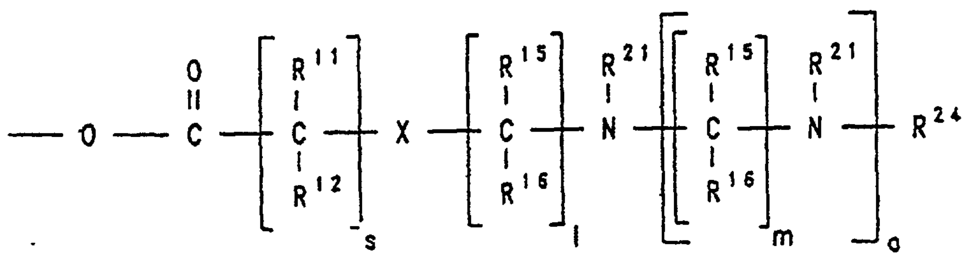

(III),

X steht für O, S, $NR^{20}$ oder $N^+(R^{20})_2$, bevorzugt für S oder $NR^{20}$,

wobei

| | |
|---|---|
| $R^{20}$ | H, $(C_1-C_6)$-Alkyl, $(C_3-C_7)$-Cycloalkyl, $-CH_2-(CH_2)_n-NR^{21}R^{24}$, $-CH_2-C(O)-R^{28}$, $CH_2CONH(C_1-C_6)$-Alkyl, das mit bis zu 5 OH-Gruppen substituiert sein kann, COO $(C_1-C_6)$-Alkyl, oder $-CH_2-P(O)((C_1-C_4)\text{-Alkyl})_2$ bedeutet, bevorzugt H, $(C_1-C_4)$-Alkyl, $-CH_2-C(O)-R^{28}$ oder $-CH_2-P(O)Me_2$, besonders bevorzugt H, $(C_1-C_4)$-Alkyl bedeutet, |
| $R^{11}$, $R^{12}$, $R^{15}$ und $R^{16}$ | bedeuten unabhängig voneinander H oder $(C_1-C_4)$-Alkyl, bevorzugt H oder Methyl, |
| $R^{21}$ | bedeutet H, $(C_1-C_6)$-Alkyl, $(C_3-C_7)$-Cycloalkyl oder $(C_1-C_4)$-Alkoxycarbonyl, bevorzugt H, $(C_1-C_4)$-Alkyl, |
| $R^{24}$ | bedeutet H, $(C_1-C_{18})$-Alkyl, $(C_3-C_{14})$-Cycloalkyl, $(C_2-C_{18})$-Alkenyl, $(C_2-C_{18})$-Alkinyl oder $(C_6-C_{14})$-Aryl, die jeweils einfach, zweifach oder dreifach durch $(C_6-C_{14})$-Aryl, $(C_6-C_{14})$-Aryloxy, $(C_3-C_{14})$-Cycloalkyl, Hydroxy, $(C_1-C_4)$-Alkoxy, $-C(O)-R^{28}$, P(O)( $(C_1-C_4)$-Alkyl$)_2$ oder Halogen substituiert sein können und direkt oder gegebenenfalls über CO mit $NR^{21}$ verknüpft sind, oder eine über die Carbonylgruppe verknüpfte *a*-Aminosäure, oder $R^{24}$ bedeutet $((C_1-C_4)\text{-Alkyl})-N((C_1-C_4)\text{-Alkyl})-(C_1-C_4)\text{-Alkyl-NH-}((C_1-C_4)\text{-Alkyl})$ oder $R^{24}$ bildet mit $R^{21}$ einen Heterocyclus, bevorzugt bedeutet |
| $R^{24}$ | H, $(C_1-C_4)$-Alkyl, das gegebenenfalls durch Phenyl, $NH_2$ oder $(C_3-C_7)$-Cycloalkyl substituiert sein kann und direkt oder gegebenenfalls über CO mit $NR^{21}$ verknüpft ist, eine natürliche, über die Carbonylgruppe verknüpfte α-Aminosäure, $-CH_2C(O)R^{28}$, $-CH_2-P(O)Me_2$, $-(CH_2)_3N(Me)(CH_2)_2NHMe$, besonders bevorzugt bedeutet $R^{24}$ H, oder $(C_1-C_4)$-Alkyl, |

$R^{28}$ steht für OH, O-($C_1$-$C_6$)-Alkyl, O-($C_3$-$C_6$)-Cycloalkyl, $NH_2$, -NH-($C_1$-$C_6$)-Alkyl, das mit bis zu 5 OH-Gruppen substituiert sein kann, -NH-($C_3$-$C_7$)-Cycloalkyl oder -N-bis($C_1$-$C_4$)-Alkyl, bevorzugt steht $R^{28}$ für OH, $NH_2$, O($C_1$-$C_4$)-Alkyl, -NH-($C_1$-$C_4$)-Alkyl, oder -N-bis($C_1$-$C_4$)-Alkyl,besonders bevorzugt steht $R^{28}$ unabhängig voneinander für OH, $NH_2$ oder O($C_1$-$C_4$)-Alkyl,

worin

l 2 oder 3, bevorzugt 2,
s 1, 2,3, 4 oder 5, bevorzugt 1, 4 oder 5, besonders bevorzugt 1,
m 0, 1, 2, 3 oder 4, bevorzugt 2 oder 3,
n 1, 2 oder 3, bevorzugt 1 oder 2,
o 0, 1, 2 oder 3, bevorzugt 0, 1 oder 2, besonders bevorzugt 0,

**2.** Verfahren zur Herstellung von Verbindungen der Formel I gemäß Anspruch 1, **dadurch gekennzeichnet, daß** ein Wirkstoff der Formel W-$(OH)_b$ wobei W wie oben definiert ist und b 1, 2 oder 3 sein kann, aber größer oder gleich a ist mit A) einer Verbindung der Formel (X)

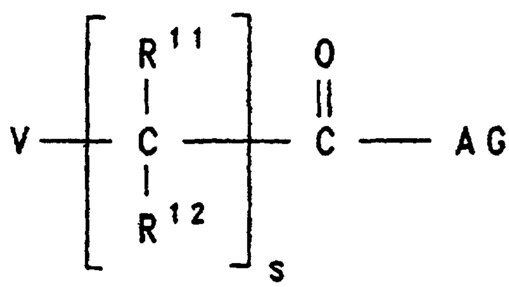

(X)

zu einer Verbindung der Formel (XX)

$$[OH]_{b-a}\text{-W-}[OCO\text{-}(CR^{11},R^{12})_s\text{-}V]_a \qquad (XX)$$

umgesetzt wird
und anschließend B) die resultierende Verbindung der Formel (XX) ihrerseits mit Nucleophilen der Formel (XI), umgesetzt wird

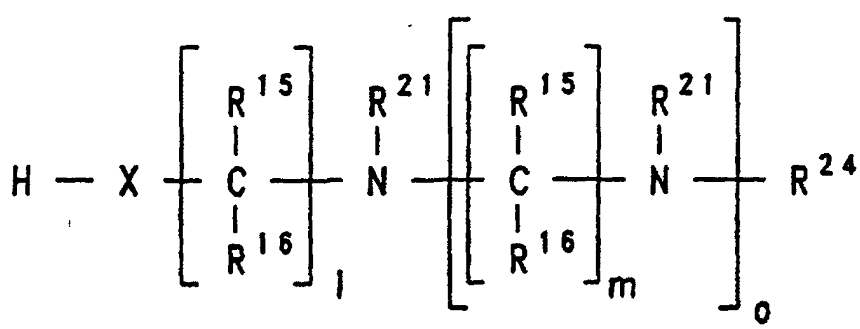

(XI)

wobei W, a, b, $R^{11}$, $R^{12}$, $R^{15}$, $R^{16}$, $R^{21}$, $R^{24}$, l, m, o, und s die obengenannten Bedeutungen haben, V ist eine

geeignete Abgangsgruppe, bevorzugt Br, Cl, OTs, besonders bevorzugt Br,
AG steht für eine zur Veresterung geeignete Abgangsgruppe, bevorzugt Br oder Cl oder eine Gruppe wie sie sich aus den Aktivestermethoden für Carbonsäuren ergibt.

**3.** Eine Verbindung der Formel I gemäß Anspruch 1 zur Anwendung als Arzneimittel.

**4.** Verwendung einer Verbindung der Formel I gemäß Anspruch 1 zur Herstellung von Arzneimitteln zur Behandlung von Viruserkrankungen.

**5.** Arzneimittel, enthaltend eine wirksame Menge einer Verbindung gemäß Anspruch 1.

**6.** Verwendung einer Verbindung gemäß Anspruch 1 zur Behandlung des erworbenen Immunschwäche Syndroms oder des ARC (Äids related complex).

## Claims

**1.** A compound of the formula I in which radicals which occur several times and are defined in the same way can be independent of one another

$$W\text{-}[R^5]_a \qquad I$$

in which W is a mono-, bis- or tris-dehydroxylated radical of a compound of the following formulae

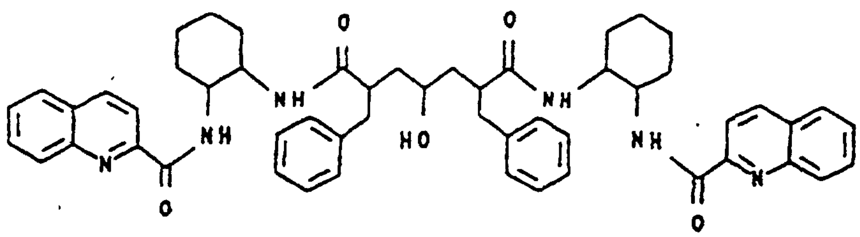

F. 1

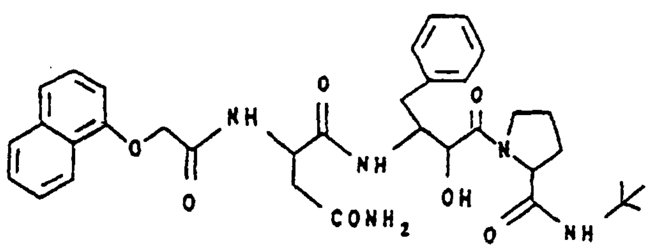

F. 2

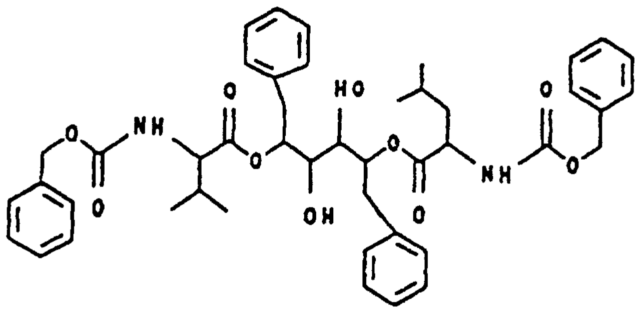

F. 3

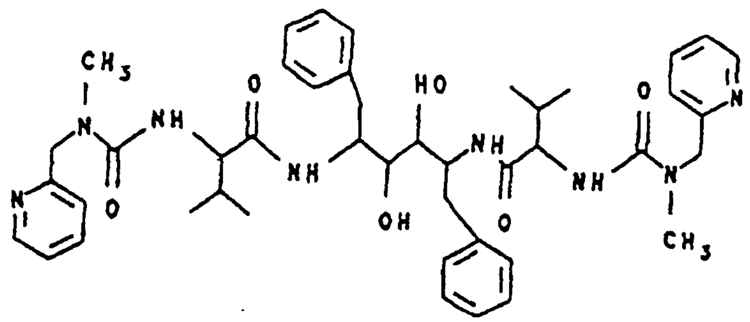

F. 4

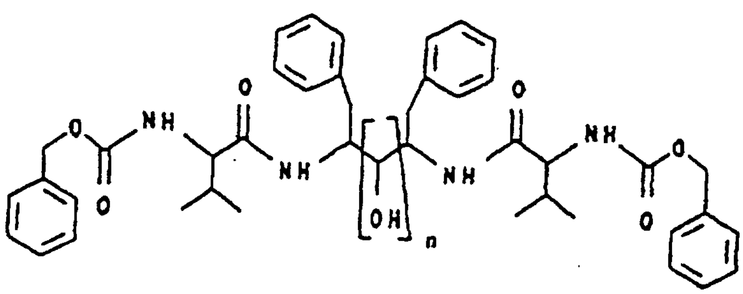

F. 5

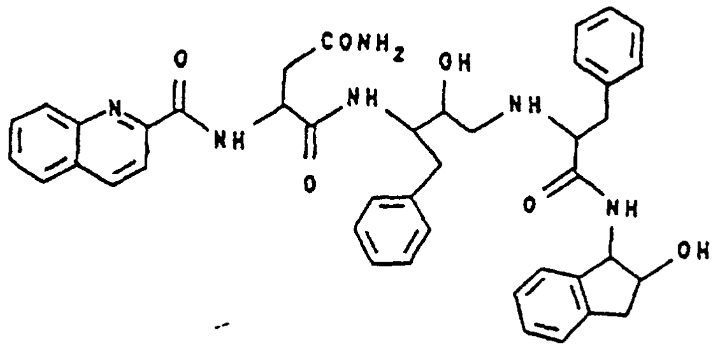

F. 6

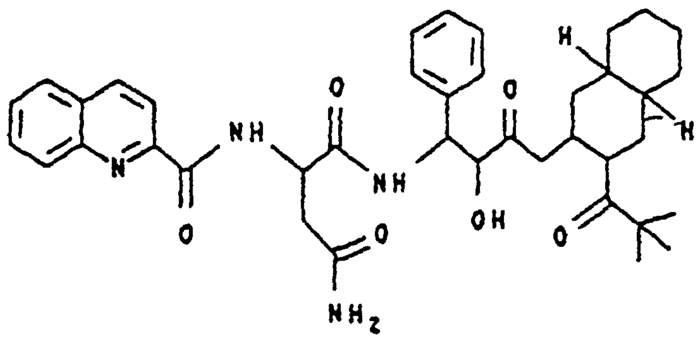

F. 7

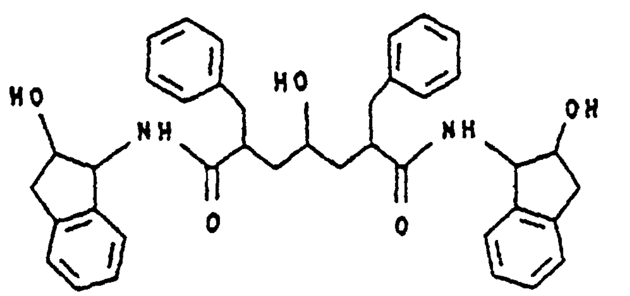

F. 8

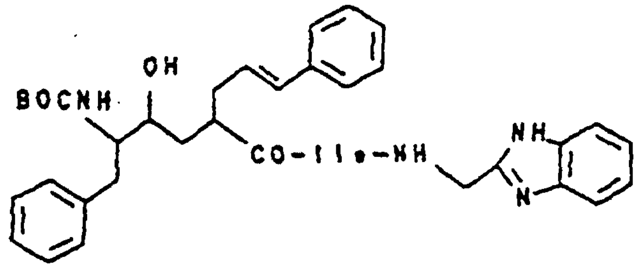

F. 9

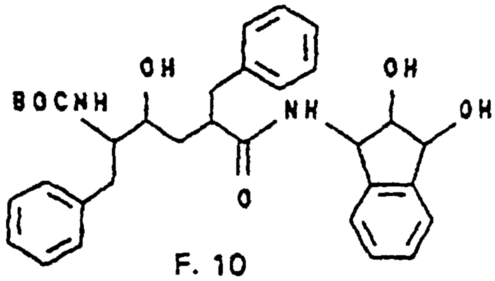

F. 10

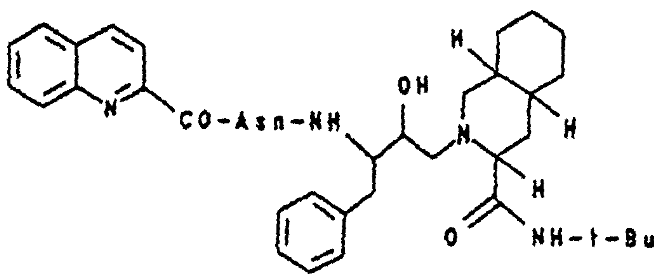

F. 11

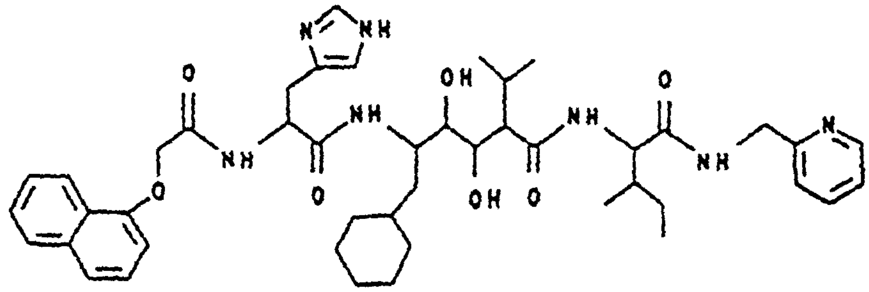

F. 12

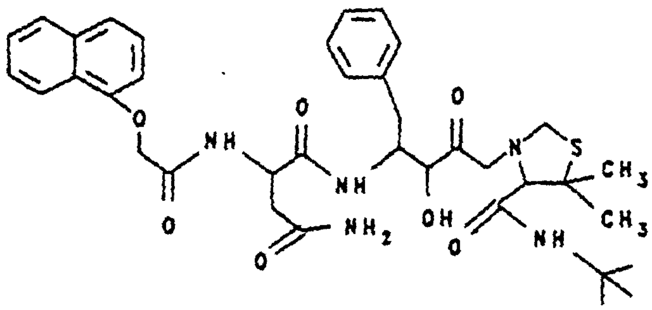

F. 13

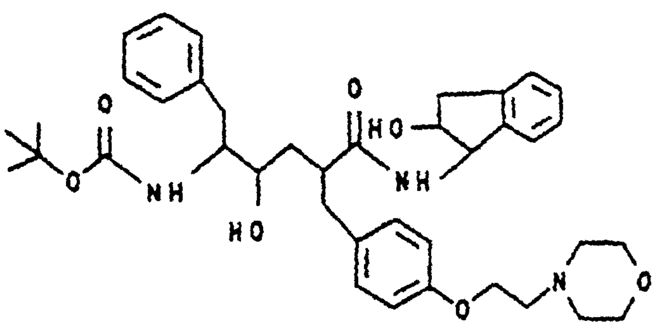

F. 14

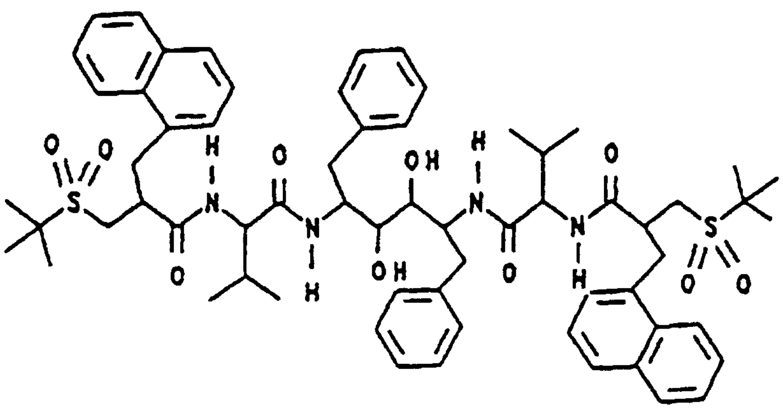

F. 15

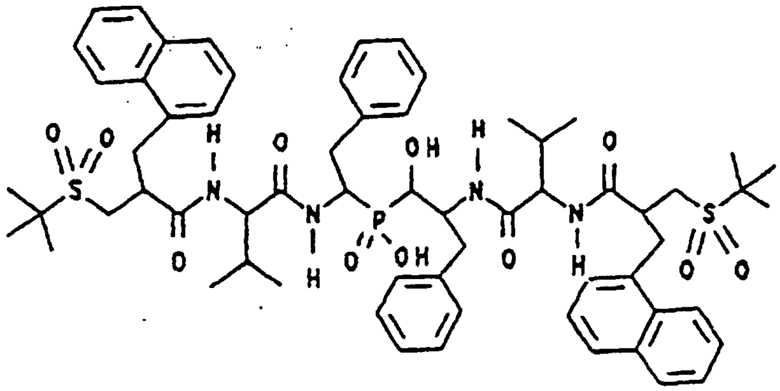

F. 16

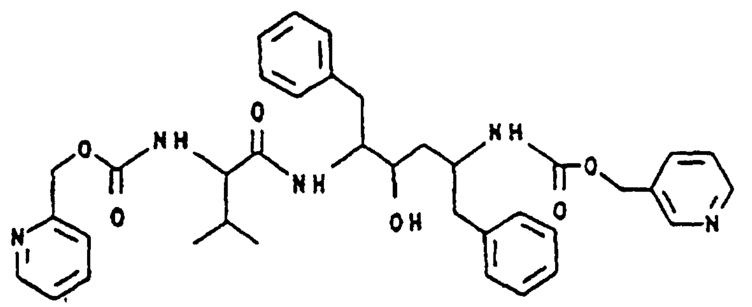

F. 17

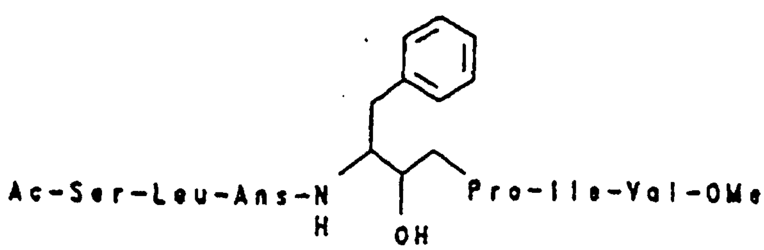

F. 18

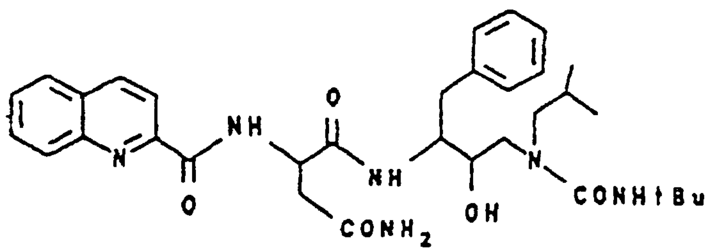

F. 19

a is 1, 2 or 3 and $R^5$ is a radical of the formula III, IV or V

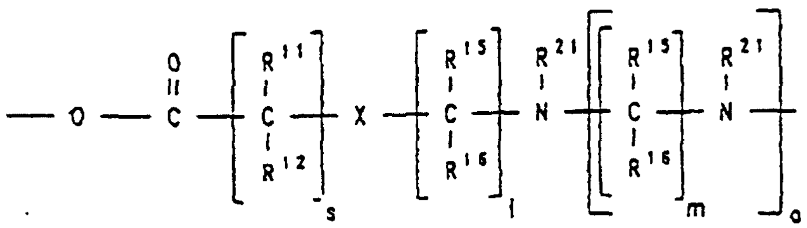 (III).

X is O, S, $NR^{20}$ or $N^+(R^{20})_2$, preferably S or $NR^{20}$,

in which

| | |
|---|---|
| $R^{20}$ | is H, $(C_1-C_6)$-alkyl, $(C_3-C_7)$-cycloalkyl, $-CH_2-(CH_2)_n-NR^{21}R^{24}$, $-CH_2-C(O)-R^{28}$, $CH_2CONH(C_1-C_6)$-alkyl, which can be substituted by up to 5 OH groups, $COO(C_1-C_6)$-alkyl or $-CH_2-P(O)((C_1-C_4)$-alkyl$)_2$, preferably H, $(C_1-C_4)$-alkyl, $-CH_2-C(O)-R^{28}$ or $-CH_2-P(O)Me_2$, particularly preferably H or $(C_1-C_4)$-alkyl, |
| $R^{11}$, $R^{12}$, $R^{15}$ and $R^{16}$ | independently of one another are H or $(C_1-C_4)$-alkyl, preferably H or methyl, |
| $R^{21}$ | is H, $(C_1-C_6)$-alkyl, $(C_3-C_7)$-cycloalkyl or $(C_1-C_4)$-alkyloxycarbonyl, preferably H or $(C_1-C_4)$-alkyl, |
| $R^{24}$ | is H, $(C_1-C_{18})$-alkyl, $(C_3-C_{14})$-cycloalkyl, $(C_2-C_{18})$-alkenyl, $(C_2-C_{18})$-alkynyl or $(C_6-C_{14})$-aryl, each of which can be mono-, di- or trisubstituted by $(C_6-C_{14})$-aryl, $(C_6-C_{14})$-aryloxy, $(C_3-C_{14})$-cycloalkyl, hydroxyl, $(C_1-C_4)$-alkoxy, $-C(O)-R^{28}$, $P(O)((C_1-C_4)$-alkyl$)_2$ or halogen and are linked with $NR^{21}$ directly or optionally via CO, or an $\alpha$-amino acid linked via the carbonyl group, or $R^{24}$ is $((C_1-C_4)$-alkyl$)-N((C_1-C_4)$-alkyl$)-(C_1-C_4)$-alkyl-NH-$((C_1-C_4)$-alkyl$)$, or $R^{24}$ forms a heterocyclic radical with $R^{21}$, and |
| $R^{24}$ | is preferably H, $(C_1-C_4)$-alkyl, which can optionally be substituted by phenyl, $NH_2$ or $(C_3-C_7)$-cycloalkyl and is linked with $NR^{21}$ directly or optionally via CO, a naturally occurring $\alpha$-amino acid linked via the carbonyl group, $-CH_2C(O)R^{28}$, $-CH_2-P(O)Me_2$ or $-(CH_2)_3N-(Me)(CH_2)_2NHMe$, and $R^{24}$ is particularly preferably H or $(C_1-C_4)$-alkyl, |
| $R^{28}$ | is OH, $O-(C_1-C_6)$-alkyl, $O-(C_3-C_6)$-cycloalkyl, $NH_2$, $-NH-(C_1-C_6)$-alkyl, which can be substituted by up to 5 OH groups, $-NH-(C_3-C_7)$-cycloalkyl or $-N$-bis$(C_1-C_4)$-alkyl, and $R^{28}$ is preferably OH, $NH_2$, $O(C_1-C_4)$-alkyl, $-NH-(C_1-C_4)$-alkyl or $-N$-bis$(C_1-C_4)$-alkyl, and $R^{28}$ is particularly preferably OH, $NH_2$ or $O(C_1-C_4)$-alkyl, |

in which

- l can be 2 or 3, preferably 2,
- s can be 1, 2, 3, 4 or 5, preferably 1, 4 or 5, particularly preferably 1,
- m can be 0, 1, 2, 3 or 4, preferably 2 or 3,
- n can be 1, 2 or 3, preferably 1 or 2 and
- o can be 0, 1, 2 or 3, preferably 0, 1 or 2, particularly preferably 0.

**2.** A process for the preparation of a compound of the formula I as claimed in claim 1, which comprises reacting an active compound of the formula $W\text{-}(OH)_b$, in which W is as defined above and b can be 1, 2 or 3, but is greater than or equal to a,
with

A) a compound of the formula (X)

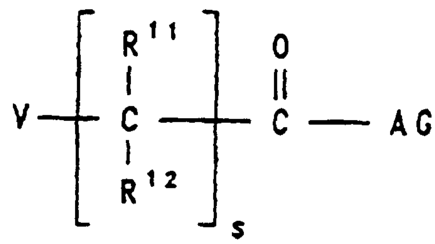

(X)

to give a compound of the formula (XX)

$$[OH]_{b-a}\text{-}W\text{-}[OCO\text{-}(CR^{11},R^{12})_s\text{-}V]_a \qquad (XX)$$

and then
B) reacting the resulting compound of the formula (XX) in turn with a nucleophile of the formula (XI)

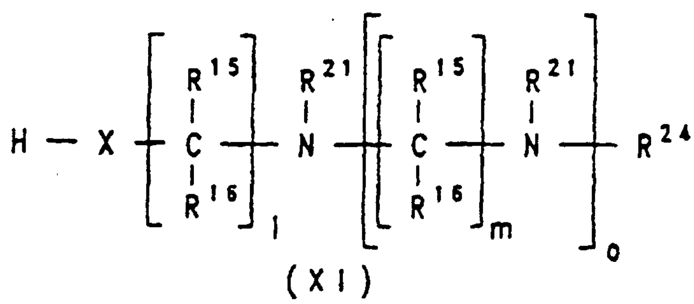

(XI)

in which W, a, b, $R^{11}$, $R^{12}$, $R^{15}$, $R^{16}$, $R^{21}$, $R^{24}$, l, m, o, and s have the abovementioned meanings, V is a suitable leaving group, preferably Br, Cl or OTs, particularly preferably Br, and
AG is a leaving group which is suitable for esterification, preferably Br or Cl, or a group such as results from the active ester methods for carboxylic acids.

**3.** A compound of the formula I as claimed in claim 1 for use as a medicament.

**4.** The use of a compound of the formula I as claimed in claim 1 for the preparation of a medicament for the treatment

of viral diseases.

**5.** A medicament comprising an active amount of a compound as claimed in claim 1.

**6.** The use of a compound as claimed in claim 1 for treatment of acquired immune deficiency syndrome or ARC (AIDS-related complex).

**Revendications**

**1.** Composés de formule I

$$W\text{-}[R^5]_a \qquad I,$$

dans laquelle des radicaux qui existent plusieurs fois et qui sont définis de la même manière peuvent être indépendants l'un de l'autre où W est un radical mono-, bis- ou tris-déshydroxylé d'un composé ayant les formules suivantes

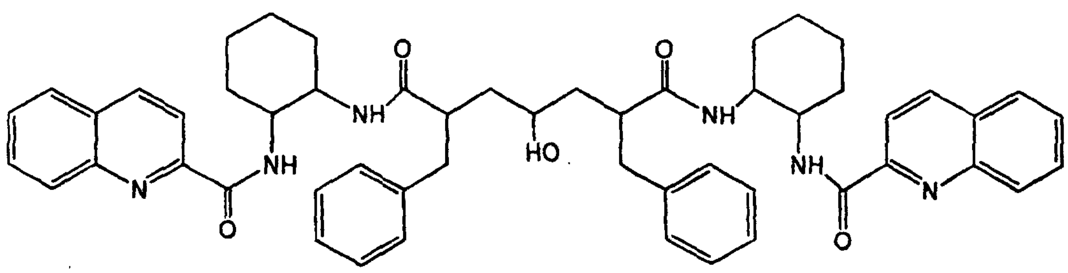

F. 1

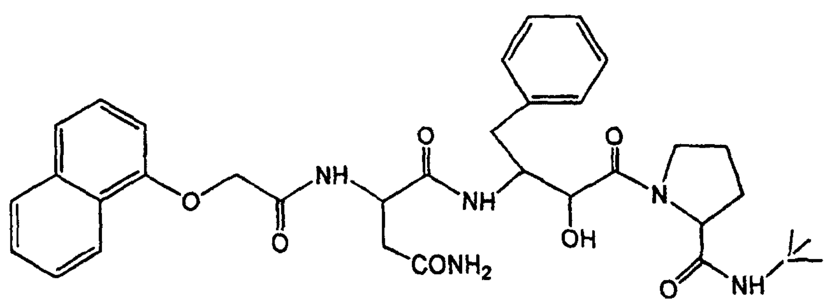

F. 2

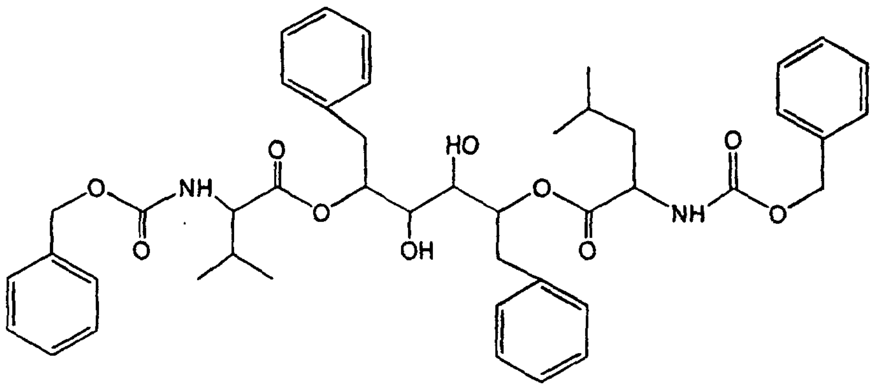

F. 3

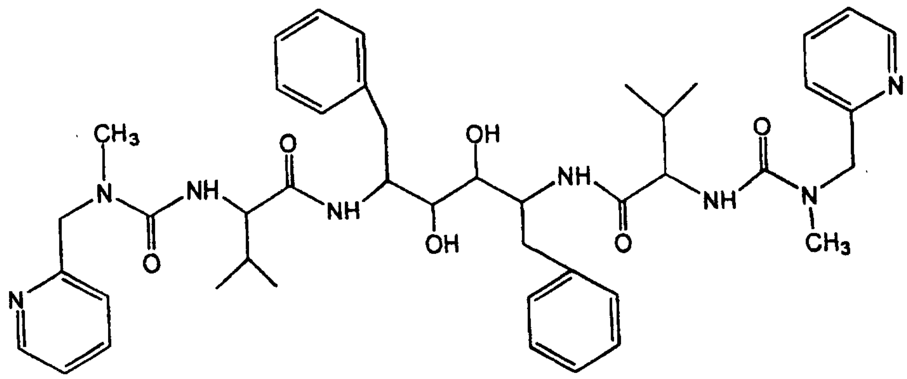

F. 4

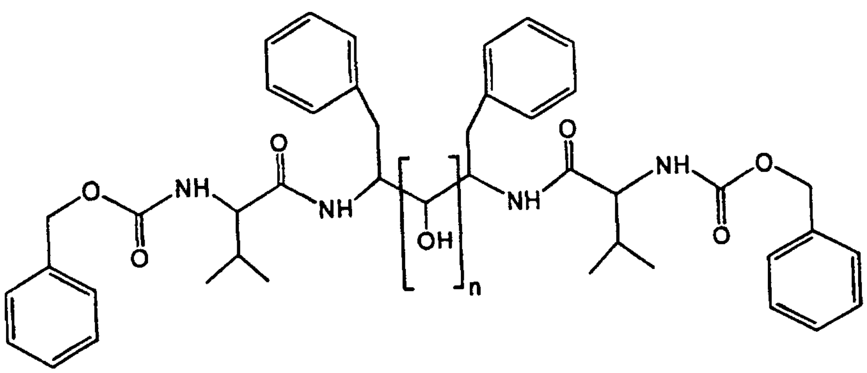

F. 5

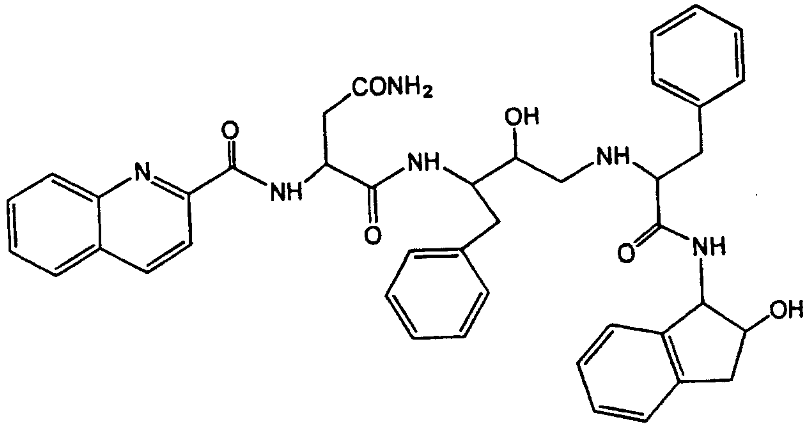

F. 6

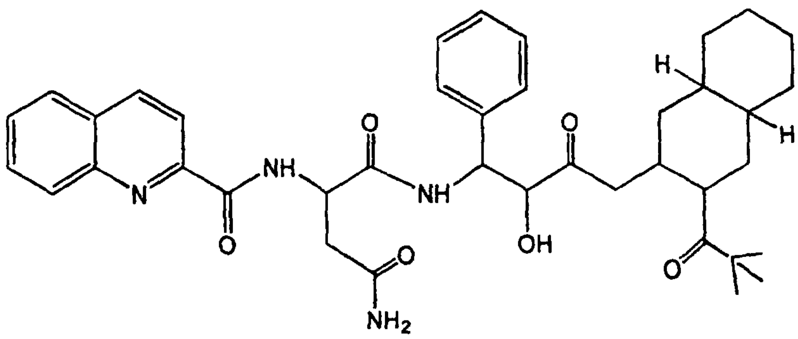

F. 7

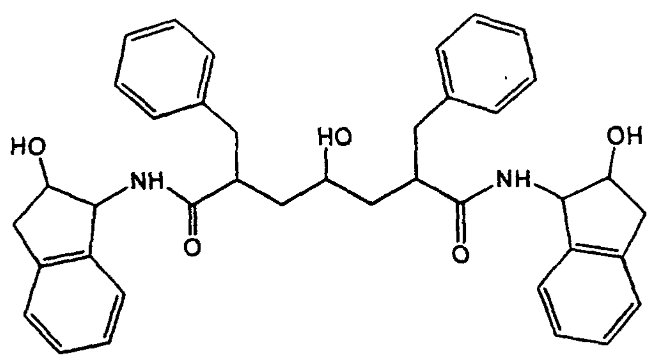

F. 8

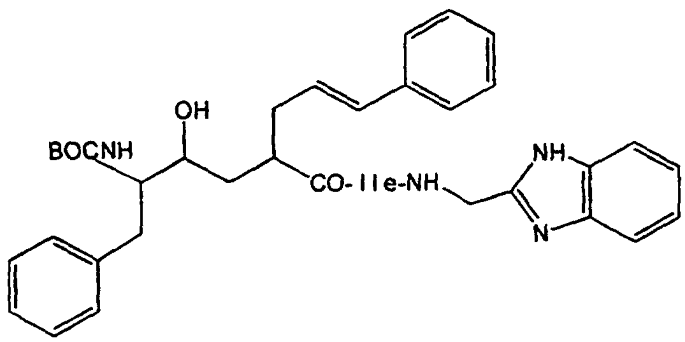

F. 9

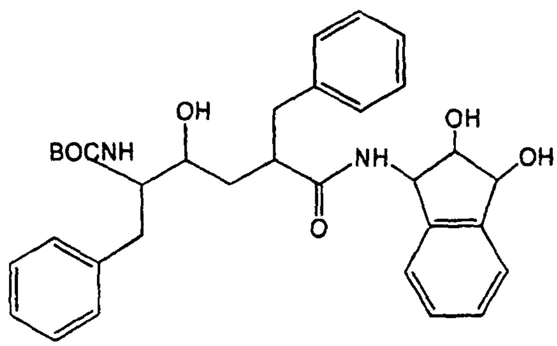

F. 10

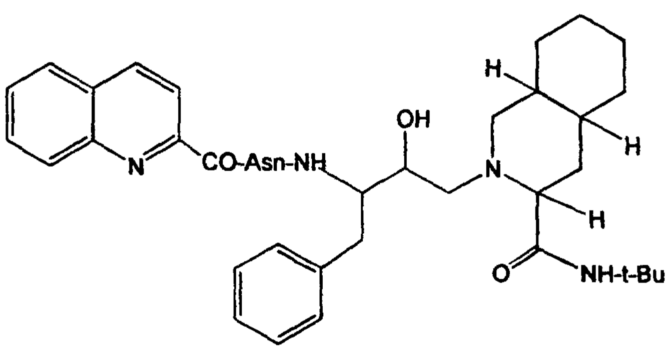

F. 11

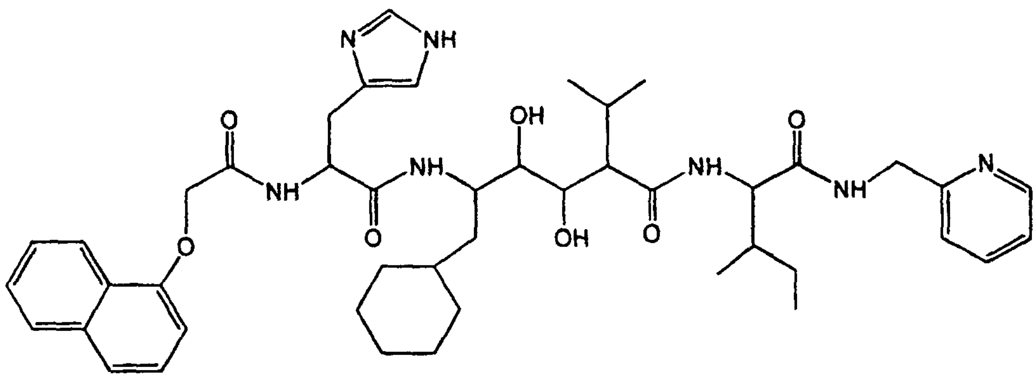

F. 12

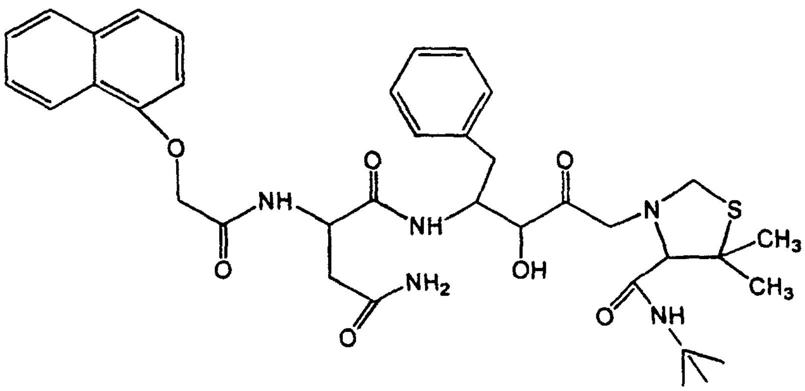

F. 13

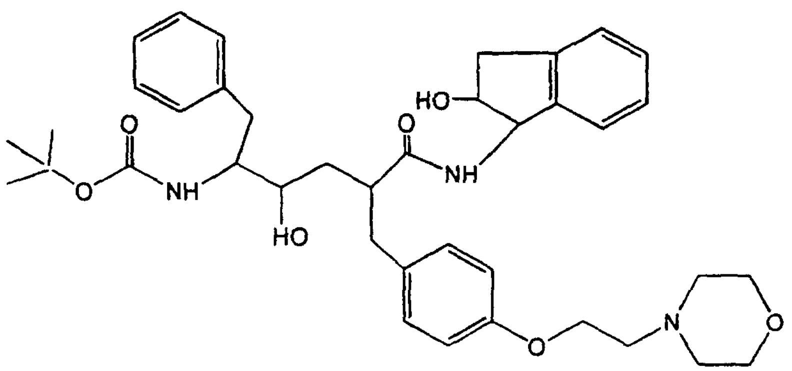

F. 14

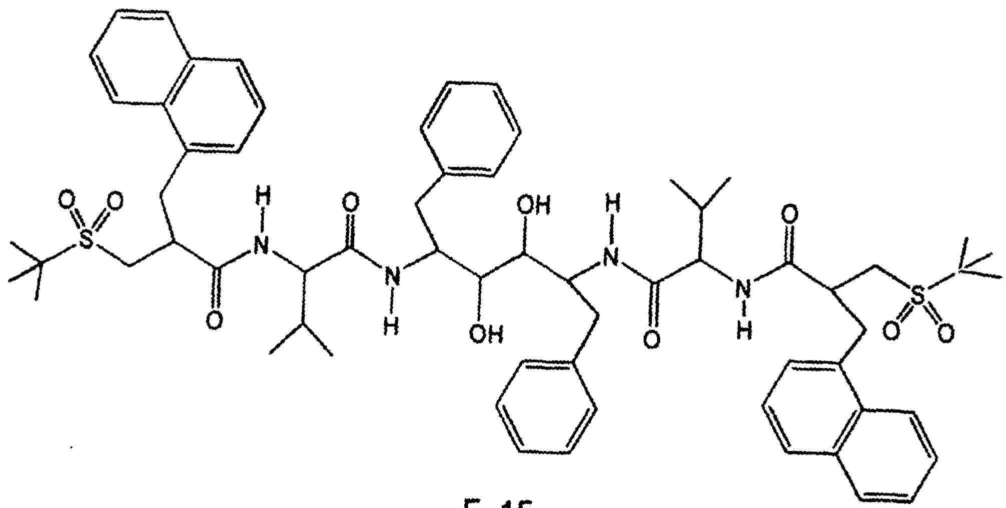

F. 15

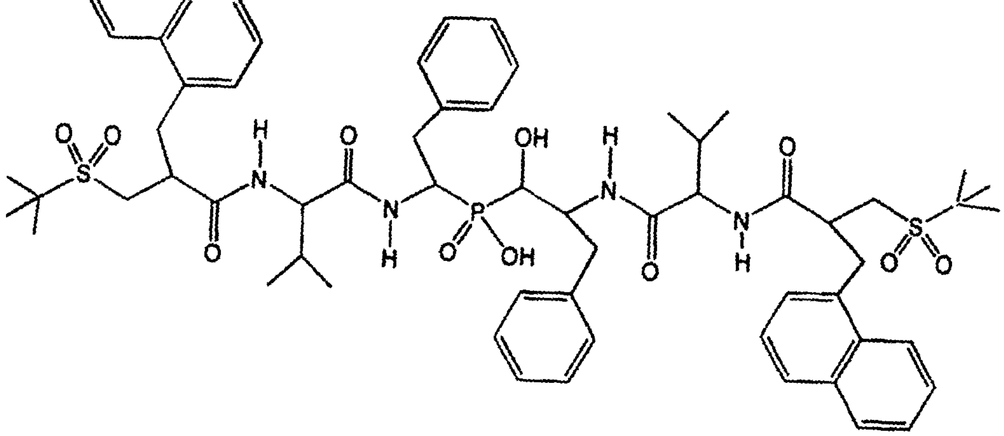

F. 16

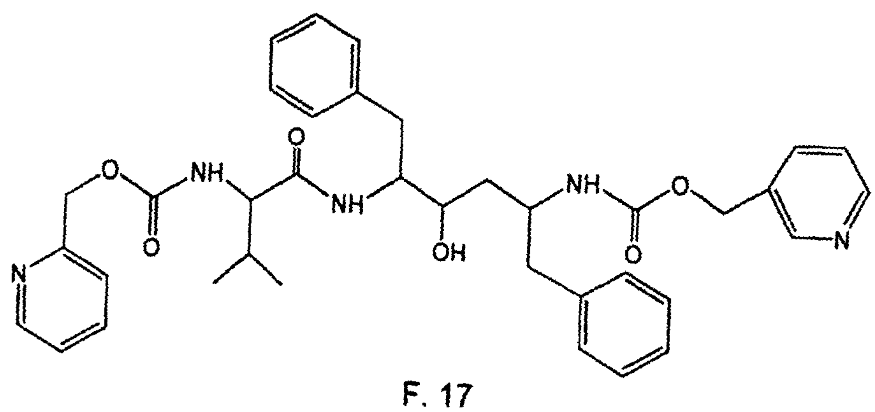

F. 17

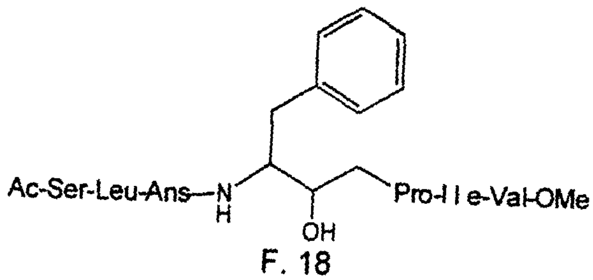

F. 18

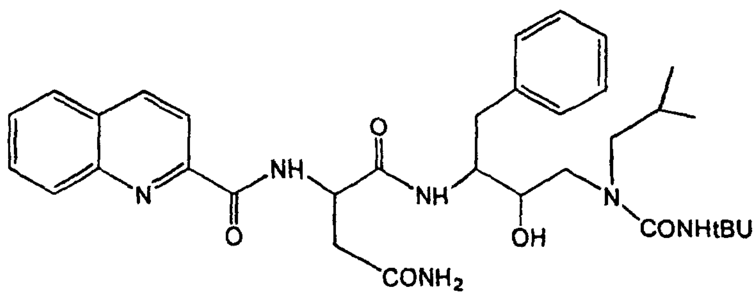

F. 19

a vaut 1, 2 ou 3 et $R^5$ est un radical de formule

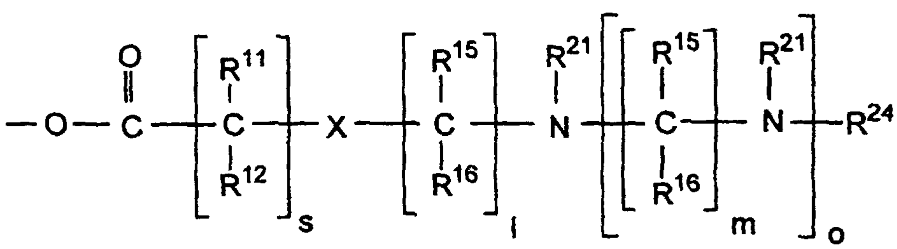

(III)

X est O, S, $NR^{20}$ ou $N^+(R^{20})_2$,
de préférence S ou $NR^{20}$,

où

$R^{20}$ est H, un radical alkyle (en $C_1$-$C_6$), cycloalkyle (en $C_3$-$C_7$), $-CH_2-(CH_2)_n-NR^{21}R^{24}$, $-CH_2-C(O)-R^{28}$, $CH_2CONH$-alkyle (en $C_1$-$C_6$), qui peut être substitué jusqu'à 5 groupes OH, COO-alkyle (en $C_1$-$C_6$), ou $-CH_2-P(O)((alkyle$ (en $C_1$-$C_4$$)_2$, de préférence H, un radical alkyle (en $C_1$-$C_4$), $-CH_2-C(O)-R^{28}$ ou $-CH_2-P(O)Me_2$,
en particulier de préférence H, un radical alkyle (en $C_1$-$C_4$),

$R^{11}$, $R^{12}$, $R^{15}$ et $R^{16}$ sont indépendamment l'un de l'autre H ou un radical alkyle (en $C_1$-$C_4$),
de préférence H ou un radical méthyle,

$R^{21}$ est H, un radical alkyle (en $C_1$-$C_6$), cylcoalkyle (en $C_3$-$C_7$) ou alcoxycarbonyle (en $C_1$-$C_4$),
de préférence H, un radical alkyle (en $C_1$-$C_4$),

$R^{24}$ est H, un radical alkyle (en $C_1$-$C_{18}$), cycloalkyle (en $C_3$-$C_{14}$), alcényle (en $C_2$-$C_{18}$), alcynyle (en $C_2$-$C_{18}$) ou aryle (en $C_6$-$C_{14}$), chacun pouvant être substitué une fois, deux fois ou trois fois par un radical aryle (en $C_6$-$C_{14}$), aryloxy (en $C_6$-$C_{14}$), cycloalkyle (en $C_3$-$C_{14}$), hydroxy, alcoxy (en $C_1$-$C_4$), $-C(O)-R^{28}$, $P(O)((alkyle$ (en $C_1$-$C_4$$))_2$ ou halogéno et sont liés directement ou éventuellement via CO avec $NR^{21}$, ou un $\alpha$-acide

aminé lié via le groupe carbonyle, ou $R^{24}$ est (alkyl (en $C_1$-$C_4$))-N(alkyl (en $C_1$-$C_4$))-alkyl (en $C_1$-$C_4$)-NH (alkyl(en $C_1$-$C_4$)) ou $R^{24}$ forme avec $R^{21}$ un hétérocycle, de préférence

$R^{24}$ est H, un radical alkyle (en $C_1$-$C_4$) qui peut être éventuellement substitué par un radical phényle, $NH_2$ ou cycloalkyle (en $C_3$-$C_7$) et est lié directement ou éventuellement via CO avec $NR^{21}$, un $\alpha$-acide aminé naturel lié via le groupe carbonyle, $-CH_2C(O)R^{28}$, $-CH_2-P(O)Me_2$,
$-(CH_2)_3N(Me)(CH_2)_2NHMe$, en particulier de préférence $R^{24}$ est H, ou un radical alkyle (en $C_1$-$C_4$),

$R^{28}$ est OH, O-alkyle (en $C_1$-$C_6$), O-cycloalkyle (en $C_3$-$C_6$), $NH_2$, -NH-alkyle (en $C_1$-$C_6$), qui peut être substitué par jusqu'à 5 groupes OH, -NH-cycloalkyle (en $C_3$-$C_7$) ou -N-bis-alkyle (en $C_1$-$C_4$), de préférence $R^{28}$ est OH, $NH_2$, O-alkyle (en $C_1$-$C_4$), -NH-alkyle (en $C_1$-$C_4$), ou -N-bis-alkyle (en $C_1$-$C_4$), en particulier de préférence $R^{28}$ est indépendamment l'un de l'autre OH, $NH_2$ ou O-alkyle (en $C_1$-$C_4$),

où

- l vaut 2 ou 3, de préférence 2,
- s vaut 1, 2, 3, 4 ou 5, de préférence 1, 4 ou 5, en particulier de préférence 1,
- m vaut 0, 1, 2, 3 ou 4, de préférence 2 ou 3,
- n vaut 1, 2 ou 3, de préférence 1 ou 2,
- o vaut 0, 1, 2 ou 3, de préférence 0, 1 ou 2, en particulier de préférence 0.

**2.** Procédé de préparation de composés de formule I selon la revendication 1, **caractérisé en ce qu'**on fait réagir un composé actif de formule $W-(OH)_b$ dans laquelle W a la définition donnée ci-dessus et b peut être 1, 2 ou 3, mais supérieur ou égal à a, avec

A) un composé de formule (X)

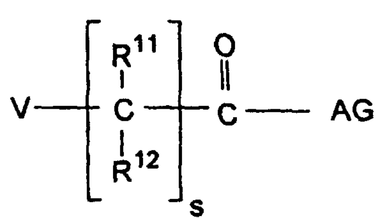

(X)

pour donner le composé de formule (XX)

$$[OH]_{b-a}-W-[OCO-(CR^{11}, R^{12})_s-V]_a \qquad (XX)$$

et finalement
B) on fait réagir le composé résultant de formule (XX) lui-même avec des composés nucléophiles de formule (XI)

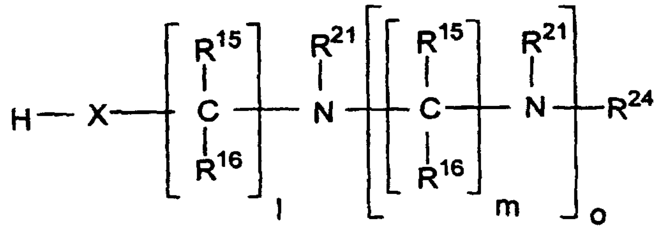

(XI)

formules dans lesquelles W, a, b, $R^{11}$, $R^{12}$, $R^{15}$, $R^{16}$, $R^{21}$, $R^{24}$ l, m, o et s ont les significations données ci-dessus, V est un groupe partant convenable, de préférence Br, Cl, OTs, en particulier de préférence Br, AG est un groupe partant qui est convenable pour l'estérification, de préférence Br ou Cl ou un groupe qui résulte de procédés d'estérification active pour les acides carboxyliques.

**3.** Composé de formule I selon la revendication 1 pour l'utilisation comme médicament.

**4.** Utilisation d'un composé de formule I selon la revendication 1 pour la préparation d'un médicament pour le traitement de maladies virales.

**5.** Médicament contenant une quantité efficace d'un composé selon la revendication 1.

**6.** Utilisation d'un composé selon la revendication 1 pour le traitement d'un syndrome immunitaire acquis ou de l'ARC (complexe en liaison avec le SIDA).

Fig. 1/1

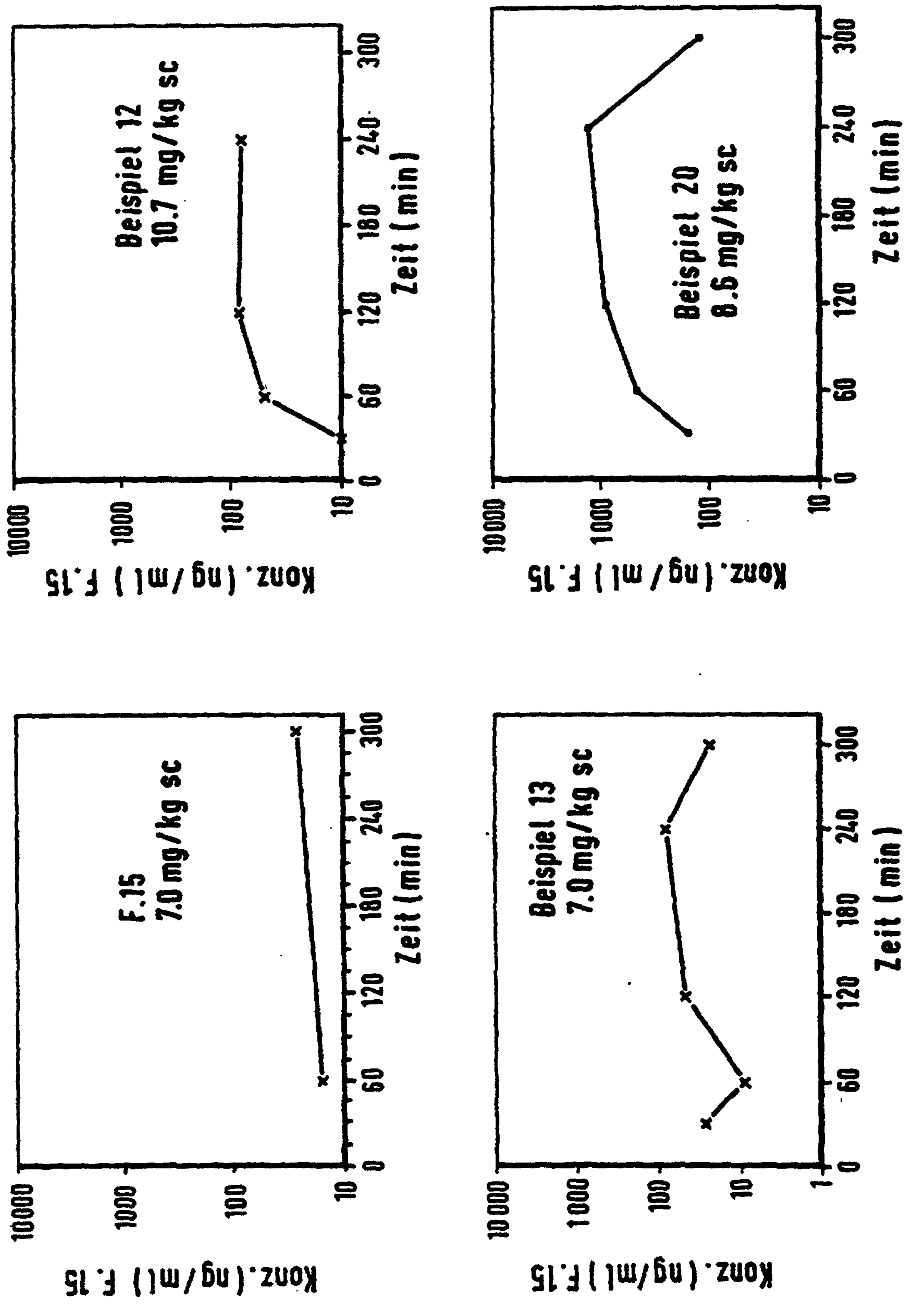
Beispiel 12
10.7 mg/kg sc
Beispiel 20
8.6 mg/kg sc
F.15
7.0 mg/kg sc
Beispiel 13
7.0 mg/kg sc
Zeit (min)
Konz. (ng/ml) F.15
0
60
120
180
240
300
10000
1000
100
10
1

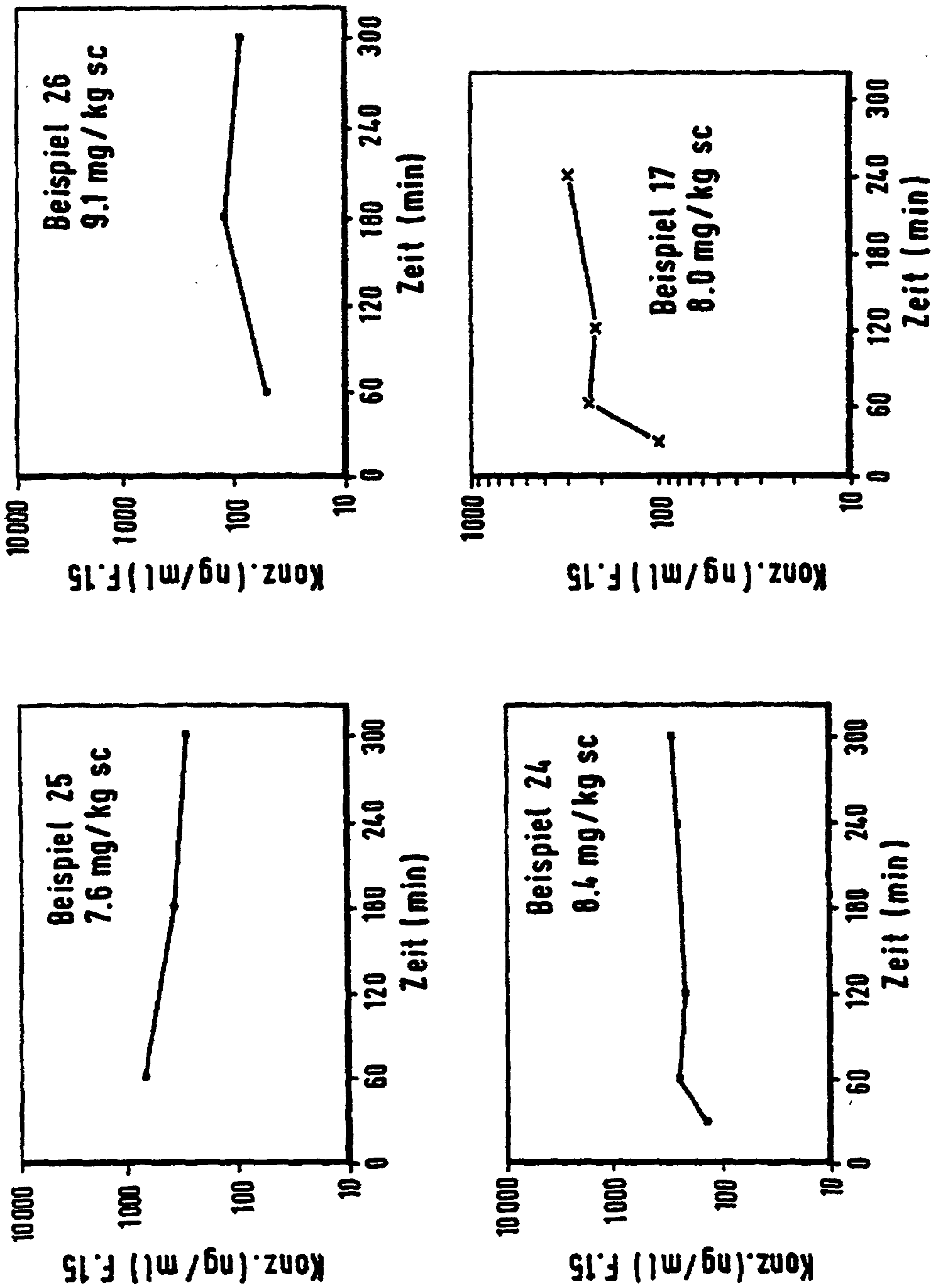
Beispiel 26
9.1 mg/kg sc
Beispiel 17
8.0 mg/kg sc
Beispiel 25
7.6 mg/kg sc
Beispiel 24
8.4 mg/kg sc
Konz.(ng/ml) F.15
Zeit (min)
0 60 120 180 240 300
10 100 1000 10000

Fig. 1/2

Fig. 2

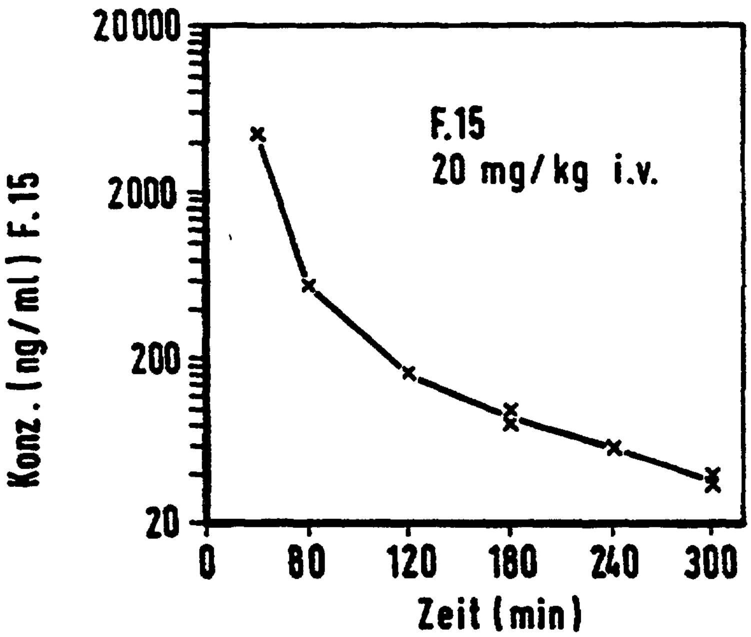
20000
2000
200
20
Konz. (ng/ml) F.15
F.15
20 mg/kg i.v.
0
80
120
180
240
300
Zeit (min)

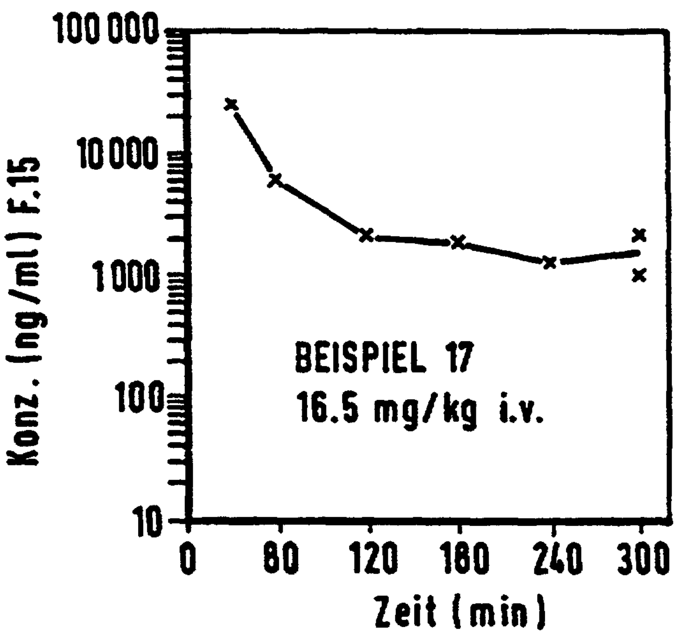
100 000
10 000
1000
100
10
Konz. (ng/ml) F.15
BEISPIEL 17
16.5 mg/kg i.v.
0
80
120
180
240
300
Zeit (min)